# FASCICULE DE BREVET EUROPEEN

(11) **EP 3 707 130 B1**
(45) Date de publication et mention de la délivrance du brevet: **03.04.2024**
(21) Numéro de dépôt: 18812242.8
(22) Date de dépôt: 07.11.2018
(51) Int. Cl.: C07D 303/28, C08G 59/22, C08G 59/32, C08L 63/00

(54) **COMPOSES BIPHENYLE PLURIEPOXYDES, PREPARATION ET UTILISATIONS**
POLYEPOXIDIERTE BIPHENYL VERBINDUGEN, DEREN HERSTELLUNG UND ANWENDUNGEN
POLYEPOXIDIZED BIPHENYL COMPOUNDS, THEIR PREPARATION AND USES

(30) Priorité: 07.11.2017 FR 1760451
(43) Date de publication de la demande: 16.09.2020
(73) Titulaire: ArianeGroup SAS, 78130 Les Mureaux (FR); Centre National de la Recherche Scientifique, 75016 Paris (FR)
(72) Inventeur: SAVONNET, Etienne, 75018 Paris (FR); DEFOORT, Brigitte, 33160 Saint-medard-en-jalles (FR); CRAMAIL, Henri, 33350 Sainte-terre (FR); GRELIER, Stéphane, 40160 Parentis-en-born (FR); GRAU, Etienne, 33000 Bordeaux (FR)
(74) Mandataire: Cabinet Beau de Loménie
(86) Numéro de dépôt international: PCT/FR2018/052746
(87) Numéro de publication internationale: WO 2019/092359

(56) Documents cités:
- EP-A1- 3 165 549
- JP-A- 2017 165 694
- US-A- 4 256 764
- DUANN Y-F ET AL.: "Thermal stability of some naphthalene- and phenyl-based epoxy resins", POLYMER DEGRADATION AND STABILITY, vol. 84, no. 2, 1 mai 2004 (2004-05-01), pages 305-310, XP004510148, ISSN: 0141-3910, DOI: 10.1016/S0141-3910(04)00008-4
- FETOUAKI S ET AL: "Synthèse d'une nouvelle résine époxyde à base du 2,2'-dihydroxydiphényle", EUROPEAN POLYMER JOURNAL, vol. 38, no. 4, 1 avril 2002 (2002-04-01), pages 787-793, XP004340248, ISSN: 0014-3057, DOI: 10.1016/S0014-3057(01)00014-3

## Description

La présente invention a pour principal objet un procédé de préparation de composés biphényle pluriépoxydés (i.e. dont la formule chimique renferme au moins 2 fonctions époxy). Ces composés constituent des monomères ou prépolymères, de type résine époxy, thermodurcissables. Ils sont particulièrement intéressants en ce qu'ils conviennent parfaitement comme précurseurs pour l'obtention de polyépoxydes (résines époxy thermodurcies) présentant de hautes propriétés mécaniques et de tenue en température et en ce que, pour nombre d'entre eux, ils peuvent être obtenus, outre par des voies de synthèse conventionnelles (connues dans le domaine de la pétrochimie), à partir de la biomasse (à partir de la lignine) (on peut alors parler de produits biosourcés)..

A ce jour, les résines époxy, polymérisables sous l'action d'un durcisseur (on parle de polymérisation réticulante), représentent la majorité des adhésifs structurels et plus de 75 % d'entre elles sont obtenues à partir du (monomère ou prépolymère) diglycidyléther de bisphénol-A (DiGlycicyl Ether de Bisphénol-A = DGEBA), qui présente la formule chimique ci-après :

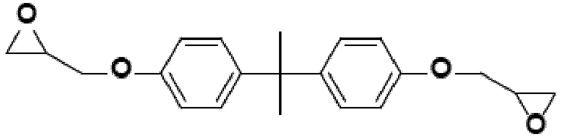

Celui-ci est lui-même obtenu à partir du bisphénol-A (BisPhenol-A = BPA), qui présente la formule chimique ci-après : 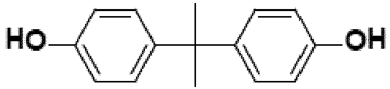 et dont la toxicité est avérée, notamment à titre de perturbateur endocrinien. Ceci ne manque pas de poser des problèmes et ne manquera pas d'en poser des plus en plus sérieux, au vu des régulations de plus en plus contraignantes qui s'imposent. De surcroît, l'homme du métier n'ignore pas que ledit BPA est obtenu à partir de ressources fossiles.

Ainsi, depuis quelques années, en référence à des considérations de santé publique et environnementales, est-on à la recherche de nouveaux précurseurs (nouveaux monomères ou prépolymères) de polyépoxydes, substituts dudit DGEBA, non toxiques, avantageusement d'origine naturelle (avantageusement biosourcés).

On s'est intéressé à la vanilline, composé dont la formule chimique est reproduite ci-après :

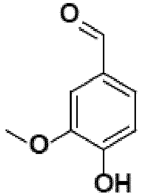

La vanilline est *a priori* un candidat très intéressant dans la mesure où elle consiste en un des rares composés aromatiques, non toxiques, disponibles industriellement à partir de la biomasse.

L'utilisation de la vanilline et de ses dérivés, comme blocs constitutifs de base, d'origine naturelle, de polymères renouvelables et notamment de résines époxy, a ainsi été étudiée avec un grand intérêt. Récemment, des résines époxy durcies à base de vanilline, présentant des Tg entre 80°C et 176°C, ont été synthétisées.

A propos de cette utilisation de la vanilline, on peut notamment se référer à Fache, M.; Darroman, E.; Besse, V.; Auvergne, R.; Caillol, S.; Boutevin, B. dans Green Chem. 2014, 16 (4), 1987.

Cependant, les monomères avec groupes époxy obtenus à partir de la vanilline ou de ses dérivés, ne permettent pas l'obtention de polyépoxydes qui présentent de hautes propriétés mécaniques et de tenue en température.

De la même façon, on s'est intéressé aux eugénols (eugénol et isoeugénol). A ce propos, on peut notamment se référer à François, C.; Pourchet, S.; Boni, G.; Fontaine, S.; Gaillard, Y.; Placet, V.; Galkin, M. V.; Orebom, A.; Samec, J.; Plasseraud, L. dans RSC Adv. 2016, 6 (73), 68732-68738, et à Qin, J.; Liu, H.; Zhang, P.; Wolcott, M.; Zhang, dans J. Polym. Int. 2014, 63 (4), 760-765.

Les polyépoxydes obtenus à partir de ces monomères n'ont pas donné pleine satisfaction. On rappelle ci-après, à toutes fins utiles, les formules chimiques desdits eugénol et isoeugénol :

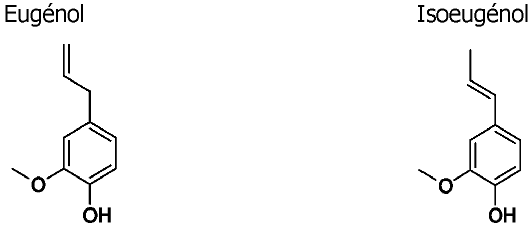

La demande de brevet JP-A- 2017 165 694 décrit des composés époxy et des résines thermodurcissables obtenues à partir de ces composés.

Dans un tel contexte, il est du mérite des inventeurs de proposer un procédé de préparation de composés biphényle pluriépoxydés qui répondent à la formule (I) ci-après : 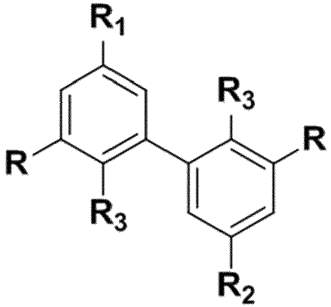 dans laquelle :
R = -O-Alk, avec Alk un groupe alkyle, linéaire ou ramifié, comportant de 1 à 6 atomes de carbone, R étant avantageusement un groupe méthoxy (-O-CH₃) ;
R₃ = -O-Z, avec Z un groupe alkyle, linéaire ou ramifié, comportant de 2, avantageusement de 3, à 8 atomes de carbone, et renfermant une fonction époxy, ou -O-Alk', avec Alk' un groupe alkyle, linéaire ou ramifié, comportant de 1 à 6 atomes de carbone ; et
+ lorsque R₃ = -O-Z, alors
   soit R₁ et R₂, identiques ou différents, sont choisis indépendamment parmi - CH₂-OH et -CH₂-O-Z ;
   soit R₁=R₂=-CHO ;
   soit R₁ et R₂, identiques ou différents, sont choisis indépendamment parmi -OH et -O-Z ;
   soit R₁ et R₂, identiques ou différents, sont choisis indépendamment parmi - COOH et -COO-Z,
   soit R₁ et R₂, identiques ou différents, sont choisis indépendamment parmi - CH₂-CH=CH₂ et -CH₂-époxy,
   soit R₁ et R₂, identiques ou différents, sont choisis indépendamment parmi - CH=CH-CH₃ et 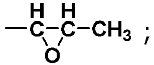 et
+ lorsque R₃ = -O-Alk', alors
R₁ = R₂ et est choisi parmi
-CH₂-O-Z,
-O-Z,
-COO-Z,
avec Z tel que défini ci-dessus en référence à R₃ = -O-Z (i.e. Z un groupe alkyle, linéaire ou ramifié, comportant de 2, avantageusement de 3, à 8 atomes de carbone, et renfermant une fonction époxy),
-CH₂-époxy, et 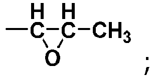
ledit procédé comprenant :
   a) la mise à disposition d'un dimère choisi parmi la divanilline, l'alcool divanillique, la diméthoxyhydroquinone, l'acide divanillique, le dieugénol, le dimère de l'isoeugénol, lesdits dimères présentant au moins deux fonctions -OH phénoliques et deux fonctions -O-CH₃, et les analogues desdits dimères présentant lesdites au moins deux fonctions - OH phénoliques et deux fonctions -O-(C₂-C₆)alkyle ;
   b) l'éventuelle alkylation des fonctions -OH phénoliques d'un dimère choisi parmi la divanilline, l'alcool divanillique, l'acide divanillique, le dieugénol, le dimère de l'isoeugénol et leurs analogues pour l'obtention de ladite divanilline alkylée, dudit alcool divanillique alkylé, dudit acide divanillique alkylé, dudit dieugénol alkylé, dudit dimère de l'isoeugénol alkylé et de leurs analogues alkylés ; l'alkylation de la divanilline ou d'un de ses analogues étant suivie d'une oxydation pour l'obtention de la di(C₁-C₆)alcoxyhydroquinone alkylée ; et
   c1) en l'absence d'une telle alkylation, l'époxydation des deux fonctions -OH phénoliques de la divanilline ou d'un de ses analogues ou l'époxydation des deux, voire trois, voire même quatre, fonctions présentes sur le noyau biphényle susceptibles d'être époxydées, dudit alcool divanillique, de ladite diméthoxyhydroquinone, dudit acide divanillique, dudit dieugénol, dudit dimère de l'isoeugénol ou d'un de leurs analogues ; ou
   c2) suite à une telle alkylation, l'époxydation des deux fonctions non alkylées, encore présentes sur le noyau biphényle, de l'alcool divanillique alkylé, de la di(C₁-C₆)alcoxyhydroquinone alkylée, de l'acide divanillique alkylé, du dieugénol alkylé, du dimère de l'isoeugénol alkylé ou d'un de leurs analogues.

Les composés de formule (I) - composés biphényles (avec R = alcoxy) pluriépoxydés (on peut aussi dire polyépoxydés) - sont pluri(2, 3 ou 4)époxydés (poly(2,3,4)époxydés) dans la mesure où deux au moins des groupes R₁, R₂ et des deux groupes R₃ de ladite formule (I) renferment une fonction époxy (R₃ époxydé (=-O-Z) avec R₁ ou(et) R₂ possiblement époxydé(s) aussi ou R₃ non époxydé (=O-Alk') et alors R₁ = R₂ époxydé), et qu'ils se répartissent en deux familles principales selon la valeur de R₃.

Pour ce qui concerne le groupe alcoxy, R = -O-Alk, tel que défini ci-dessus, il consiste avantageusement en un groupe -O-Alk_{inférieur}, ledit groupe alkyle inférieur ne comportant que 1 à 4 atomes de carbone, voire très avantageusement que 1 ou 2 atomes de carbone. On comprend plus loin que la valeur de R est *a priori* fixée par la nature des produits de départ utilisés. Ainsi, à partir de l'éthyl vanilline de synthèse, on obtient des composés de formule (I) dans laquelle R=-O-C₂H₅, à partir de la vanilline, de l'eugénol et de isoeugénol (de synthèse ou, plutôt avantageusement d'origine naturelle), des composés de formule (I) dans laquelle R = -O-CH₃. Lesdits composés de formule (I) dans laquelle R = -O-CH₃ sont particulièrement préférés. Ainsi la suite de la présente description et les exemples sont-ils largement développés, de façon nullement limitative, en référence à cette valeur préférée.

Pour ce qui concerne la valeur de R₃, on a donc, pour les deux familles principales évoquées ci-dessus :
R₃ = -O-Z, alors les deux groupes R₃ apportent, chacun, une fonction époxy (la même) et le composé est susceptible d'être di-, tri- ou tétra-époxydé au vu de la nature des groupes R₁ et R₂ (on a compris que pour un composé de formule (I) dans laquelle R₃ = -O-Z, R₁ ou(et) R₂, s'il(s) renferme(nt) Z, renferme(nt) obligatoirement le même Z (voir la définition ci-dessus des groupes R₁, R₂ et R₃, assurément confortée par la partie ci-après de la description relative à l'aspect procédé de l'invention) ; ou
R₃ = -O-Alk', un groupe alcoxy, alors les groupes R₁ = R₂ apportent, chacun, la même fonction époxy et le composé est un composé diépoxydé. Si R₁ = R₂ inclut Z, c'est évidemment à la définition de Z (donnée en référence à R₃ = -O-Z) qu'il convient de se référer (voir la définition ci-dessus des groupes R₁, R₂ et R₃, assurément confortée par la partie ci-après de la description relative à l'aspect procédé de l'invention).

Chacune desdites deux familles principales est elle-même constituée, selon une même logique, de sous-familles.

La construction desdites familles et sous-familles découle évidemment, d'une part, de la nature des produits de départ (avantageusement issus de la biomasse), plus exactement des fonctions présentes dans la formule chimique desdits produits de départ et dans celle de produits intermédiaires obtenus à partir desdits produits de départ, fonctions susceptibles d'être époxydées (fonctions alcool, hydroxy, acide, - CH=CH₂ ou -CH=CH-) et, d'autre part, des réactions mises en œuvre pour l'époxydation desdites fonctions.

On se propose de préciser ci-après chacune desdites familles et sous-familles.
* **1^{ère} famille** : **R₃** = **-O-Z,** avec Z un groupe alkyle, linéaire ou ramifié, comportant de 2, avantageusement de 3, à 8 atomes de carbone, et renfermant une fonction époxy (ladite fonction époxy renfermant 2 desdits 2 à 8 atomes de carbone (totaux)). Notons ici que l'expression « groupe alkyle ramifié » inclut « un groupe alkyle ramifié conventionnel et un groupe alkyle incluant un cycloalkyle (avec l'un des atomes de carbone de la chaîne alkyle appartenant aussi audit cycloalkyle»).

On peut indiquer, de façon nullement limitative, que, selon des variantes avantageuses, indépendantes :
- Z est linéaire,
- Z renferme ladite fonction époxy en bout de chaîne ;
lesdites variantes avantageuses, prises en combinaison, constituant une variante très avantageuse (Z, (C₂-C₈)alkyle, linéaire, renfermant ladite fonction époxy en bout de chaîne, i.e. R₃ = -O-[CH₂-]ₙ-époxy avec n, un entier de 0, avantageusement de 1, à 6).

A titre nullement limitatif, on peut préciser ici des valeurs de Z :
Z = -[CH₂]ₙ-époxy, avec n un entier de 0 à 6, avantageusement de 1 à 6 et notamment n = 4, très avantageusement n = 1,
Z = -CH₂-CH(CH₃)-CH₂-CH₂-époxy,
Z = 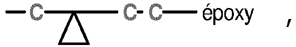 et
Z = CH₂- C(CH₃)₂-CH₂-CH₂-époxy.

Selon une variante préférée, on a Z = -[CH₂-]ₙ-époxy avec 1 ≤ n ≤ 4 et selon une variante particulièrement préférée, on a Z = -CH₂-époxy et donc R₃ = -O-CH₂-époxy.

Dans cette première famille, comme indiqué ci-dessus, les composés biphényle peuvent être di-, tri- ou tétra-époxydés.

Selon un aspect, la présente invention concerne un procédé de préparation de composés biphényle de formule (I) dans laquelle : R₃ = -O-Z (voir ci-dessus) et R₁ et R₂, identiques ou différents, sont choisis indépendamment parmi -CH₂-OH et -CH₂-O-Z. Ces composés sont des composés dérivés de l'alcool divanillique. La présente invention concerne particulièrement la préparation des composés de formule (I) avec R₃ =-O-[CH₂-]ₙ-époxy (avec n, un entier de 0, avantageusement de 1, à 6) et, R₁ et R₂, identiques ou différents, choisis indépendamment parmi -CH₂-OH et -CH₂-O-[CH₂-]ₙ-époxy. Le procédé de l'invention concerne tout particulièrement la préparation desdits composés avec R₃ =-O-CH₂-époxy et, R₁ et R₂, identiques ou différents, choisis indépendamment parmi -CH₂-OH et -CH₂-O-CH₂-époxy, à savoir :
- le diglycidyl éther d'alcool divanillique (DiGEDVA),
- le triglycidyl éther d'alcool divanillique (TriGEDVA),
- le tétraglycidyl éther d'alcool divanillique (TetraGEDVA),

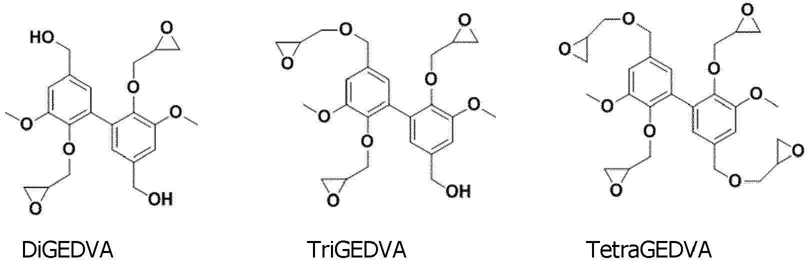

le procédé comprenant :
   a) la mise à disposition d'alcool divanillique ;
   b) l'époxydation de deux, trois, ou quatre fonctions -OH phénoliques de l'alcool divanillique.
      **b)** Selon un aspect, la présente invention concerne aussi la préparation de composés biphényles de formule (I) dans laquelle R₃= -O-Z (voir ci-dessus) et R₁ et R₂ sont identiques, égaux à -CHO. Ces composés sont des dérivés de la divanilline. Ils ont conservé les fonctions aldéhydes de la divanilline mais ils ont été époxydés en les fonctions hydroxy de celle-ci (devenues -O-Z). La présente invention concerne ainsi particulièrement la préparation des composés de formule (I) avec R₃ = -O-[CH₂-]ₙ-époxy (avec n, un entier de 0, avantageusement de 1, à 6) et R₁ = R₂ =-CHO. Elle concerne tout particulièrement la préparation du composé de formule (I) dans laquelle R₃ = -O-CH₂-époxy et R₁ = R₂ =-CHO, à savoir le diglycidyl éther de divanilline (DiGEDV), dont la formule (I) est reproduite ci-après : 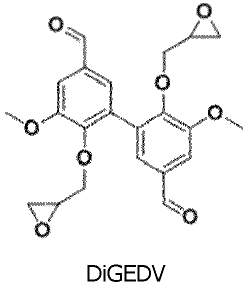
préparation qui comprend :
   a) la mise à disposition de divanilline ;
   b) l'époxydation des deux fonctions -OH phénoliques de la divanilline.
      **c)** Selon un aspect, la présente invention concerne aussi la préparation des composés de formule (I) dans laquelle R₃ = -O-Z (voir ci-dessus) et R₁ et R₂, identiques ou différents, sont choisis indépendamment parmi -OH et -O-Z. Ces composés sont des composés dérivés de la diméthoxyhydroquinone. La présente invention concerne particulièrement la préparation des composés de formule (I) avec R₃ =-O-[CH₂-]ₙ-époxy (avec n, un entier de 0, avantageusement de 1, à 6) et, R₁ et R₂, identiques ou différents, choisis indépendamment parmi -OH et -O-[CH₂-]ₙ-époxy ainsi que les mélanges d'au moins deux desdits composés. Elle concerne tout particulièrement la préparation desdits composés avec R₃ =-O-CH₂-époxy et, R₁ et R₂, identiques ou différents, choisis indépendamment parmi -OH et -O-CH₂-époxy, à savoir :
      - le diglycidyl éther de la diméthoxyhydroquinone (DiGEDMHQ),
      - le triglycidyl éther de la diméthoxyhydroquinone (TriGEDMHQ),
      - le tétraglycidyl éther de la diméthoxyhydroquinone (TetraGEDMHQ) (ce dernier composé étant particulièrement préféré), 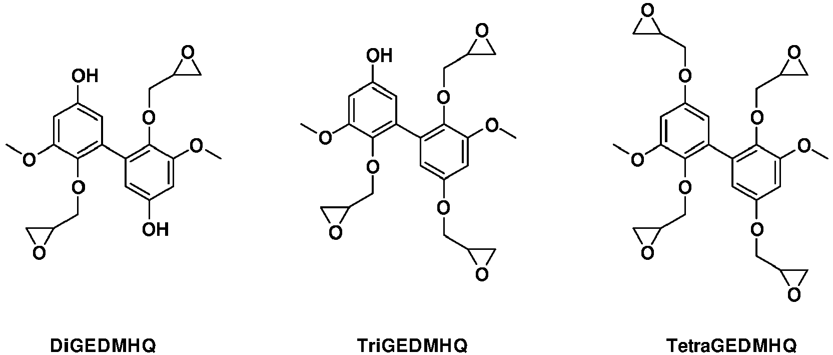
préparation qui comprend :
   a) la mise à disposition de diméthoxyhydroquinone ;
   b) l'époxydation de deux, trois, ou quatre fonctions -OH phénoliques de la diméthoxyhydroquinone.
      **d)** Selon un aspect, la présente invention concerne aussi la préparation des composés biphényle de formule (I) dans laquelle R₃ = -O-Z (voir ci-dessus) et R₁ et R₂, identiques ou différents, sont choisis parmi -COOH et -COO-Z. Ces composés sont des dérivés d'un divanillate d'alkyle (ester), en fait de l'acide divanillique. La présente invention concerne particulièrement la préparation des composés de formule (I) dans laquelle R₃ =-O-[CH₂-]ₙ-époxy (avec n, un entier de 0, avantageusement de 1, à 6) et R₁ et R₂, identiques ou différents, sont choisis parmi -COOH et -COO-[CH₂-]ₙ-époxy. Elle concerne tout particulièrement la préparation desdits composés avec R₃ =-O-CH₂-époxy et, R₁ et R₂, identiques ou différents, choisis indépendamment parmi -COOH et - COO-CH₂-époxy, à savoir :
      - le diglycidyl éther de l'acide divanillique (DiGEDVAc),
      - le triglycidyl éther de l'acide divanillique (TriGEDVAc),
      - le tétraglycidyl éther de l'acide divanillique (TétraGEDVAc), 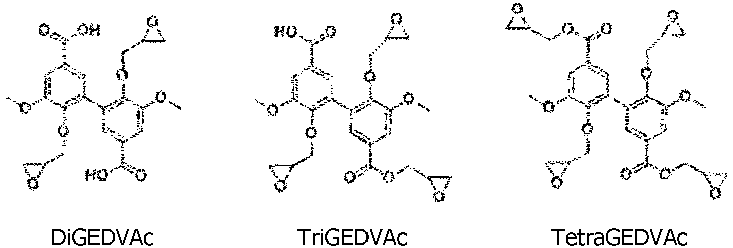
la préparation comprenant :
   a) la mise à disposition d'acide divanillique ;
   b) l'époxydation de deux, trois, ou quatre fonctions -OH phénoliques de l'acide divanillique.
      **e)** Selon un aspect, la présente invention concerne aussi la préparation des composés biphényle de formule (I) dans laquelle R₃ = -O-Z (voir ci-dessus) et R₁ et R₂, identiques ou différents, sont choisis parmi -CH₂-CH=CH₂ et -CH₂-époxy. Ces composés sont des dérivés du dieugénol. La présente invention concerne particulièrement la préparation des composés de formule (I) dans laquelle R₃ =-O-[CH₂-]ₙ-époxy (avec n, un entier de 0, avantageusement de 1, à 6) et R₁ et R₂, identiques ou différents, sont choisis parmi -CH₂-CH=CH₂ et -CH₂-époxy. Elle concerne tout particulièrement la préparation desdits composés avec R₃ =-O-CH₂-époxy et, R₁ et R₂, identiques ou différents, choisis indépendamment parmi -CH₂-CH=CH₂ et -CH₂-époxy, à savoir :
      - le diglycidyl éther de dieugénol (DiGEDEG),
      - le triglycidyl éther de dieugénol (TriGEDEG),
      - le tétraglycidyl éther de dieugénol (TétraGEDEG), 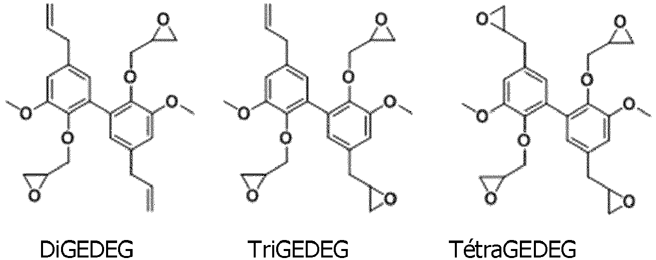
la préparation comprenant :
   a) la mise à disposition de dieugénol ;
   b) l'époxydation de deux, trois, ou quatre fonctions -OH phénoliques du dieugénol.
      **f)** Selon un aspect, la présente invention concerne aussi la préparation des composés biphényle de formule (I) dans laquelle R₃ = -O-Z (voir ci-dessus) et R₁ et R₂, identiques ou différents, sont choisis sont choisis indépendamment parmi -CH=CH-CH₃ et 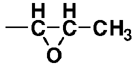
Ces composés sont des dérivés du diisoeugénol. La présente invention concerne particulièrement la préparation des composés de formule (I) dans laquelle R₃ =-O-[CH₂-]ₙ-époxy (avec n, un entier de 0, avantageusement de 1, à 6) et R₁ et R₂, identiques ou différents, sont choisis parmi -CH=CH-CH₃ et 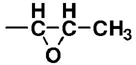 Elle concerne tout particulièrement la préparation desdits composés avec R₃ =-O-CH₂-époxy et, R₁ et R₂, identiques ou différents, choisis indépendamment parmi -CH=CH-CH₃ et 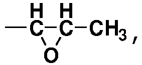 à savoir un composé de formule : 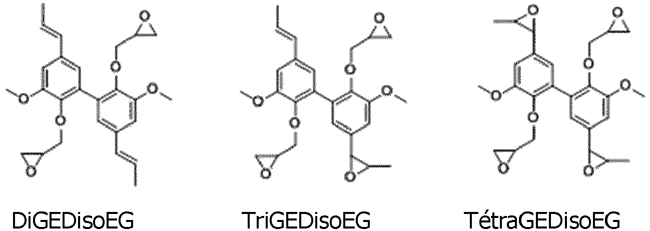 la préparation comprenant :
a) la mise à disposition de dimère de l'isoeugénol ;
b) l'époxydation de deux, trois, ou quatre fonctions -OH phénoliques du dimère de l'isoeugénol.

On note incidemment que lesdits TriGEDisoEG et TétraGEDisoEG ne présentent pas toutes leurs fonctions époxy en bout de chaîne. Lhomme du métier a d'ores et déjà compris que les fonctions époxy qui ne se trouvent pas en bout de chaîne sont celles qui correspondent aux doubles liaisons du diisoeugénol.
* **2^{éme} famille** : **R₃** = **-O-Alk',** avec Alk' un groupe alkyle, linéaire ou ramifié, comportant de 1 à 6 atomes de carbone. Alk' est indépendant de Alk mais de la même façon, il consiste avantageusement en un groupe Alk'_{inférieur}, ledit groupe alkyle inférieur ne comportant que 1 à 4 atomes de carbone. Très avantageusement, ledit groupe alkyle (Alk') ne comporte qu'un (1) atome de carbone. On a alors, très avantageusement, R₃ = -OCH₃ (un groupe méthoxy).

Les groupes R₃ des composés de formule (I) (composés biphényle pluriépoxydés) de la seconde famille n'étant ainsi pas époxydés (mais alkylés), il convient que leurs groupes R₁ et R₂ le soient. Les composés de la seconde famille sont, comme indiqué ci-dessus, di-époxydés, *via* lesdits groupes R₁ et R₂ (R₁ = R₂).

Lesdits groupes R₁ et R₂, au vu des matières premières et des synthèses non complexes préconisées (voir ci-après), sont tels que précisés ci-dessus :
R₁ = R₂ = -CH₂-O-Z, ou -O-Z, ou -COO-Z, avec, pour ces trois valeurs, Z tel que défini ci-dessus en référence à R₃ = -O-Z, i.e. Z un groupe alkyle, linéaire ou ramifié, comportant de 2, avantageusement de 3, à 8 atomes de carbone, et renfermant une fonction époxy, ou -CH₂-époxy, ou encore

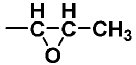

Tout comme indiqué ci-dessus : l'expression « groupe alkyle ramifié » inclut « un groupe alkyle ramifié conventionnel et un groupe alkyle incluant un cycloalkyle (avec l'un des atomes de carbone de la chaîne alkyle appartenant aussi audit cycloalkyle») et, selon des variantes avantageuses, indépendantes :
- Z est linéaire ;
- Z renferme ladite fonction époxy en bout de chaîne ;
lesdites variantes avantageuses, prises en combinaison, constituant une variante très avantageuse (Z, (C₂-C₈)alkyle, linéaire, renfermant ladite fonction époxy en bout de chaîne, i.e. R₃ = -O-[CH₂-]ₙ-époxy avec n, un entier de 0, avantageusement de 1, à 6).

De même, à titre nullement limitatif, on peut rappeler des valeurs de Z :
Z = -[CH₂]ₙ-époxy, avec n un entier de 0 à 6, avantageusement de 1 à 6 et notamment n = 4, très avantageusement n = 1 ;
Z = -CH₂-CH(CH₃)-CH₂-CH₂-époxy,
Z = 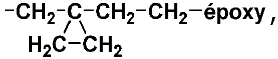 et
Z = CH₂- C(CH₃)₂-CH₂-CH₂-époxy.

De même, selon une variante préférée, on a Z = -[CH₂-]ₙ-époxy avec 1 ≤ n ≤ 4 et selon une variante particulièrement préférée, on a Z = -CH₂-époxy (et donc des valeurs correspondantes de R₁ = R₂ = -CH₂-O-Z, ou -O-Z, ou -COO-Z).

Ainsi :
**g)** Selon un aspect, la présente invention concerne aussi la préparation des composés biphényle de formule (I) dans laquelle : R₃ = -O-Alk' (avantageusement -O-CH₃ (voir ci-dessus)) et R₁ = R₂ =-CH₂-O-Z, avec Z tel que défini ci-dessus. Ces composés sont des composés dérivés de l'alcool divanillique alkylé. La présente invention concerne particulièrement la préparation des composés de formule (I) avec R₃ =-O-Alk' (avantageusement -O-CH₃ (voir ci-dessus)) et R₁ = R₂ = -CH₂-O-[CH₂-]ₙ-époxy (avec n, un entier de 0, avantageusement de 1, à 6). Elle concerne tout particulièrement la préparation desdits composés avec R₃ = -O-Alk' (avantageusement -O-CH₃ (voir ci-dessus)) et R₁ = R₂ = -CH₂-O-CH₂-époxy, et notamment le diglycidyl éther de l'alcool divanillique méthylé (DiGEmDVA) de formule : 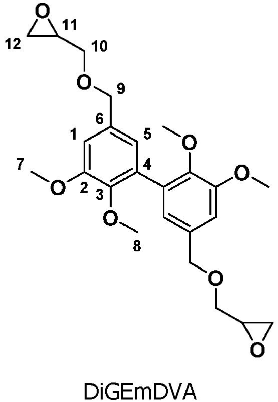 la préparation comprenant :
   a) la mise à disposition d'alcool divanillique ;
   b) la méthylation des fonctions -OH phénoliques de l'alcool divanillique, pour l'obtention d'alcool divanillique méthylé ;
   c) l'époxydation des deux fonctions non alkylées, encore présentes sur le noyau biphényle, de l'alcool divanillique méthylé.
**h)** Selon un aspect, la présente invention concerne aussi la préparation des composés biphényle de formule (I) dans laquelle : R₃ = -O-Alk' (avantageusement - OCH₃ (voir ci-dessus)) et R₁ = R₂ =-O-Z, avec Z tel que défini ci-dessus. Ces composés sont des composés dérivés de la diméthoxyhydroquinone alkylée. La présente invention concerne particulièrement la préparation des composés de formule (I) avec R₃ =-O-Alk' (avantageusement -O-CH₃ (voir ci-dessus)) et R₁ = R₂ = -O-[CH₂-]ₙ-époxy (avec n, un entier de 0, avantageusement de 1, à 6). Elle concerne tout particulièrement la préparation desdits composés avec R₃ = -O-Alk' (avantageusement -O-CH₃ (voir ci-dessus)) et R₁ = R₂ = -O-CH₂-époxy, et notamment le diglycidyl éther de la diméthoxyhydroquinone méthylée (DiGEmDMHQ) de formule : 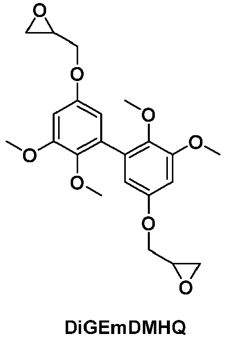 la préparation qui comprend :
   a) la mise à disposition de diméthoxyhydroquinone ;
   b) la méthylation des fonctions -OH phénoliques de la diméthoxyhydroquinone, pour l'obtention de diméthoxyhydroquinone méthylée ;
   c) l'époxydation des deux fonctions non alkylées, encore présentes sur le noyau biphényle, de la diméthoxyhydroquinone méthylée.
**i)** Selon un aspect, la présente invention concerne aussi la préparation des composés biphényle de formule (I) dans laquelle : R₃ = -O-Alk' (avantageusement -O-CH₃ (voir ci-dessus)) et R₁ = R₂ =-COO-Z, avec Z tel que défini ci-dessus. Ces composés sont des composés dérivés d'un divanillate d'alkyle (ester) alkylé, ou de l'acide divanillique alkylé. La présente invention concerne particulièrement la préparation des composés de formule (I) avec R₃ =-O-Alk' (avantageusement -O-CH₃ (voir ci-dessus)) et R₁ = R₂ = -COO-[CH₂-]ₙ-époxy (avec n, un entier de 0, avantageusement de 1, à 6). Elle concerne tout particulièrement la préparation desdits composés avec R₃ = -O-Alk' (avantageusement -O-CH₃ (voir ci-dessus)) et R₁ = R₂ = - COO-CH₂-époxy, et notamment le diglycidyl éther de l'acide divanillique méthylé (DiGEmDVAc) de formule : 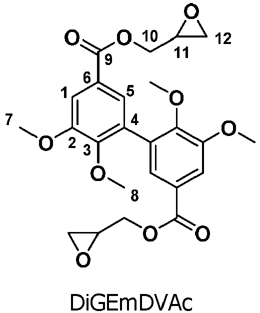 la préparation comprenant :
   a) la mise à disposition d'acide divanillique ;
   b) la méthylation des fonctions -OH phénoliques de l'acide divanillique, pour l'obtention d'acide divanillique méthylé ;
   b) l'époxydation des deux fonctions non alkylées, encore présentes sur le noyau biphényle, de l'acide divanillique méthylé.
**j)** Selon un aspect, la présente invention concerne aussi la préparation des composés biphényle de formule (I) dans laquelle : R₃ = -O-Alk' (avantageusement -O-CH₃ (voir ci-dessus)) et R₁ = R₂ =-CH₂-époxy. Ces composés sont des composés dérivés de l'eugénol alkylé. La présente invention concerne notamment la préparation du diglycidyl éther du dieugénol méthylé (DiGEmDEG) de formule : 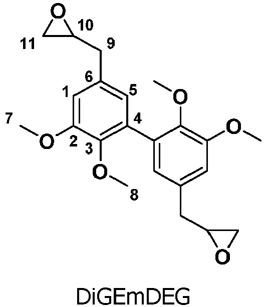 la préparation comprenant :
   a) la mise à disposition de dieugénol ;
   b) la méthylation des fonctions -OH phénoliques du dieugénol, pour l'obtention de dieugénol méthylé ;
   b) l'époxydation des deux fonctions non alkylées, encore présentes sur le noyau biphényle, du dieugénol méthylé.
**k)** Selon un aspect, la présente invention concerne aussi la préparation des composés biphényle de formule (I) dans laquelle : R₃ = -O-Alk' (avantageusement - OCH₃ (voir ci-dessus)) et R₁ = R₂ =

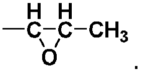

Ces composés sont des composés dérivés de l'isoeugénol alkylé. La présente invention concerne notamment la préparation du diglycidyl éther du dimère de l'isoeugénol méthylé (DiGEmDisoEG) de formule : 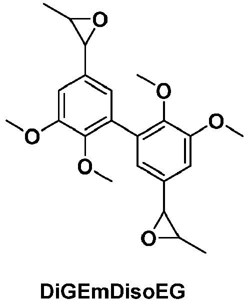 la préparation comprenant :
a) la mise à disposition de dimère de l'isoeugénol ;
b) la méthylation des fonctions -OH phénoliques du dimère de l'isoeugénol, pour l'obtention de dimère de l'isoeugénol méthylé ;
b) l'époxydation des deux fonctions non alkylées, encore présentes sur le noyau biphényle, du dimère de l'isoeugénol méthylé.

L'homme du métier a d'ores et déjà compris le grand intérêt des composés biphényles obtenus selon le procédé de l'invention, comme précurseurs de polyépoxydes (comme monomères ou prépolymères à polymériser (à réticuler) sous l'action d'un durcisseur), du fait de la présence des 2, 3 ou 4 groupes époxy (aptes à réagir avec ledit durcisseur) dans leur formule. Par ailleurs, la présence des deux noyaux aromatiques s'est révélée particulièrement intéressante en référence aux propriétés mécaniques et à la tenue en température desdits polyépoxydes et la présence de la liaison Carbone-Carbone entre lesdits deux noyaux aromatiques s'est révélée étonnamment opportune en référence au taux de coke résiduel après dégradation thermique desdits polyépoxydes.

On se doit par ailleurs d'insister sur la mise en oeuvre non complexe du procédé de l'invention et, possiblement, l'obtention de nombreux composés à partir de produits naturels tels la vanilline, l'eugénol et l'isoeugénol (voir ci-après).

Comme indiqué ci-dessus, le procédé de préparation des composés de formule (I) comprend :
a) la mise à disposition d'un dimère choisi parmi la divanilline, l'alcool divanillique, la diméthoxyhydroquinone, l'acide divanillique, le dieugénol, le dimère de l'isoeugénol, lesdits dimères présentant au moins deux fonctions -OH phénoliques et deux fonctions -O-CH₃, et les analogues desdits dimères présentant lesdites au moins deux fonctions - OH phénoliques et deux fonctions -O-(C₂-C₆)alkyle,
b) l'éventuelle alkylation des fonctions -OH phénoliques d'un dimère choisi parmi la divanilline, l'alcool divanillique, l'acide divanillique, le dieugénol, le dimère de l'isoeugénol et leurs analogues pour l'obtention de ladite divanille alkylée, dudit alcool divanillique alkylé, dudit acide divanillique alkylé, dudit dieugénol alkylé, dudit dimère de l'isoeugénol alkylé et de leurs analogues alkylés ; l'alkylation de la divanilline ou d'un de ses analogues étant suivie d'une oxydation pour l'obtention de la di(C₁-C₆)alcoxyhydroquinone alkylée ; et
c1) en l'absence d'une telle alkylation, l'époxydation des deux fonctions -OH phénoliques de la divanilline ou d'un de ses analogues ou l'époxydation des deux, voire trois, voire même quatre, fonctions présentes sur le noyau biphényle susceptibles d'être époxydées, dudit alcool divanillique, de ladite diméthoxyhydroquinone, dudit acide divanillique, dudit dieugénol, dudit dimère de l'isoeugénol ou d'un de leurs analogues ; ou
c2) suite à une telle alkylation, l'époxydation des deux fonctions non alkylées, encore présentes sur le noyau biphényle, de l'alcool divanillique alkylé, de la di(C₁-C₆)alcoxyhydroquinone alkylée, de l'acide divanillique alkylé, du dieugénol alkylé, du dimère de l'isoeugénol alkylé ou d'un de leurs analogues.

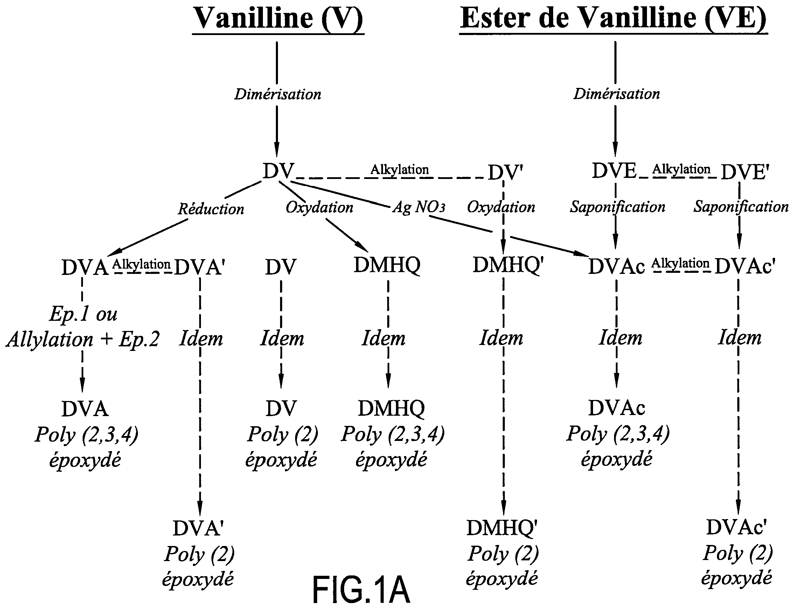

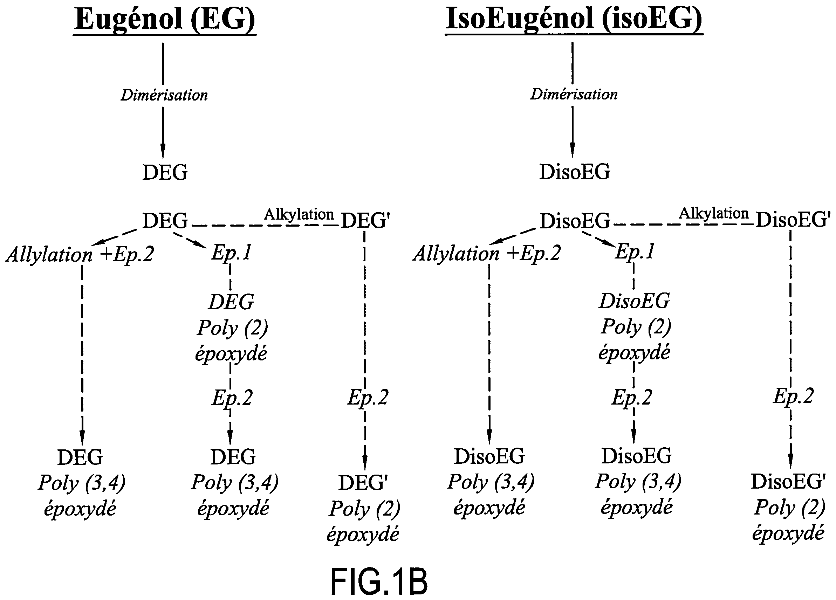

Ledit procédé est illustré, de façon schématique et non limitative, dans un contexte de préparation de composés de formule (I) dans laquelle R = -O-CH₃, sur les figures 1A et 1B. On comprend que le même schéma réactionnel convient pour la préparation de composés de formule (I) dans laquelle R = -O-(C₂-C₆)Alk (ledit R figurant dans la formule du produit de départ). Sur lesdites figures 1A et 1B, ainsi que dans la suite de la présente description, on a bien évidemment, poly(2)époxydé, poly(3)époxydé et poly(4)époxydé équivalent à, respectivement, di-époxydé, tri-époxydé et tétra-époxydé et poly(2,3,4)époxydé qui indique l'obtention possible des composés di-,tri-, tétra-époxydés tandis que poly(3,4)époxydé indique l'obtention possible des composés tri- et tétra-époxydés.

Bien évidemment, lorsque le procédé génère des mélanges d'au moins deux composés de l'invention (voir ci-après), il peut inclure une étape de séparation (conventionnelle, et notamment par chromatographie sur colonne) desdits au moins deux composés.

On se propose, à propos des réactifs et réactions en cause, de fournir, de façon non limitative, les informations ci-après.

On rappelle que la vanilline (V), l'eugénol (EG) et l'isoeugénol (isoEG) sont des produits naturels et que donc, nombre de composés de l'invention, obtenus à partir de tels produits naturels, sont des produits biosourcés. Ces produits - vanilline, eugénol et dimère de l'isoeugénol - ainsi que des produits présentant des formules relativement proches (esters de vanilline (VE), analogues de la vanilline avec un groupe alcoxy en C₂-C₆ en lieu et place du groupe méthoxy, ...) peuvent aussi être obtenus par synthèse conventionnelle. De tels produits, présentant des formules relativement proches, sont commercialisés, par exemple le vanillate de méthyle, l'éthyl vanilline... De tels produits conviennent assurément aussi pour la mise en œuvre du procédé tel que présentement décrit.

La dimérisation de tels produits ne pose pas de difficultés. Un couplage oxydatif est en cause. L'utilisation de la Laccase issue de *Trametes versicolor,* dans un tel contexte, a été largement décrite. Ainsi, les exemples 1, 4 et 7 de la demande EP 3 002 333 illustrent respectivement la préparation de la divanilline (DV), du divanillate de méthyle (exemple de DVE (ester de divanilline) de la figure 1A) et du dieugénol (DEG). Le dimère de l'isoeugénol (DisoEG) peut évidemment être obtenu dans les mêmes conditions que le dieugénol (DEG).

Les dimères ainsi obtenus (DV, DVE, DEG et DisoEG) peuvent être alkylés (notamment méthylés) en vue de la préparation de composés de l'invention de formule (I) dans laquelle R₃ = -O-Alk' (notamment R₃ = -O-CH₃). Le type d'alkylation en cause (plus exactement d'éthérification en cause : -OH devient R₃ = -O-Alk') ne pose pas de difficulté à l'homme du métier. Le bisphénol est mis en présence d'une base, telle le carbonate de potassium, dans un solvant (tel le diméthylformamide (DMF)) et un iodoalkyle (I-Alk', tel le iodométhane) est ajouté lentement. La réaction, à température élevée (par exemple 80 °C) dure plusieurs heures. A l'issue de la réaction, on filtre avantageusement le milieu réactionnel et le composé alkylé est récupéré par précipitation dans l'eau froide. Dans le cadre du procédé de l'invention, on peut ainsi mettre en œuvre cette alkylation :
- sur la divanilline (DV) pour obtenir la divanilline alkylée (DV') (avantageusement la divanilline méthylée) à partir de laquelle la diméthoxyhydroquinone alkylée (DMHQ'), alkylée en ses fonctions précurseurs de R₃ (et non en ses fonctions précurseurs de R₁ et/ou R₂), peut ensuite être préparée par oxydation, oxydation décrite ci-après pour le passage de la divanilline (DV) à la diméthoxyhydroquinone (DMHQ)) ;
- sur, éventuellement, un ester de divanilline (DVE), tout particulièrement le divanillate de méthyle, pour obtenir ledit ester de divanilline alkylé (DVE'), lequel ester de divanilline alkylé (DVE') peut être saponifié en lieu et place de l'acide divanillique (DVAc) (voir ci-après) ;
   (voir la figure 1A)
- sur le dieugénol (DEG) pour obtenir le dieugénol alkylé (DEG'), et
- sur le dimère de l'isoeugénol (DisoEG) pour obtenir le dimère de l'isoeugénol alkylé (DisoEG')
   (voir la figure 1B).

Les dimères choisis parmi la divanilline (DV), la divanilline alkylée (DV'), les esters de divanilline (DVE) et les esters de divanilline alkylés (DVE') peuvent être transformés en d'autres dimères dont la formule renferme des fonctions de type alcool, hydroxy et acide, fonctions susceptibles d'être époxydées ultérieurement. De tels dimères, avec de telles fonctions époxydables, en sus de leurs fonctions -OH phénoliques (époxydables), sont particulièrement intéressants, en ce qu'ils sont des précurseurs potentiels de composés de formule (I) avec trois ou quatre fonctions époxy.

Pour ce qui concerne la divanilline (DV) (voir la figure 1A) :
- ses fonctions aldéhyde peuvent être réduites en utilisant conventionnellement un réducteur de type borohydrure de sodium. Une telle réaction est illustrée dans l'exemple 8 de la demande EP 3 002 333. Elle conduit à l'alcool divanillique (DVA), qui éventuellement peut être alkylé conformément à la réaction d'alkylation décrite ci-dessus. Ledit alcool divanillique (DVA) renferme quatre fonctions susceptibles d'être époxydées (ses deux fonctions -OH phénoliques et ses deux fonctions alcool -CH₂OH), ledit alcool divanillique alkylé (DVA') renferme lui deux fonctions susceptibles d'être époxydées (ses deux fonctions alcool -CH₂OH) ;
- elle peut être oxydée selon la réaction d'oxydation de Dakin pour conduire à la diméthoxyhydroquinone (DMHQ) : du percarbonate de sodium est ajouté lentement à une solution aqueuse de soude renfermant ladite divanilline (DV). Le milieu ayant réagi est ensuite acidifié. La diméthoxyhydroquinone (DMHQ) formée est récupérée par extraction avec un solvant (tel l'acétate d'éthyle). Ladite diméthoxyhydroquinone (DMHQ) renferme quatre fonctions -OH phénoliques susceptibles d'être époxydées. L'alkylation sélective de deux desdites fonctions est difficilement gérable ; c'est pourquoi on a préconisé ci-dessus l'alkylation préalable de la divanilline (DV). On comprend que la réaction d'oxydation de Dakin mise en oeuvre sur la divanilline alkylée (DV') conduit à la diméthoxyhydroquinone alkylée (DMHQ') (alkylée en R₃) qui elle présente deux fonctions-OH phénoliques susceptibles d'être époxydées (et deux fonctions -OH alkylées) ;
- elle peut aussi être oxydée en acide divanillique (DVAc). Ledit acide (DVAc) est obtenu à partir de la divanilline (DV) par oxydation en milieu basique, et en présence d'un catalyseur homogène d'oxydation, tel AgNO₃. Ledit acide divanillique (DVAc) renferme quatre fonctions susceptibles d'être époxydées (ses deux fonctions -OH phénoliques et ses deux fonctions acides -COOH). Il peut être alkylé, dans les conditions indiqué ci-dessus, pour conduire à l'acide divanillique alkylé (DVAc') qui lui ne renferme que deux fonctions susceptibles d'être époxydées (ses fonctions acides - COOH).

On a parlé ci-dessus des esters de divanilline (DVE) et de leur éventuelle alkylation pour obtenir de tels esters de divanilline alkylés (DVE'). On peut, à partir desdits esters (DVE) et esters alkylés (DVE'), obtenir aussi, respectivement, l'acide divanillique (DVAc) et l'acide divanillique alkylé (DVAc'). Une réaction de saponification, telle qu'illustrée dans l'exemple 13 de la demande de brevet EP 3 002 333 (en présence de KOH et de méthanol)), est en cause (voir la figure 1A). Plus généralement, la saponification est mise en oeuvre en milieu alcoolique en présence d'une base forte.

On note incidemment que des étapes de purification peuvent être mises en oeuvre après l'obtention des dimères et/ou des dimères alkylés.

On en vient maintenant à l'époxydation des dimères ainsi obtenus - DV, DVA, DVA', DMHQ, DMHQ', DVAc, DVAc' (figure 1A), DEG, DEG', DisoEG, DisoEG' (figure 1B).

De manière générale, on préconise l'époxydation (d'une fonction époxydable, dans le présent contexte de type alcool, hydroxy, acide, double liaison), selon l'une ou l'autre des méthodes ci-après :
- par réaction avec l'épichlorhydrine ou équivalent (cette réaction a été dénommée Ep.1 sur les figures 1A et 1B annexées), i.e. par réaction avec un composé de formule Cl-Z, avec Z qui a la définition donnée pour les composés de formule (I), à savoir un groupe alkyle, linéaire ou ramifié, comportant de 2, avantageusement de 3, à 8 atomes de carbone, et renfermant une fonction époxy. On rappelle ici que, de façon nullement limitative, selon des variantes avantageuses, indépendantes :
   - Z est linéaire,
   - Z renferme ladite fonction époxy en bout de chaîne ;
lesdites variantes avantageuses, prises en combinaison, constituant une variante très avantageuse (Z, (C₂-C₈)alkyle, linéaire, renfermant ladite fonction époxy en bout de chaîne, i.e. Z = -[CH₂-]ₙ-époxy avec n, un entier de 0, avantageusement de 1, à 6).

On rappelle aussi les valeurs de Z déjà indiquées :
Z = -[CH₂]ₙ-époxy, avec n un entier de 0 à 6, avantageusement de 1 à 6 et notamment n = 4, avantageusement n = 1,
Z = -CH₂-CH(CH₃)-CH₂-CH₂-époxy,
Z = 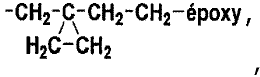 et
Z = -CH₂- C(CH₃)₂-CH₂-CH₂-époxy.

Ainsi, parmi les « épichlorhydrines » de formule Cl-Z, convenant aux époxydations souhaitées, celles répondant aux formules ci-après : Cl-[CH₂]ₙ-époxy, avec n un entier de 0 (la 2-chloro oxirane) à 6, avantageusement de 1 (l'épichlorhydrine) à 6, selon une variante préférée 1 ≤ n ≤ 4, et notamment n = 4*, selon une variante particulièrement préférée n = 1 (l'épichlorhydrine),

Cl-CH₂-CH(CH₃)-CH₂-CH₂-époxy*,

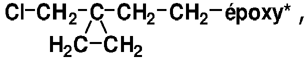

et

Cl-CH₂-C(CH₃)₂-CH₂-CH₂-époxy*

sont largement préconisées.
Celles marquées d'un astérisque sont notamment commercialement disponibles chez Enamine BB. On préconise tout particulièrement l'utilisation de l'épychlorhydrine (Cl-CH₂-époxy). Ladite épychlorhydrine est notamment commercialisée par Solvay. Elle est essentiellement synthétisée à partir de propylène. La réaction d'époxydation en cause (avec le réactif Cl-Z) se déroule en deux temps. Tout d'abord, à chaud, en présence d'un catalyseur de transfert de phase (tel, par exemple, le bromure de tétrabutylammonium (TEBAC), la substitution nucléophile des -OH par le composé Cl-Z est facilitée (par assistance à l'ion phénolate dans la phase organique). Généralement, il n'y a pas besoin d'un solvant dans la mesure où le composé Cl-Z joue le rôle de solvant. Le milieu réactionnel est ensuite refroidi et additionné de soude. Les inventeurs ont montré (voir plus loin, et notamment les exemples) que cette réaction d'époxydation peut être contrôlée et conduire, plus ou moins sélectivement, à des mélanges de composés di-, tri- et/ou tétra-époxydés. Les paramètres influents sont essentiellement la quantité de soude et la durée de la seconde partie de la réaction mise en présence de ladite soude ;
- par la succession de deux réactions, une première réaction d'allylation (allylation conventionnelle avec un halogénure d'allyle type bromure d'allyle : X-Allyl (telle qu'illustrée dans l'exemple 19 de la demande EP 3 002 333) (connue sous la dénomination de réaction de Williamson) ou allylation « verte » avec du méthyl carbonate d'allyle telle que décrite dans Chem. Commun., 2017, 53, 5175-5178 (connue sous la dénomination d'allylation de Tsuji-trost) ; le groupe allyle, précurseur du groupe Z (de la formule (I)), comportant de 2, avantageusement 3, à 8 atomes de carbone) et une seconde réaction d'époxydation proprement dite (époxydation de la double liaison introduite par ledit halogénure d'allyle ou ledit méthyl carbonate d'allyle : époxydation utilisant un oxydant, telle méta-chloroperoxy acide benzoïque ou l'oxone). Cette succession des deux réactions a été dénommée « allylation + Ep.2 » sur les figures 1A et 1B annexées. On comprend que le groupe allyle permet la transformation de fonctions alcool, hydroxy et acide en, respectivement, des fonctions -CH₂-O-Allyl, - O-Allyl et -COO-Allyl et que lesdites fonctions allylées sont toutes susceptibles d'être époxydées (les doubles liaisons, présentes en bout de chaîne ou pas, étant alors transformées en groupes époxy). On comprend que le groupe allyle époxydé correspond au groupe Z de la formule (I). L'époxydation des fonctions allyle apportées peut en fait aussi être contrôlée, en gérant la quantité d'oxydant et la durée de la réaction d'époxydation (en présence dudit oxydant), de sorte qu'elle peut être partielle (elle ne concerne alors pas toutes les doubles liaisons apportées) ou totale (elle concerne alors toutes les doubles liaisons apportée). On peut ainsi obtenir des mélanges d'au moins deux composés allylés partiellement époxydés, d'au moins un composé allylé partiellement époxydé et du composé allylé totalement époxydé (composé de formule (I) isolable). A l'issue d'une époxydation totale, facile à mettre en œuvre (avec quantité d'oxydant et durée de la réaction d'époxydation (des doubles liaisons apportées) suffisantes), on peut obtenir directement ledit composé allylé totalement époxydé (de formule (I)) ;
- par époxydation oxydante (seconde réaction (réaction d'époxydation proprement dite) indiquée ci-dessus et dénommée Ep.2 sur la figure 1B) de doubles liaisons présentes dans la formule de la molécule en cause. On pense évidemment ici aux fonctions -CH₂-CH=CH₂ du dieugénol (DEG), du dieugénol (DEG) poly(2)époxydé et du dieugénol alkylé (DEG') ainsi qu'aux fonctions -CH=CH-CH₃ du dimère de l'isoeugénol (DisoEG), du dimère de l'isoeugénol (DisoEG) poly(2)époxydé et du dimère de l'isoeugénol alkylé (DisoEG') (voir plus loin). De telles époxydations, comme indiqué ci-dessus, peuvent être totales ou partielles. Avantageusement, elles sont totales.

Pour ce qui concerne cette étape d'époxydation (qui, pour époxyder plusieurs types de fonctions d'un même dimère, peut se dérouler en plusieurs « phases » d'époxydation (par exemple, époxydation de fonctions -OH puis époxydation d'au moins une double liaison)) telle que généralement décrite ci-dessus, on peut, à toutes fins utiles, apporter les précisions ci-après, en référence à chaque dimère en cause.

L'alcool divanillique (DVA) et l'alcool divanillque alkylé (DVA') sont opportunément, dans le cadre de l'invention, époxydés selon la première ou la deuxième des méthodes d'époxydation présentées ci-dessus. Il y a respectivement, pour DVA, 2 fonctions -OH phénoliques époxydables ainsi que deux fonctions -CH₂-OH époxydables et, pour DVA', 2 fonctions -CH₂-OH époxydables. Les inventeurs ont montré qu'il est possible, à partir de l'alcool divanillique (DVA), d'obtenir, selon la première desdites méthodes, des mélanges de deux ou trois DVA pluriépoxydés choisis parmi le DVA di-époxydé (R₃ = -O-Z (-OH époxydé par Cl-Z)) et R₁ = R₂ = -CH₂-OH (non époxydé), le DVA tri-époxydé (R₃ = -O-Z (-OH époxydé par Cl-Z) et R₁ ou R₂ = - CH₂-O-Z (-CH₂-OH époxydé par Cl-Z) et le DVA tétra-époxydé (R₃ = -O-Z (-OH époxydé par Cl-Z) et R₁ = R₂ = -CH₂-O-Z (-CH₂-OH époxydé par Cl-Z)) ; et ce, avec contrôle de la composition desdits mélanges, comme indiqué ci-dessus, en jouant sur la quantité de soude ajoutée et la durée de la réaction avec ladite soude. Ceci est illustré par l'exemple 1 ci-après, dans lequel Cl-Z est l'épichlorhydrine. On rappelle toutefois la définition générale de Z ainsi que les définitions préférées, données de façon non limitative, par exemple Z=-[CH₂]ₙ-époxy. On comprend ici comment les composés de l'invention de la sous-famille a, présentée ci-dessus, peuvent être obtenus. On précise, à toutes fins utiles, que, si nécessaire, tel ou tel DVA pluriépoxydé peut être isolé (notamment par chromatographie sur colonne) d'un mélange le contenant. Comme indiqué ci-dessus, pour l'obtention du DVA poly(4)époxydé, il est aussi tout à fait possible de mettre en œuvre successivement les deux étapes d'allylation (réaction de Williamson ou de Tsuji-trost) et d'époxydation oxydante (totale). L'époxydation partielle conduit à des produits intermédiaires allylés partiellement époxydés. A partir de l'alcool divanillique alkylé (DVA'), pour l'époxydation de ses deux fonctions alcool (-CH₂-OH), on peut également procéder, comme indiqué ci-dessus, selon l'une ou l'autre des première et deuxième méthodes d'époxydation précisées ci-dessus (réaction avec l'épichlorhydrine (illustrée dans l'exemple 6 ci-après) ou équivalent (Cl-Z), ou allylation (conventionnelle ou verte, qui introduit un groupe allyle) + époxydation (proprement dite, oxydante (totale))). On comprend ici comment les composés de l'invention de la sous-famille **g,** présentée ci-dessus, peuvent être obtenus.

La divanilline (DV) a ses deux fonctions -OH phénoliques susceptibles d'être époxydées. Pour cette époxydation, on peut également procéder selon l'une ou l'autre des première et deuxième méthodes d'époxydation précisées ci-dessus (réaction avec l'épichlorhydrine (illustrée dans l'exemple 2 ci-après) ou équivalent (Cl-Z), ou allylation (conventionnelle ou verte, qui introduit un groupe allyle) + époxydation (proprement dite, oxydante (totale))). On comprend ici comment les composés de l'invention de la sous-famille **b,** présentée ci-dessus, peuvent être obtenus.

La diméthoxyhydroquinone (DMHQ) et la diméthoxyhydroquinone alkylée (DMHQ') sont opportunément, dans le cadre de l'invention, époxydées selon la première (réaction avec l'épichlorhydrine (illustrée dans l'exemple 3 ci-après) ou équivalent (Cl-Z)) ou la deuxième (allylation (conventionnelle ou verte, qui introduit un groupe allyle) + époxydation (proprement dite, oxydante) des méthodes d'époxydation présentées ci-dessus. Il y a respectivement, pour DMHQ, 4 fonctions -OH phénoliques époxydables et, pour DMHQ', 2 fonctions -OH phénoliques époxydables. Les inventeurs ont aussi montré que l'époxydation avec l'épichlorhydrine ou équivalent pouvait donner des mélanges de composition variable en composés DMHQ poly(2,3,4)époxydés, en fonction de la quantité de soude ajoutée et la durée de la réaction avec ladite soude. Dans le présent cas d'espèce, il est plus difficile de contrôler la composition desdits mélanges. Desdits mélanges, il est toutefois toujours possible d'extraire (notamment par chromatographie) sélectivement des DMHQ poly(2,3,4)époxydés. On comprend ici comment les composés de l'invention de la sous-famille **c,** présentée ci-dessus, peuvent être obtenus. Comme indiqué ci-dessus, pour l'obtention du DMHQ poly(4)époxydé, il est aussi tout à fait possible de mettre en oeuvre successivement les deux étapes d'allylation (réaction de Williamson ou de Tsuji-trost) et d'époxydation oxydante (totale). L'époxydation partielle conduit à des produits intermédiaires allylés partiellement époxydés. A partir de la diméthoxyhydroquinone alkylée (DMHQ'), pour l'époxydation de ses deux fonctions -OH phénoliques non alkylées, on peut également procéder, comme indiqué ci-dessus, selon l'une ou l'autre des première et deuxième méthodes d'époxydation précisées ci-dessus (réaction avec l'épichlorhydrine ou équivalent (Cl-Z), ou allylation (conventionnelle ou verte, qui introduit un groupe allyle) + époxydation (proprement dite, oxydante (totale))). On comprend ici comment les composés de l'invention de la sous-famille **h,** présentée ci-dessus, peuvent être obtenus.

L'acide divanillique (DVAc) et l'acide divanillique alkylé (DVAc') sont opportunément, dans le cadre de l'invention, époxydés selon la première (réaction avec l'épichlorhydrine (illustrée dans l'exemple 4 ci-après) ou équivalent (Cl-Z)) ou la deuxième (allylation (conventionnelle ou verte, qui introduit un groupe allyle) + époxydation (proprement dite, oxydante) des méthodes d'époxydation présentées ci-dessus. Il y a respectivement, pour DVAc, 2 fonctions -OH phénoliques époxydables ainsi que deux fonctions -COOH époxydables et, pour DVAc', deux fonctions -COOH époxydables. Les inventeurs ont aussi montré que l'époxydation avec l'épichlorhydrine ou équivalent pouvait donner des mélanges de composition variable en composés DVAc poly(2,3,4)époxydés, en fonction de la quantité de soude ajoutée et la durée de la réaction avec ladite soude. A partir d'un mélange ternaire ou binaire les renfermant, les composés poly(2)époxydé, poly(3)époxydé et poly(4)époxydé peuvent être isolés (notamment par chromatographie sur colonne). On comprend ici comment les composés de l'invention de la sous-famille **d,** présentée ci-dessus, peuvent être obtenus. Comme indiqué ci-dessus, pour l'obtention du DVAc poly(4)époxydé, il est tout à fait possible de mettre en oeuvre successivement les deux étapes d'allylation (réaction de Williamson ou de Tsuji-trost) et d'époxydation oxydante (totale). L'époxydation partielle conduit à des produits intermédiaires allylés partiellement époxydés. A partir de l'acide divanillique alkylé (DVAc'), pour l'époxydation de ses deux fonctions -OH phénoliques non alkylées, on peut également procéder, comme indiqué ci-dessus, selon l'une ou l'autre des première et deuxième méthodes d'époxydation précisées ci-dessus (réaction avec l'épichlorhydrine ou équivalent (Cl-Z), ou allylation (conventionnelle ou verte, qui introduit un groupe allyle) + époxydation (proprement dite, oxydante (totale))). On comprend ici comment les composés de l'invention de la sous-famille **i,** présentée ci-dessus, peuvent être obtenus.

Pour ce qui concerne le dieugénol (DEG) (respectivement le dieugénol alkylé (DEG')) et le dimère de l'isoeugénol (DisoEG) (respectivement le dimère de l'isoeugénol alkylé (DisoEG'), on conçoit que leur époxydation relève d'un même schéma réactionnel (voir la figure 1B). On propose ci-après quelques commentaires sur l'époxydation du dieugénol (DEG) et du dieugénol alkylé (DEG'). Ils sont évidemment directement transposables à l'époxydation du dimère de l'isoeugénol (DisoEG) et du dimère de l'isoeugénol alkylé (DisoEG').

Le dieugénol (DEG) possède 4 fonctions époxydables : 2 fonctions -OH phénoliques ainsi que deux doubles liaisons. Il peut être poly(2)époxydé selon la première des méthodes d'époxydation présentées ci-dessus (réaction avec l'épichlorhydrine (illustrée dans l'exemple 5 ci-après) ou équivalent (Cl-Z)) : ses 2 fonctions -OH phénoliques sont alors époxydées. Il peut aussi être poly(3) ou poly(4)époxydé selon deux variantes. Selon une première variante, le poly(2)époxydé obtenu peut lui-même être époxydé selon la troisième méthode d'époxydation ci-dessus : il s'agit de l'époxydation oxydante d'au moins une de ses doubles liaisons. La mise en œuvre de cette époxydation oxydante peut en effet être contrôlée pour époxyder une seule double liaison (époxydation partielle) ou (avantageusement) les deux doubles liaisons (époxydation totale) (voir également l'exemple 5 ci-après). Notons incidemment qu'il n'est pas exclu d'intervertir l'ordre des deux réactions d'époxydations successives décrites ci-dessus. Selon une deuxième variante, il est possible de mettre en œuvre la deuxième des méthodes d'époxydation présentées ci-dessus (allylation (conventionnelle ou verte, qui introduit un groupe allyle) + époxydation (proprement dite, oxydante (partielle ou avantageusement totale)) directement sur le dieugénol. A l'issue de l'étape d'allylation, le dimère possède quatre doubles liaisons : les deux originales du dieugénol (DEG) et les deux introduites par l'allylation. On conçoit que les quatre fonctions qui possèdent une double liaison peuvent être identiques, de type -CH₂-CH₂=CH₂ (notamment si l'allylation est mise en œuvre avec Br-CH₂-CH₂=CH₂) mais que ceci n'est en rien une obligation. L'époxydation oxydante mise en œuvre sur le dimère possédant ses quatre doubles liaisons génère alors le DEG poly(3,4)époxydé (le DEG poly(3)époxydé (époxydation partielle) et/ou le DEG poly(4)époxydé (époxydation totale)). On comprend ici comment les composés de l'invention de la sous-famille **e** (et **f**), présentée(s) ci-dessus, peuvent être obtenus. Si les fonctions -OH phénoliques du dieugénol (DEG) ont été préalablement alkylées, le dieugénol alkylé (DEG') ne présente que ses deux doubles liaisons comme fonctions épooxydables. Celles-ci sont époxydées selon la troisième méthode d'époxydation ci-dessus : l'époxydation oxydante (totale). On obtient alors le dieugénol alkylé poly(2)époxydé (DisoEG' poly(2)époxydé). On comprend ici comment les composés de l'invention de la sous-famille **j** (et **k**), présentée(s) ci-dessus, peuvent être obtenus. On note incidemment que l'époxydation des doubles liaisons du dimère de l'isoeugénol (DisoEG), alkylé ou non, conduit assurément à des composés de l'invention dont au moins certaines des fonctions époxy ne se situent pas en bout de chaîne.

A la considération de la description du procédé ci-dessus, et tout particulièrement de la deuxième des méthodes d'époxydation présentées ci-dessus (allylation (conventionnelle ou verte, qui introduit un groupe allyle) + époxydation (proprement dite, oxydante (partielle))), ont été divulgués des produits intermédiaires :
- les produits intermédiaires allylés de type DVA allylé (DVA poly(4)allylé), DVA alkylé allylé (DVA' poly(2)allylé), de type DV allylée (DV poly(2)allylée), de type DMHQ allylée (DMHQ poly(4)allylée), de type DMHQ alkylée allylée (DMHQ' poly(2)allylée), de type DVAc allylé (DVAc poly(4)allylé), de type DVAc alkylé allylé (DVAc' poly(2)allylé), de type DEG allylé (DEG poly(2)allylé = un composé biphényle tétra-allylé), de type DisoEG allylé (DisoEG poly(2)allylé = un autre composé biphényle tétra-allylé). A toutes fins utiles, on rappelle que le groupe allyle, précurseur du groupe Z (de la formule (I)), comporte de 2, avantageusement 3, à 8 atomes de carbone. Ledit groupe allyle consiste avantageusement en le groupe -[CH₂]ₘ-CH=CH₂ (avec m un entier de 0, avantageusement de 1, à 6), très avantageusement en le groupe -CH₂-CH=CH₂ ; et
- certains desdits produits intermédiaires allylés partiellement époxydés, i.e. dont toutes les fonctions allylées n'ont pas été époxydées, tel par exemple ledit DVA (ou DMHQ ou DVAc) poly(4)allylé dont seulement 1, 2 ou 3 des fonctions allyles ont été époxydées, ledit DV (ou DVA' ou DMHQ' ou DVAc') poly(2)allylé dont seulement 1 des fonctions allyles a été époxydée, ledit DEG poly(2)allylé ou DisoEG poly(2)allylé dont seulement 1 des fonctions allyles a été époxydée.

Les composés biphényles pluriépoxydés de formule (I) préparés selon le procédé décrit ci-dessus peuvent être incorporés dans des résines époxy thermodurcissables. Lesdites résines sont susceptibles de renfermer en outre des additifs et/ou diluant(s). On rappelle que lesdits composés de formule (I) - isolés ou en mélange - conviennent tout particulièrement comme substituts du DGEBA (voir l'introduction du présent texte ainsi que les exemples ci-après). Lesdites résines sont thermodurcissables de par la présence des fonctions époxy des composés de formule (I) (avantageusement localisées en bout de chaîne, très avantageusement situées en bout de chaînes linéaires, de préférence situées en bout de courtes chaînes linéaires (voir ci-dessus 1 ≤ n ≤ 4 et de façon particulièrement préféré n = 1), chacun desdits composés de formule (I) renfermant au moins deux fonctions époxy. Ces fonctions époxy sont, de façon connue *per se,* réticulables sous l'action de la chaleur et en présence d'un durcisseur (agent de poymérisation réticulante ou agent de réticulation (bifonctionnel)). L'homme du métier connait de tels agents, largement utilisés avec les résines époxy de l'art antérieur (notamment celles à base de DGEBA). On cite ici, de façon nullement limitative, le diaminodiphényl sulfone (DDS), l'isophorone diamine (IPDA), le dicyandiamide, la 4,4-méthylène-bis(2-isopropyl-6-méthylaniline) (notamment commercialisée par Loza Ltd, sous la dénomination commerciale Lonzacure^{®} M-MIPA), et la 4,4'-méthylène-bis(2,6-diisopropylamineaniline (notamment commercialisée par Loza Ltd, sous la dénomination commerciale Lonzacure^{®} M-DIPA).

Les résines époxy thermodurcissables mentionnées ci-dessus peuvent être traitées thermiquement, en présence d'au moins un thermodurcisseur, pour donner des résines époxy thermodurcies obtenues (= les polyépoxydes obtenus). On a compris que les composés obtenus par le procédé de l'invention ont été conçus comme précurseurs desdites résines époxy thermodurcies, comme précurseurs desdits polyépoxydes.. Lesdits composés, associés à au moins un durcisseur, conviennent parfaitement comme matériau époxy (substituts avantageux de la DGEBA) pour des applications dans les domaines des adhésifs et des composites.

L'invention est maintenant illustrée par les exemples ci-après et les figures annexées.

Les figures 1A et 1B sont des schémas illustrant le procédé de l'invention tel que décrit ci-dessus.

La figure 2 montre le résultat d'une chromatographie menée sur un mélange de pluriGEDVA (voir l'exemple 1).

Les figures 3A à 13A sont des spectres RMN ¹H de composés (ou mélanges de composés) de l'invention préparés dans les exemples ci-après ; les figures 3B à 13B sont des spectres RMN ¹³C de composés de l'invention préparés dans les exemples ci-après (il n'y a toutefois pas de figure 7B).

### I. Préparation des composés de formule (I), dans laquelle R₃_= O-CH₂-époxy, dérivés de la vanilline, du vanillate de méthyle ou de l'euaénol (isolés et/ou en mélanne)

### Exemple 1

### A1. Synthèse de composés de formule (I) à partir de l'alcool divanillique (DVA)

Les différentes étapes du schéma réactionnel ci-dessous ont successivement été mises en oeuvre.

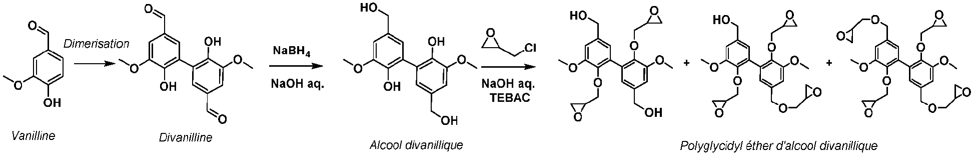

### Synthèse de la divanilline (DV)

Pour la préparation de la divanilline, on a procédé selon le mode opératoire décrit dans l'exemple 1 de la demande de brevet EP 3 002 333. Plus précisément, on a procédé comme suit.

La vanilline (20 g) (celle utilisée, commercialisée par la société Acros, n'était pas biosourcée. A toutes fins utiles, on indique qu'aurait pu être utilisée celle, biosourcée, commercialisée par la société Borregaard) a été solubilisée dans de l'acétone (160 mL) et une solution tampon d'acétate (1,5 L, préparée à partir de 2,63 g d'acide acétique et 8,4 g d'acétate de sodium). Au mélange résultant, a été ajoutée la laccase issue de *Trametes Versicolor* (170 mg). Pour être recyclée sous forme active, ladite laccase a besoin d'oxygène. Le milieu réactionnel a donc été laissé sous agitation avec un bullage d'air constant pendant 24 heures. La divanilline a ensuite été récupérée par filtration de la solution tampon sur filtre Büchner. Le filtrat a été récupéré et réutilisé pour d'autres réactions de dimérisation.

### Purification de la divanilline (DV) synthétisée

Des traces de vanilline étaient susceptibles d'être présentes dans la divanilline récupérée. Pour les éliminer, ladite divanilline a été solubilisée dans une solution aqueuse de NaOH (200 mL à 0,5 M ; quelques gouttes de solution 5M ont été opportunément ajoutées pour faciliter la solubilisation). Un large volume d'éthanol (600 mL) a ensuite été ajouté à la solution ainsi qu'une solution aqueuse d'acide chlorhydrique (115 mL à 2 M) jusqu'à atteindre, pour le mélange, un pH = 3. En effet, la divanilline et la vanilline sont toutes les deux solubles à pH basique dans l'éthanol. En revanche, la divanilline n'est pas soluble dans l'éthanol à pH acide, contrairement à la vanilline. L'ajout de l'acide permet donc de séparer les deux produits par précipitation de la divanilline.

Le produit obtenu a été filtré, puis séché à l'étuve, afin d'enlever toute trace de solvant. Les opérations de synthèse et purification ont été répétée. Le rendement était à chaque fois d'environ 95 %.

On a confirmé l'obtention de la divanilline (DV) par spectroscopie RMN : 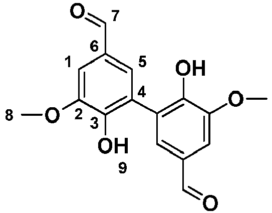
**RMN ¹H** (400 MHz, DMSO-d6, δ (ppm) : δ 9.69 (s, H₇), 7.57 (d, H₁), 7.16 (d, H₅), 3.76 (s, H₈).
**RMN ¹³C** (400 MHz, DMSO-d6, δ (ppm)) : δ 191.62 (s, C₇), 150.88 (s, C₃), 148.61 (s, C₂), 128.64 (s, C₆), 128.21 (s, C₄), 125.02 (s, C₅), 109.6 (s, C₁), 56.25 (C₈).

### Synthèse de l'alcool divanillique (DVA)

Pour la préparation de l'alcool divanillique, on a procédé selon le protocole décrit dans l'exemple 8 de la demande de brevet EP 3 002 333. Plus précisément, on a procédé comme suit.

La divanilline purifiée (20 g) a été réduite avec du borohydrure de sodium (NaBH₄) afin de former l'alcool divanillique. Pour cela, elle a été solubilisée dans de la soude à 0,5 M (180 mL ; quelques gouttes de solution 5M ont été opportunément ajoutées pour faciliter la solubilisation). Puis le NaBH₄ (3 g) a été incorporé et le mélange a été maintenu sous agitation jusqu'à complète dissolution. Au bout d'une heure d'agitation, la réaction a été arrêtée par ajout, goutte à goutte, d'une solution aqueuse d'acide chlorhydrique (160 mL à 2M) jusqu'à atteindre un pH = 3. L'alcool divanillique a alors précipité. Il a été récupéré par filtration. Le produit ainsi récupéré a été séché à l'étuve. La synthèse a été répétée. Le rendement était à chaque fois d'environ 80 %.

On a confirmé l'obtention de l'alcool divanillique (DVA) par spectroscopie RMN : 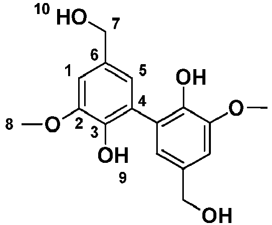
**RMN ¹H** (400 MHz, DMSO-d6, δ (ppm)) : δ 8.22 (s, H₉), 6.88 (d, H₁), 6.67 (d, H₅), 5.01 (t, H₁₀), 4.41 (d, H₇), 3.82 (s, H₈).
**RMN ¹³C** (400 MHz, DMSO-d6, δ (ppm)) : δ 147.94 (s, C₃), 142.77 (s, C₂), 133.08 (s, C₆), 125.92 (s, C₄), 121.83 (s, C₅), 109.50 (s, C₁), 63.38 (s, C₇), 56.25 (s, C₈).

### Synthèse de composés de formule (I) (prépolymères pluriépoxydés)

La dernière étape a consisté à époxyder l'alcool divanillique (DVA) avec de l'épichlorhydrine et a conduit à l'obtention de mélanges de différents polyglycidyl éthers d'alcool divanillique. Elle a été mise en œuvre dans des conditions différentes pour l'obtention de mélanges différents.

Les composés pluriépoxydés susceptibles d'être obtenus et dont la présence a été confirmée (quantitativement et qualitativement) étaient ceux dont la formule est indiquée dans le schéma réactionnel ci-dessus, à savoir :
- le diglycidyl éther de l'alcool divanillique **(DiGEDVA),**
- le triglycidyl éther de l'alcool divanillique **(TriGEDVA),** et
- le tétraglycidyl éther de l'alcool divanillique **(TétraGEDVA).**

a) On décrit ci-après les conditions expérimentales mises en œuvre pour l'obtention un mélange de 25 % de TriGEDVA et de 75 % de TétraGEDVA (% en masse).

Dans un premier temps, le DVA (20 g) a été mis en présence d'épichlorhydrine (100 mL) et de bromure de tétrabutylammonium (TEBAC) (2 g). Le TEBAC est un agent de transfert de phase qui permet au phénol de réagir avec l'épichlorhydrine, introduite en excès pour former un di-époxyde. Le mélange réactionnel a été laissé sous agitation à 80 °C pendant 1 h 30 ; puis il a été refroidi à température ambiante.

Par la suite, une solution aqueuse de soude (NaOH) (160 mL à 10 M : 10 NaOH eq./OH) a été ajoutée. L'ajout de la base a permis de fermer les époxydes ouverts mais également de déprotoner les alcools benzyliques qui, à leur tour, sont époxydés par substitution nucléophile avec l'épichlorhydrine. La solution a ensuite été agitée mécaniquement pendant 20 h dans un bain d'eau froide.

A la fin de la réaction, du dichlorométhane (DCM) (300 mL) a été ajouté au milieu réactionnel pour faire précipiter les sels (NaCl). Les phases liquides ont été séparées du milieu réactionnel et les sels rincés avec 100 mL de DCM. Les phases liquides ont été rassemblées et la phase aqueuse a été extraite avec 2x50 mL de DCM. Les différentes phases organiques ont été rassemblées et lavées avec 100 mL d'eau. La phase organique a été concentrée en utilisant un évaporateur rotatif et l'épichlorhydrine a finalement été évaporée sous vide. Le rendement était quantitatif. La proportion de composés di-, tri-, et tétra-époxydés a été quantifiée par HPLC (chromatographie en phase liquide à haute performance). L'appareil utilisé était un SpectraSYSTEM^{®}, monté d'une colonne Phenomenex 5µ C18 100A. Le détecteur utilisé était un système SpectraSYSTEM^{®} UV2000 de Thermo Séparation Products. Les analyses ont été réalisées avec un éluant composé d'acétonitrile et d'eau en proportion isocratique 50/50.

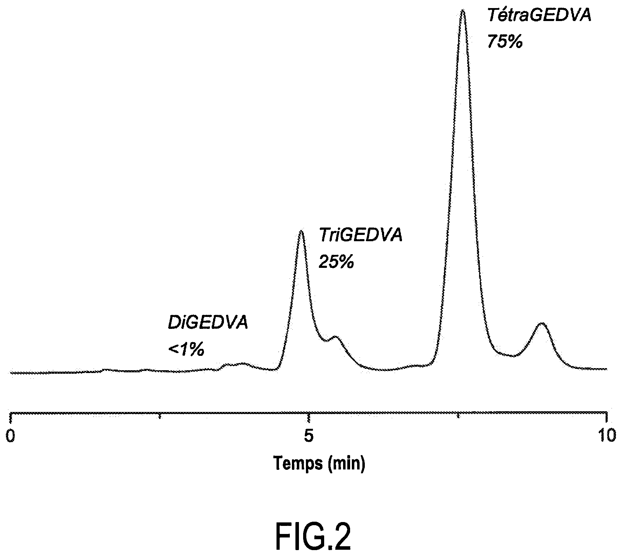

La figure 2 annexée montre le chromatographe obtenu.
b) On a reproduit (en tous points) la procédure décrite au point a) ci-dessus mais avec ajout d'une solution aqueuse de NaOH (50 mL à 5 M) et avec agitation mécanique pendant seulement 1 h. On a alors obtenu un mélange de 80 % de DiGEDVA, de 15 % de TriGEDVA et de 5 % de TétraGEDVA (% en masse).
c) On a reproduit (en tous points) la procédure décrite au point a) ci-dessus mais avec ajout d'une solution aqueuse de NaOH (50 mL à 5 M) et avec agitation mécanique pendant seulement 8 h. On a alors obtenu un mélange de 35 % de DiGEDVA, 50 % de TriGEDVA et 15 % de TétraGEDVA (% en masse).

Pour obtenir, isolément, ces différents composés de formule (I) (di-, tri- et téra-époxydés), une étape de purification par chromatographie flash ou instantanée, sur un appareil Grace Reveleris^{®}, équipé d'une cartouche de sillice et d'un dététecteur UV, a été mise oeuvre sur les mélanges, en utilisant un gradient de solvant dichlorométhane/méthanol de 99/1 à 90/10 (en volume) pendant 30 minutes.

On a confirmé l'identité desdits composés de formule (I) par spectroscopie RMN : 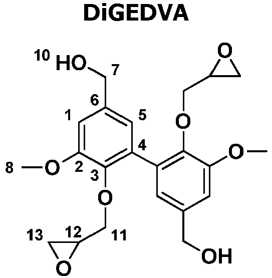
**RMN ¹H** (400MHz, DMSO-d6, δ (ppm)) : δ 7.0 (d, H₁), 6.71 (d, H₅), 5.16 (t, H₁₀), 4.47 (d, H₇), 3.88 (m, H₁₁), 3.83 (s, H₈), 3.74 (m, H_{11b}), 2.95 (m, H₁₂), 2.6 (t, H₁₃), 2.36 (t, H_{13b}).
**RMN ¹³C** (400MHz, DMSO-d6, δ (ppm)) : δ 152.33 (s, C₃), 144.47 (s, C₂), 138.26 (s, C₆), 132.59 (s, C₄), 120.86 (s, C₅), 110.79 (s, C₁), 74.22 (s, C₁₁), 63.14 (s, C₇), 56.18 (s, C₈), 50.53 (s, C₁₂), 43.97 (s, C₁₃).

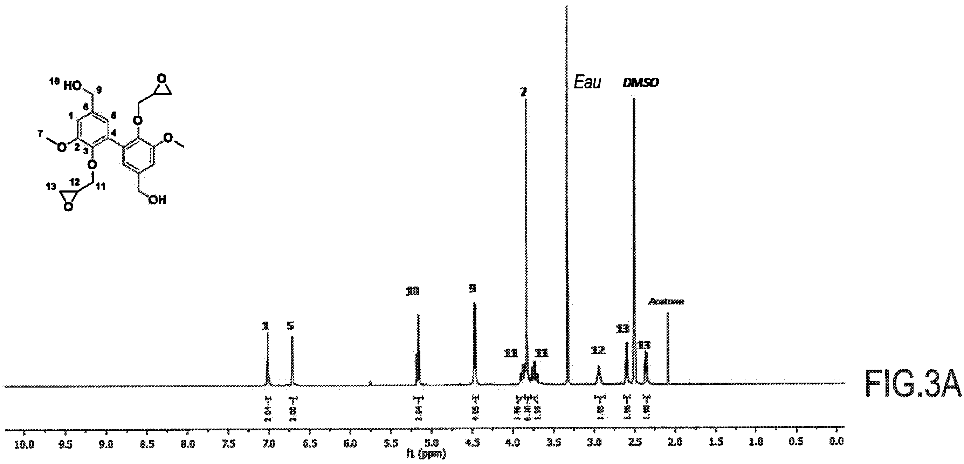

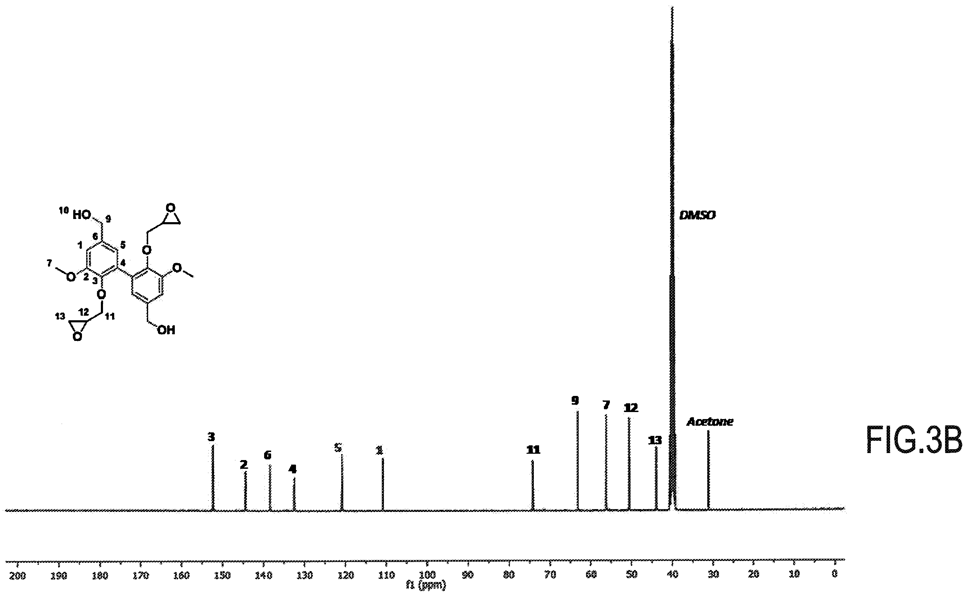

Les spectres sont respectivement montrés sur les figures 3A et 3B. 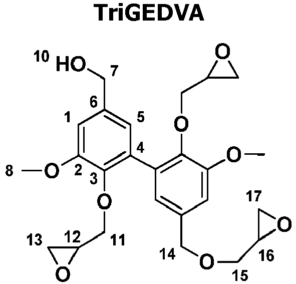
**RMN ¹H** (400MHz, DMSO-d6, δ (ppm)) : δ 7.01 (d, H₁), 6.75 (d, H₅), 5.18 (t, H₁₀), 4.47 (d, H₇ H₁₄), 3.92 (m, H₁₁), 3.84 (s, H₈), 3.76 (m, H_{11b}), 3.69 (m, H₁₅), 3.29 (m, H_{15b}), 3.14 (m, H₁₆), 2.97 (m, H₁₂), 2.72 (m, H₁₇), 2.6 (m, H₁₃), 2.5 (m, H_{17b}), 2.36 (m, H_{13b}).
**RMN ¹³C** (400MHz, DMSO-d6, δ (ppm)) : δ 152.02 (s, C₃), δ 151.89 (s, C₃), 144.38 (s, C_{2'}), 143.68 (s, C₂), 138.12 (s, C_{6'}), 133.39 (s, C₆), 132.06 (s, C_{4'}), 131.76 (s, C₄), 121.78 (s, C₅), 120.26 (s, C₅), 111.55 (s, C_{1'}), 110.46 (s, C₁), 73.85 (s, C₁₄), 71.81 (s, C₁₅), 70.79 (s, C₁₁), 62.67 (s, C₇), 55.90 (s, C₈), 50.42 (s, C₁₂), 50.16 (s, C₁₆), 43.42 (s, C₁₃ C₁₇).

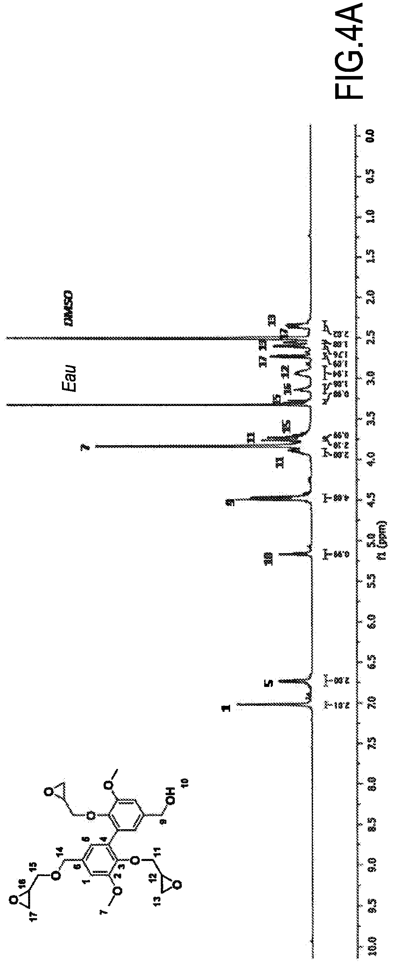

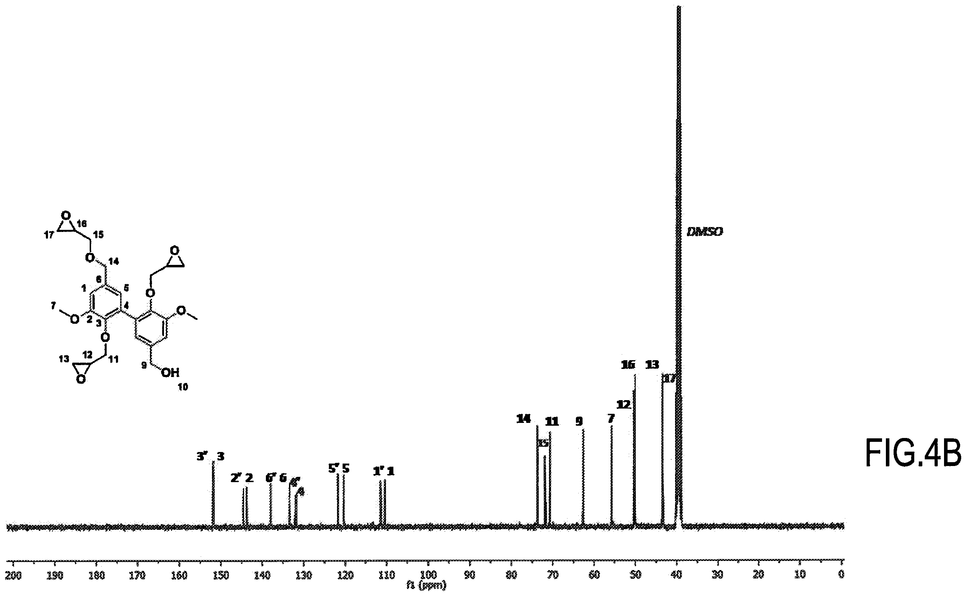

Les spectres sont respectivement montrés sur les figures 4A et 4B. 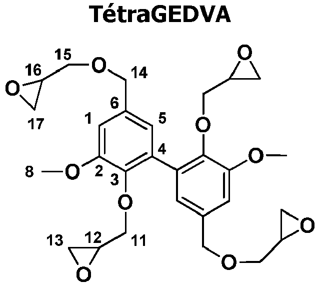
RMN ¹H (400MHz, DMSO-d6, δ (ppm)): δ 7.02 (d, H₁), 6.76 (d, H₅), 4.50 (s, H₁₄), 3.92 (m, H₁₁), 3.86 (s, H₈), 3.76 (m, H_{11b}), 3.70 (m, H₁₅), 3.28 (m, H_{15b}), 3.14 (m, H₁₆), 2.97 (m, H₁₂), 2.73 (m, H₁₇), 2.60 (m, H₁₃), 2.55 (m, H_{17b}), 2.35 (m, H_{13b}).
RMN ¹³C (400MHz, DMSO-d6, δ (ppm)): δ 152.10 (s, C₃), 144.51 (s, C₂), 133.51 (s, C₆), 131.81 (s, C₄), 121.83 (s, C₅), 111.52 (s, C₁), 73.77 (s, C₁₄), 71.90 (s, C₁₅), 63.14 (s, C₁₁), 55.79 (s, C₈), 50.30 (s, C₁₂), 50.03 (s, C₁₆), 43.44 (s, C₁₃ C₁₇).

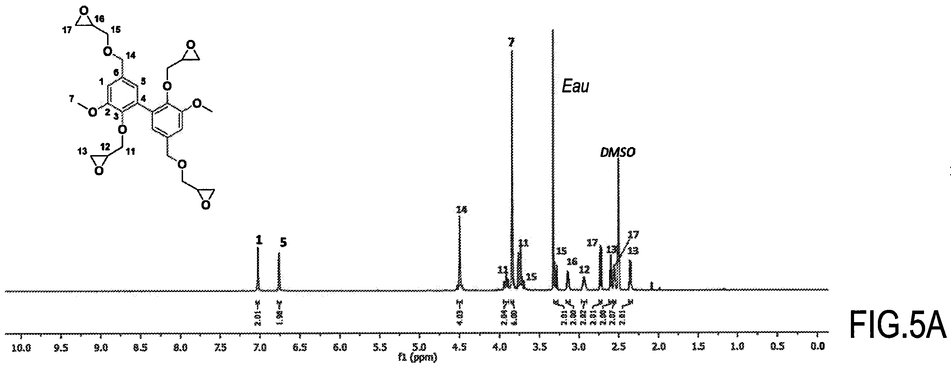

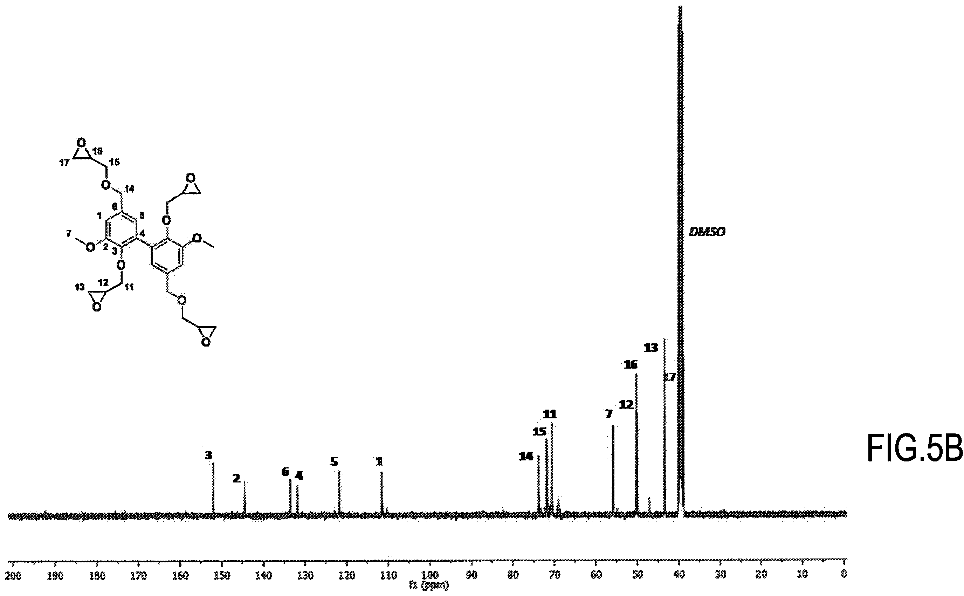

Les spectres sont respectivement montrés sur les figures 5A et 5B.

### B1. Polyépoxydes obtenus à partir desdits composés de formule (I) (isolés et/ou en mélange) (prépolymères pluriépoxydés)

Pour la polymérisation (polymérisation réticulante) des composés de l'invention obtenus dans le cadre de cet exemple (DiGEDVA, TriGEDVA et TétraGEDVA, isolément et en mélange : TriGEDVA (25 %) + TétraGEDVA (75 % (voir ci-dessus)), on a utilisé, à titre de durcisseur, le diaminodiphényl sulfone (le Diamino-Diphenyl Sulfone (DDS). Celui-ci répond à la formule :

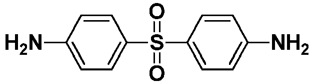

Ledit durcisseur a été utilisé en le ratio stoechiométrique : époxy/amine = 2/1 et la réaction a été mise en œuvre à 180 °C pendant 2 h.

La même réaction a été mise en oeuvre avec le diglycidyléther de bisphénol A (DGEBA ; prépolymère de l'art antérieur obtenu à partir du Bisphénol A (BPA)).

Les polyépoxydes obtenus ont notamment été évalués par leur température de transition alpha (elle peut être assimilée à une température de transition vitreuse. Elle a été déterminée par DMA (Dynamic Mechanical Analysis)), par leur taux de coke résiduel après dégradation à 900°C (Char900 ; déterminé par TGA (ThermoGravimetric Analysis)) et par leur module d'Young. Les résultats figurent dans le tableau 1 ci-après.

**Tableau 1**

| **Prépolymères précurseurs du polyépoxyde** | **Ta (°C)** | **Char900 (%)** | **Module d'Young (GPa)** |
|---|---|---|---|
| **DGEBA** | 203 | 18 | 1,5 |
| **DiGEDVA** | 206 | 51 | 1,5 |
| **TriGEDVA** | 254 | 49 | 1,4 |
| **TétraGEDVA** | 312 | 48 | 1,8 |
| **TriGEDVA (25 %) + TétraGEDVA (75 %)** | 280 | 50 | 1,4 |

Les chiffres dudit Tableau 1 confirment l'intérêt des composés de l'invention.

L'aromaticité plus élevée des polyépoxydes de l'invention renforce leur structure et conduit à des réseaux avec des Tα de 206 à 312°C et des modules de Young de 1,4 à 1,8 GPa.

La masse résiduelle à 900°C est d'environ 50 % pour les polyépoxydes de l'invention pour seulement 18 % pour le polyépoxyde de l'art antérieur. Ceci est très intéressant dans la mesure où une haute valeur de masse résiduelle indique de bonnes propriétés de retard à la flamme des matériaux. Des tests de flamme ont été réalisés sur divers échantillons pour vérifier cette affirmation. Les époxydes obtenus à partir du DGEBA, au contact direct de la flamme, brûlent et la combustion augmente et se propage rapidement à tout l'échantillon. Au contraire, pour les époxydes de l'invention, la combustion s'arrête rapidement du fait de la formation d'une couche protectrice de coke à la surface des matériaux.

### Exemple 2

### A2. Synthèse d'un composé de formule (I) à partir de la divanilline (DV)

Les différentes étapes du schéma réactionnel ci-dessous ont été successivement mises en oeuvre.

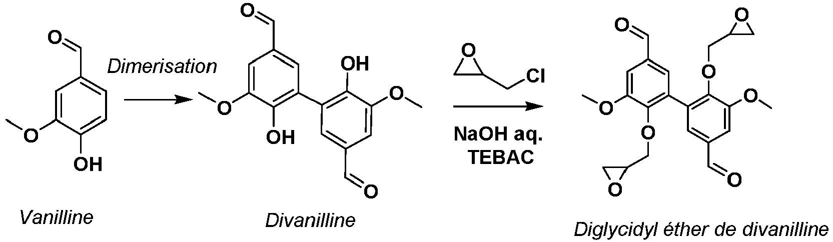

### Synthèse de la divanilline (DV)

On a procédé comme explicité ci-dessus (selon le protocole de l'exemple 1 de la demande de brevet EP 3 002 333).

### Synthèse d'un composé de formule (I) (prépolymère pluriépoxydé) : le diglycidyléther de divanilline (DiGEDV)

Les fonctions aldéhydes de la divanilline ont été conservées. Les fonctions OH ont été époxydées avec de l'épichlorhydrine. On a procédé comme suit.

3 g de divanilline (10 mmol) ont été dissous dans 15 mL d'épichlorhydrine. 0,3 g de bromure de tétrabutylammonium (TEBAC) (0,95 mmol) ont été ajoutés et le mélange résultant a été agité à 80 °C pendant 12 h. 8 mL d'une solution de NaOH (5 M) (40 mmol) ont ensuite été ajoutés et le mélange a été agité à température ambiante pendant 1h30. Le produit a finalement été extrait avec du dichlorométhane et lavé avec de l'eau. Le dichlorométhane et l'épichlorhydrine ont été éliminés de la phase organique en utilisant un évaporateur rotatif. Le rendement était de 90%. Une purification a été opportunément réalisée par chromatographie flash en utilisant un gradient de solvant dichlorométhane/méthanol (de 99/1 à 90/10 (en volume) pendant 30 minutes).

On a confirmé l'identité du composé de formule (I) obtenu par spectroscopie RMN : 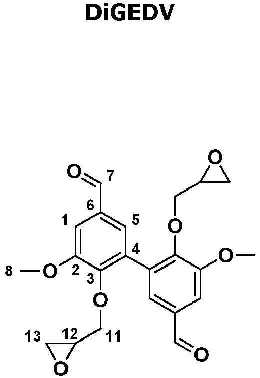
**RMN ¹H** (400MHz, DMSO-d6, δ (ppm)) : δ 9.94 (s, H₇), 7.60 (d, H₁), 7.48 (s, H₅), 3.93 (s, H₈), 7.0 (s, H₅), 6.71 (s, H₁), 5.16 (t, H₁₀), 4.47 (d, H₇), 4.18 (m, H₁₁), 3.95 (s, H₈), 3.85 (m, H_{11b}), 2.98 (m, H₁₂), 2.61 (t, H₁₃), 2.40 (t, H_{13b}).
**RMN ¹³C** (400MHz, DMSO-d6, δ (ppm)) : δ 191.79 (s, C₇), δ 152.48 (s, C₃), 150.67 (s, C₂), 131.84 (s, C₆), 131.29 (s, C₄), 126.40 (s, C₅), 111.48 (s, C₁), 74.24 (s, C₁₁), 63.14 (s, C₇), 55.89 (s, C₈), 50.12 (s, C₁₂), 43.44 (s, C₁₃).

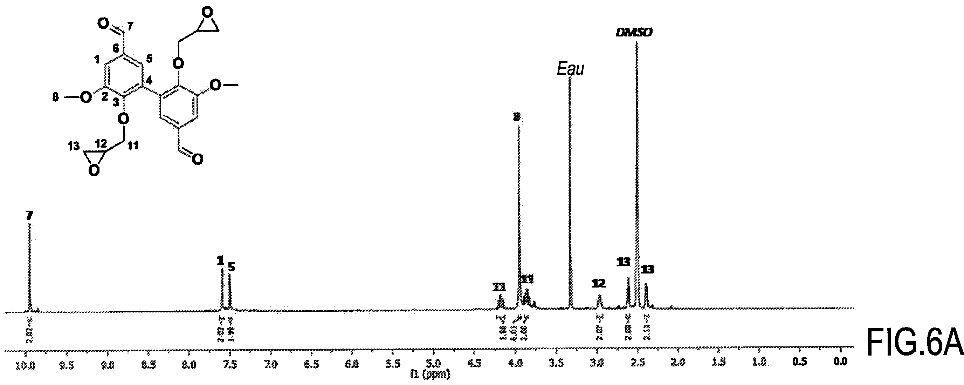

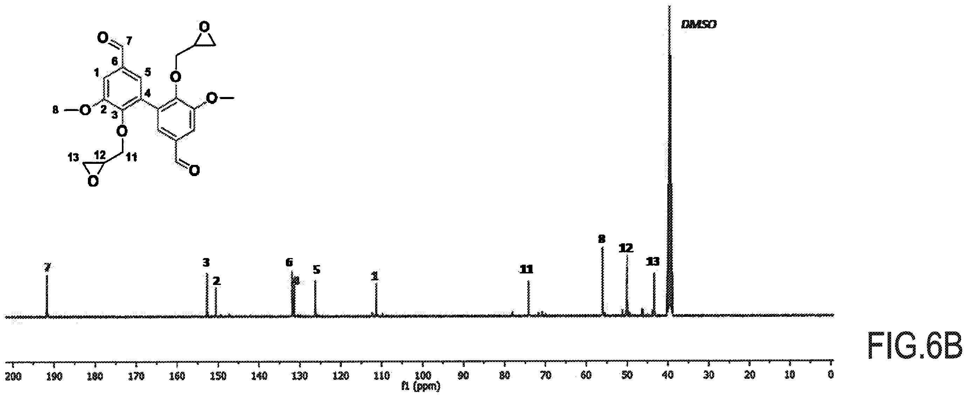

Les spectres sont respectivement montrés sur les figures 6A et 6B.

### B2. Polyépoxyde obtenu à partir dudit composé de formule (I) (prépolymère pluriépoxydé)

Pour la polymérisation du composé de l'invention obtenu dans le cadre de cet exemple (le diglycidyl éther de divanilline : DiGEDIV), on a également utilisé, à titre de durcisseur, le diaminodiphényl sulfone (DDS), dont la formule chimique a été rappelée ci-dessus.

Ledit durcisseur a été utilisé en le ratio stoechiométrique : époxy/amine = 2/1 et la réaction a été mise en œuvre à 180 °C pendant 2 h.

Comme ci-dessus, on s'est intéressé à la température de transition vitreuse (déterminée par DMA (Dynamic Mechanical Analysis)) et au taux de coke résiduel après dégradation à 900°C (déterminé par TGA (ThermoGravimetric Analysis)) du polyépoxyde préparé. Les résultats figurent dans le tableau 2 ci-après.

**Tableau 2**

| **Prépolymère précurseur du polyépoxyde** | **Ta (°C)** | **Char900 (%)** |
|---|---|---|
| **DiGEDV** | 180 | 54 |

Les chiffres dudit Tableau 2 confirment l'intérêt des composés de l'invention.

### Exemple 3

### A3. Synthèse de composés de formule (I) (en mélange) à partir de la diméthoxyhydroquinone

Les différentes étapes du schéma réactionnel ci-dessous ont été successivement mises en œuvre.

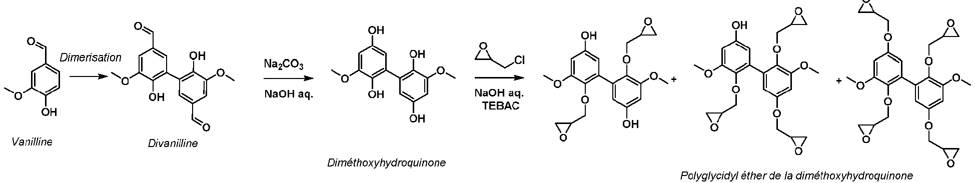

### Synthèse de la divanilline (DV)

On a procédé comme explicité ci-dessus (selon le protocole de l'exemple 1 de la demande de brevet EP 3 002 333).

### Synthèse de la diméthoxyhydroquinone (DMHQ)

6 mmol de divanilline (≈ 1 g) ont été dissoutes dans 10 ml de NaOH (0,5 M). 7 mmole de percarbonate de sodium ont ensuite été ajoutées lentement. Le mélange a ensuite été agité à température ambiante pendant 12 h. A l'issue de cette agitation, la solution a été acidifiée avec une solution aqueuse d'HCl (2 M) jusqu'à atteindre un pH = 3. La phase aqueuse a alors été extraite avec de l'acétate d'éthyle. Les phases organiques ont été recueillies et lavées à l'eau puis séchées sur du sulfate de magnésium (MgSO₄). L'acétate d'éthyle a ensuite été éliminé sous vide en utilisant un évaporateur rotatif. Une purification supplémentaire a été réalisée par chromatographie flash en utilisant un solvant à gradient de dichlorométhane/méthanol (de 99/1 à 90/10 (en volume) pendant 30 minutes). Le rendement était inférieur à 50 %. Il convient de noter que la synthèse mise en œuvre (répétée plusieurs fois) n'avait pas été optimisée, ni en vue de l'obtention d'un rendement plus élevé, ni en vue de l'obtention d'un composé pur.

L'identité de la DMHQ a été confirmée par spectroscopie RMN : 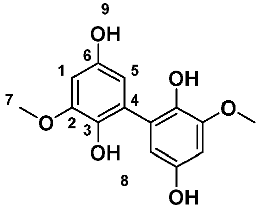
**RMN ¹H** (400 MHz, DMSO-d6, δ (ppm)) : δ 8.79 (s, H₈), 7.76 (s, H₉), 6.38 (d, H₁), 6.15 (d, H₅), 3.75 (s, H₇).
**RMN ¹³C** (400 MHz, DMSO-d6, δ (ppm)) : δ 149.76 (s, C₃), 148.62 (s, C₂), 135.77 (s, C₆), 126.65 (s, C₄), 108.35 (s, C₅), 99.35 (s, C₃), 55.65 (s, C₇).

### Synthèse de composés de formule (I) (prépolymères pluriépoxydés) : polyglycidyl éthers de la diméthoxyhydroquinone

0,5 g de diméthoxyhydroquinone (telle que préparée ci-dessus) ont été dissous dans 10 ml d'épichlorhydrine. 0,05 g de bromure de tétrabutylammonium (TEBAC) ont été ajoutés et le mélange résultant a été agité à 80 °C pendant 20 h. 5 ml d'une solution de NaOH (5 M) (40 mmol) ont ensuite été ajoutés et le nouveau mélange résultant a été agité à température ambiante pendant 24 h. Le mélange obtenu a été extrait avec du dichlorométhane et lavé avec de l'eau. Le dichlorométhane et l'épichlorhydrine ont été éliminés de la phase organique en utilisant un évaporateur rotatif. La synthèse mise en œuvre n'était pas optimisée.

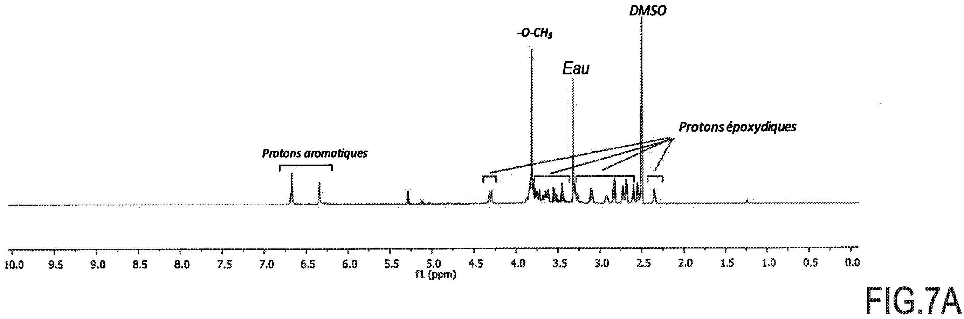

On a ainsi obtenu un mélange de polyglycidyl éthers de diméthoxyhydroquinone, lesdits polyglycidyl éthers présents en proportions variables, non évaluées. Ce mélange a été analysé par spectroscopie RMN ¹H. Le spectre obtenu est montré sur la figure 7A. Ledit spectre confirme assurément la présence de plusieurs fonctions époxy.

### B3. Polyépoxyde obtenu à partir desdits composés de formule (I) (prépolymères pluriépoxydés)

Pour la polymérisation du mélange de composés de l'invention obtenu dans le cadre de cet exemple, on a également utilisé, à titre de durcisseur, le diaminodiphényl sulfone (DDS), dont la formule chimique a été rappelée ci-dessus.

Ledit durcisseur a été utilisé en le ratio stoechiométrique : époxy/amine = 2/1. Dans cet exemple, le polyépoxyde n'a pas été successivement préparé puis analysé par DMA (pour détermination de sa Tg). Il a été généré, en petite quantité (quelques mg), lors de la mise en œuvre d'une calorimétrie différentielle à balayage (DSC), pour détermination de sa Tg.

Le taux de coke résiduel, après dégradation à 900°C, déterminé par TGA (ThermoGravimetric Analysis), l'a été sur cette petite quantité générée lors de l'analyse DSC. Les résultats figurent dans le tableau 3 ci-après.

**Tableau 3**

| **Prépolymère précurseur du polyépoxyde** | **Tg (°C)** | **Char900 (%)** |
|---|---|---|
| **PolyGEDMHQ** | 212 | 41 |

Les chiffres dudit Tableau 3 confirment l'intérêt des composés de l'invention.

### Exemple 4

### A4. Synthèse d'un composé de formule (I) à partir du vanillate de méthyle

Les différentes étapes du schéma réactionnel ci-dessous ont été successivement mises en oeuvre.

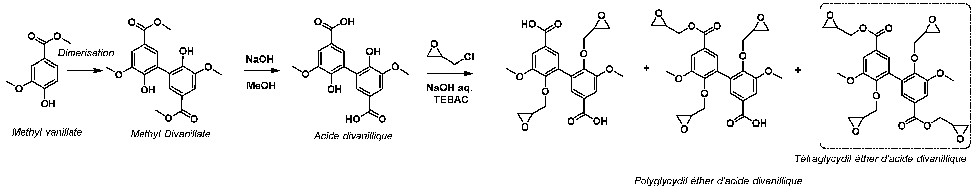

### Synthèse du divanillate de méthyle

Pour la préparation du divanillate de méthyle, à partir du vanillate de méthyle (distribué par la société Sigma-Aldrich), on a procédé selon un mode opératoire tout à fait similaire à celui décrit pour la préparation de la divanilline au point A1 ci-dessus (i.e. selon le mode opératoire décrit dans l'exemple 4 de la demande de brevet EP 3 002 333).

On a confirmé l'obtention du divanillate de méthyle par spectroscopie RMN : 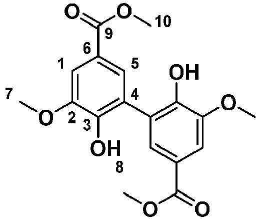
**RMN ¹H** (400 MHz, DMSO-d6, δ (ppm)) : δ 9.51 (s, H₈), 7.46 (d, H₁), 7.45 (d, H₅), 3.90 (s, H₇), 3.80 (s,H₁₀).
**RMN ¹³C** (400 MHz, DMSO-d6, δ (ppm)) : δ 166.09 (s, C₉), 148.88 (s, C₃), 147.47 (s, C₂), 125.40 (s, C₅), 124.36 (s, C₆), 119.48 (s, C₄), 110.92 (s, C₁), 56.01 (s, C₇), 51.79 (s,C₁₀).

### Synthèse de l'acide divanillique (DVAc)

On a procédé, pour cette saponification, selon le mode opératoire décrit dans l'exemple 13 de la demande de brevet EP 3 002 333.

On a confirmé l'obtention de l'acide divanillique (DVAc) par spectroscopie RMN : 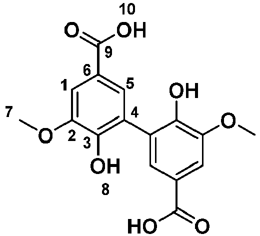
**RMN ¹H** (400 MHz, DMSO-d6, δ (ppm) : δ 9.39 (s, H₈), 7.45 (d, H₁), 7.41 (d, H₅), 3.89 (s, H₇).
**RMN ¹³C** (400 MHz, DMSO-d6, δ (ppm)) : δ 167.18 (s, C₉), 148.36 (s, C₃), 147.22 (s, C₂), 125.44 (s, C₆), 124.19 (s, C₄), 120.44 (s, C₅), 111.05 (s, C₁), 55.89 (s, C₇).

### Synthèse d'un composé de formule (I) (prépolymère pluriépoxydé) : le tétraglycidyléther de l'acide divanilique (TétraGEDVAc)

0,5 g d'acide divanillique ont été dissous dans 10 mL d'épichlorhydrine. 0,05 g de bromure de tétrabutylammonium (TEBAC) ont été ajoutés et le mélange résultant a été agité à 80 °C pendant 2 h. 5 mL d'une solution de NaOH (5 M) (40 mmol) ont ensuite été ajoutés et le nouveau mélange résultant a été agité à température ambiante pendant 20 h. Le produit a été extrait avec du dichlorométhane et lavé avec de l'eau. Le dichlorométhane et l'épichlorhydrine ont été éliminés de la phase organique en utilisant un évaporateur rotatif. Du mélange réactionnel, le tétraglycidyl éther de l'acide divanillique a pu être isolé par chromatographie flash, en utilisant un solvant à gradient de dichlorométhane/méthanol (de 99/1 à 90/10 (en volume) pendant 30 minutes). Le rendement était inférieur à 50 %. La synthèse mise en oeuvre n'était pas optimisée.

On a confirmé l'obtention dudit tétraglycidyléther de l'acide divanilique par spectroscopie RMN : 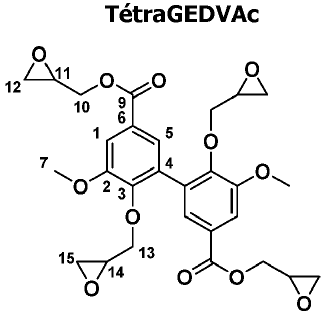
**RMN ¹H** (400MHz, DMSO-d6, δ (ppm)) : δ 7.63 (d, H₁), 7.50 (d, H₅), 4.65 (d, H₁₀), 4.15 (m, H_{10b}), 4.09 (s, H₁₄), 3.94 (m, H₇), 3.86 (m, H_{14b}), 3.35 (m, H₁₁), 2.97 (m, H₁₃), 2.83 (m, H₁₂), 2.73 (m, H₁₅), 2.62 (m, H_{12b}), 2.38 (m, H_{15b}).
**RMN ¹³C** (400MHz, DMSO-d6, δ (ppm)) : δ 164.94 (s, C₉), 152.12 (s, C₃), 149.56 (s, C₂), 131.23 (s, C₆), 124.57 (s, C₄), 124.18 (s, C₅), 112.98 (s, C₁), 74.01 (s, C₁₃), 56.08 (s, C₁₀), 50.24 (s, C₇), 49.94 (s, C₁₄), 49.04 (s, C₁₁), 43.90 (s, C₁₂), 43.36 (s, C₁₅).

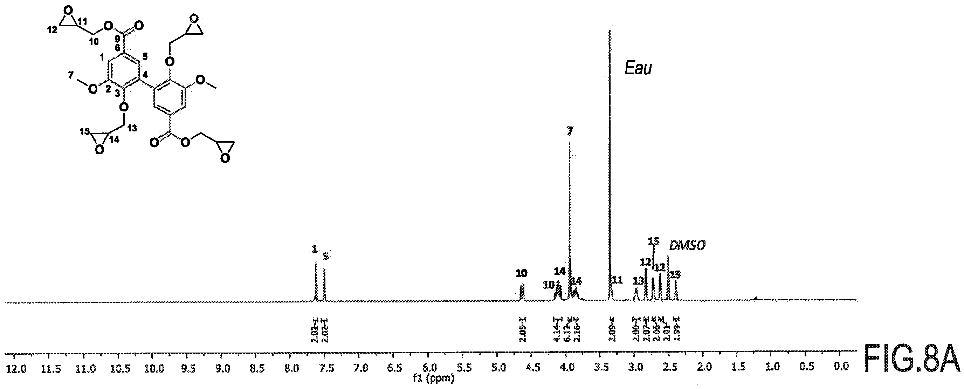

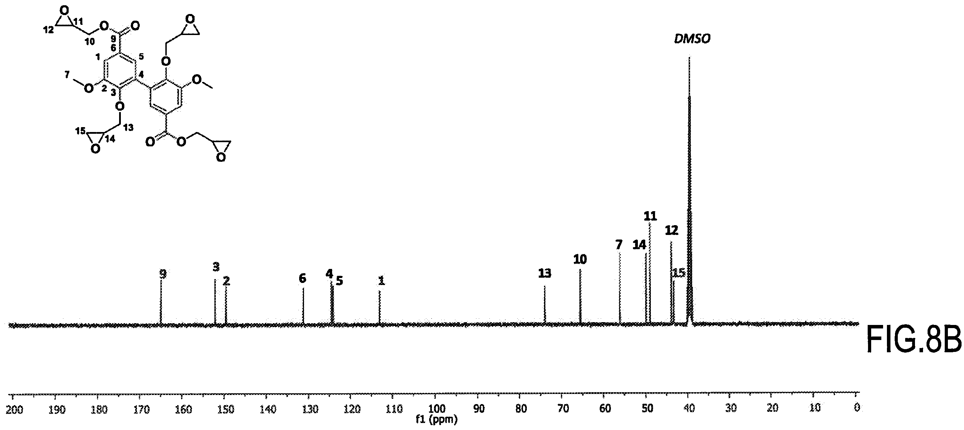

Les spectres sont respectivement montrés sur les figures 8A et 8B.

### Exemple 5

### A5. Synthèse de composés de formule (I) à partir de l'eugénol

Les différentes étapes du schéma réactionnel ci-dessous ont été successivement mises en oeuvre.

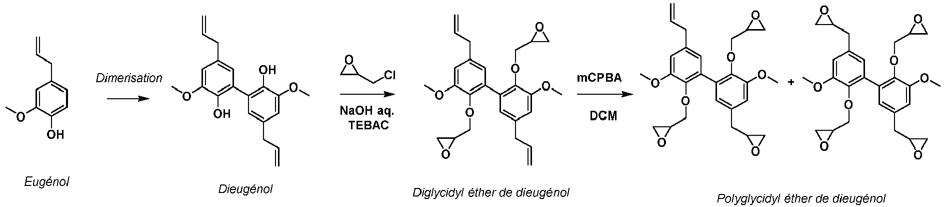

### Synthèse du dieugénol (DEG)

Pour la préparation du dieugénol, on a procédé selon un mode opératoire tout à fait similaire à celui décrit pour la préparation de la divanilline au point A1 ci-dessus. Ledit mode opératoire a déjà été décrit dans l'exemple 7 de la demande de brevet EP 3 002 333.

On a confirmé l'obtention du dieugénol (DEG) par spectroscopie RMN : 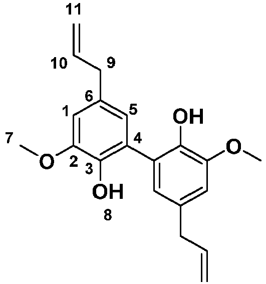
**RMN ¹H** (400MHz, DMSO-d6, δ (ppm)) : δ 8.16 (s, H₈), 6.73 (d, H₁), 6.52 (d, H₅), 5.93 (m, H₁₀), 5.05 (m, H₁₁), 3.79 (s, H₇), 3.27 (d, H₉).
**RMN ¹³C** (400MHz, DMSO-d6, δ (ppm)) : δ 147.61 (s, C₂), 141.79 (s, C₃), 138.15 (s, C₁₀), 129.60 (s, C₆), 125.91 (s, C₄), 122.84 (s, C₅), 115.39 (s, C₁₁), 110.79 (s, C₁), 55.82 (s, C₇), 39.23 (s, C₉).

### Synthèse d'un composé de formule (I) (prépolymère pluriépoxydé) : le diglycidyléther de dieugénol (DiGEDEG)

3 g de dieugénol ont été dissous dans 15 mL d'épichlorhydrine. 0,3 g de bromure de tétrabutylammonium (TEBAC) (0,95 mmol) ont été ajoutés et le mélange résultant a été agité à 80 ° C pendant 24 h. 8 mIL d'une solution de NaOH (5 M) (40 mmol) ont ensuite ajoutés et le nouveau mélange résultant a été agité à température ambiante pendant 24 h. Le produit a été extrait avec du dichlorométhane et lavé avec de l'eau. Le dichlorométhane et l'épichlorhydrine ont été éliminés de la phase organique en utilisant un évaporateur rotatif. La synthèse mise en oeuvre n'était pas optimisée : la conversion n'était pas totale. Le produit obtenu n'était pas pur.

On a confirmé l'obtention du diglycidyléther de dieugénol (DiGEDEG) par spectroscopie RMN : 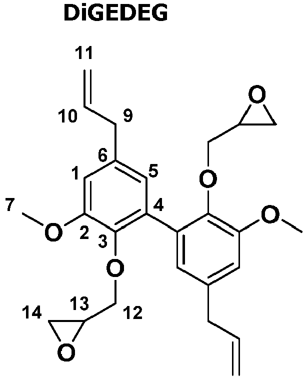
**RMN ¹H** (400MHz, DMSO-d6, δ (ppm)) : δ 6.87 (d, H₁), 6.59 (d, H₅), 5.96 (m, H₁₀), 5.02 (m, H₁₁), 3.87 (s, H₁₂), 3.81 (s, H₇), 3.73 (s, H_{12b}), 3.35 (d, H₉), 2.93 (d, H₁₃), 2.59 (d, H₁₄), 2.34 (d, H_{14b}).
**RMN ¹³C** (400MHz, DMSO-d6, δ (ppm)) : δ 151.99 (s, C₂), 143.47 (s, C₃), 137.66 (s, C₁₀), 135.03 (s, C₆), 132.09 (s, C₄), 122.39 (s, C₅), 115.79 (s, C₁₁), 112.30 (s, C₁), 73.72 (s, C₁₂), 55.73 (s, C₇), 50.04 (s, C₁₃), 43.41 (s, C₁₄), 39.22 (s, C₉).

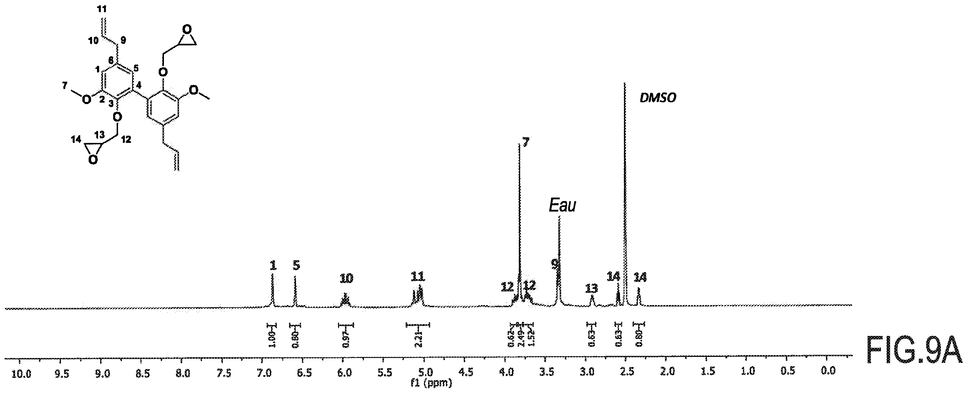

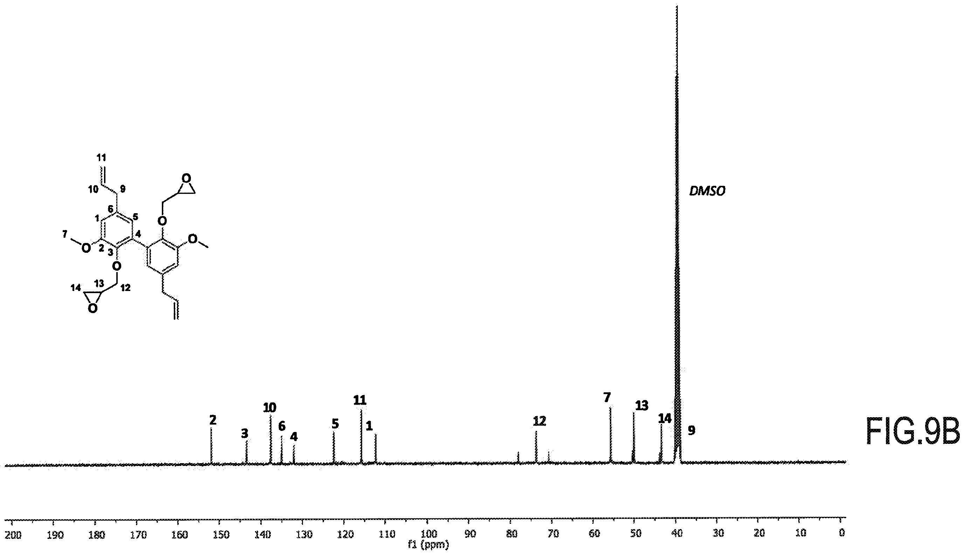

Les spectres sont respectivement montrés sur les figures 9A et 9B.

### Synthèse d'un autre composé de formule (I) (prépolymère pluriépoxydé) : le tétraglycidyléther du dieugénol (TétraGEDEG)

0,5 g de diglycidyléther de dieugénol (tel qu'obtenu ci-dessus) ont été dissous dans 7,5 mL de DCM froid. 1 g de mCPBA ont été solubilisés dans 7,5 mL de DCM froid puis sont ajoutés progressivement à la solution de DiGEDEG. Le mélange a été agité à température ambiante pendant 24 h. Le produit a ensuite été lavé deux fois avec une solution saturée de NaHCO₃ et trois fois à l'eau distillée. Le dichlorométhane a enfin été éliminé en utilisant un évaporateur rotatif. Une purification supplémentaire a été réalisée par chromatographie flash en utilisant un solvant à gradient de dichlorométhane/méthanol (de 99/1 à 90/10 (en volume) pendant 30 minutes). Le rendement de cette synthèse non optimisée) était inférieur à 50 %. Toutefois, seul le tétraglycidyléther du dieugénol a ainsi été synthétisé (au vu de la quantité de mCPBA employée et de la durée de la réaction (24 h) avec ledit mCPBA) et isolé.

On a confirmé l'obtention du tétraglycidyléther de dieugénol par spectroscopie RMN : 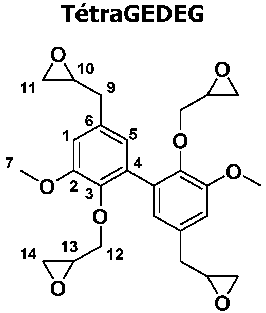
**RMN ¹H** (400MHz, DMSO-d6, δ (ppm)) : δ 6.98 (d, H₁), 6.71 (d, H₅), 3.93 (m, H₁₂), 3.83 (s, H₇), 3.73 (m, H₁₂), 3.13 (m, H₁₀), 2.94 (m, H₁₃), 2.77 (m, H₉ H₁₁), 2.60 (m, H_{11b} H₁₄), 2.36 (m, H_{14b}).
**RMN ¹³C** (400MHz, DMSO-d6, δ (ppm)) : δ 151.94 (s, C₂), 143.79 (s, C₃), 132.84 (s, C₆), 131.98 (s, C₄), 122.94 (s, C₅), 112.86 (s, C₁), 73.74 (s, C₁₂), 55.75 (s, C₇), 51.98 (s, C₁₁), 50.03 (s, C₁₀), 46.10 (s, C₁₃), 43.39 (s, C₁₄), 37.5 (s, C₉).

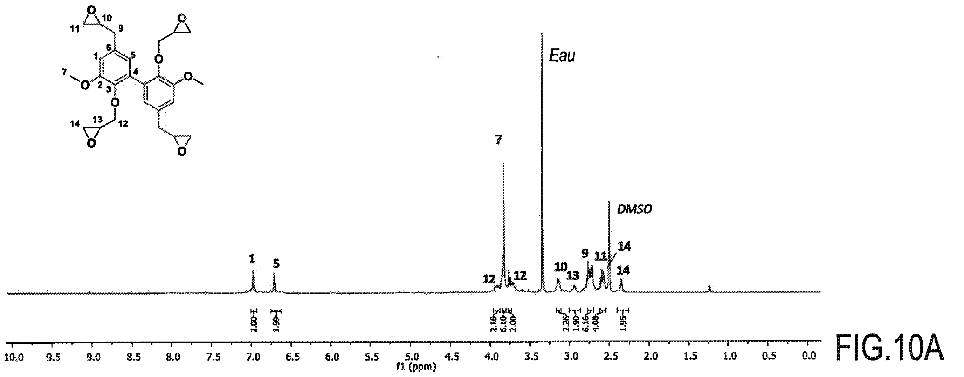

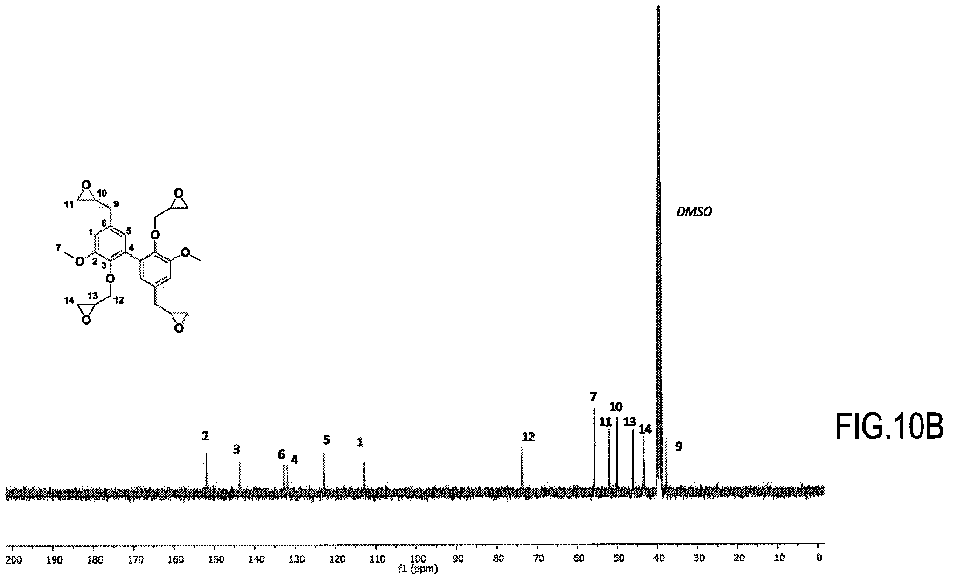

Les spectres sont respectivement montrés sur les figures 10A et 10B.

### B5. Polyépoxyde obtenu à partir du composé de formule (I) : le diglycidyléther de dieugénol (DiGEDEG) (prépolymère pluriépoxydé)

Pour la polymérisation du composé diépoxydé de l'invention obtenu dans le cadre de cet exemple (le diglycidyl éther de dieugénol : DiGEDEG), on a également utilisé, à titre de durcisseur, le diaminodiphényl sulfone (DDS), dont la formule chimique a été rappelée ci-dessus.

Ledit durcisseur a été utilisé en le ratio stoechiométrique : époxy/amine = 2/1. Dans cet exemple aussi, le polyépoxyde n'a pas été successivement préparé puis analysé par DMA (pour détermination de sa Tg). Il a été généré, en petite quantité (quelques mg), lors de la mise en œuvre d'une calorimétrie différentielle à balayage (DSC), pour détermination de sa Tg.

Le taux de coke résiduel, après dégradation à 900°C, déterminé par TGA (ThermoGravimetric Analysis), l'a été sur cette petite quantité générée lors de l'analyse DSC. Les résultats figurent dans le tableau 4 ci-après.

**Tableau 4**

| **Prépolymère précurseur du polyépoxyde** | **Tg (°C)** | **Char900 (%)** |
|---|---|---|
| **DiGEDEG** | 144 | 38 |

**Les** chiffres dudit Tableau 4 confirment l'intérêt des composés de l'invention.

### II. Préparation de composés de formule (I), dans laquelle R₃ = O-CH₃, dérivés de la vanilline, du vanillate de méthyle ou de l'euaénol

### Exemple 6

### A6. Synthèse de composés de formule (I) (composés phénoliques pluriépoxydés) à partir de biphénols méthylés

L'éthérification dans les conditions explicitées ci-dessous a été mise en œuvre sur les biphénols identifiés ci-après.

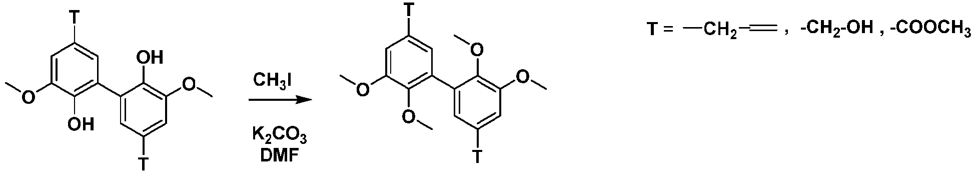

### Synthèse des biphénols méthylés

26 mmol de bisphénol (voir ci-après) et 15,2 g de carbonate de potassium (110 mmol) ont été dissous dans 120 mL de DMF. 9,6 ml d'iodométhane (158 mmol) ont ensuite été lentement ajoutés au mélange. Après 15 h à 80 ° C, le mélange a été filtré et la solution résultante a été versée dans de l'eau froide. Le composé méthylé a précipité et a été récupéré par filtration et séché sous vide. Le rendement typique était de 80 %.

On a mis en œuvre l'éthérification avec successivement :
- l'alcool divanillique (DVA ; T = CH₂OH) (tel qu'obtenu au point A1 de l'exemple 1 ci-dessus), pour obtenir l'alcool divanillique méthylé (mDVA). Ledit mDVA a été caractérisé par spectroscopie RMN : 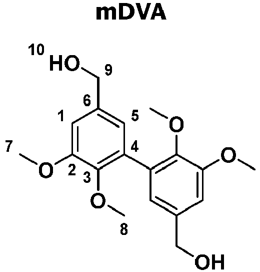
   **RMN ¹H** (400 MHz, DMSO-d6, δ (ppm)) : δ 6.99 (d, H₁), 6.67 (d, H₅), 5.15 (s, H₁₀), 4.47 (s, H₉), 3.83 (s, H₇), 3.50 (s, H₈).
   **RMN ¹³C** (400 MHz, DMSO-d6, δ (ppm)) : δ 152.00 (s, C₃), 144.81 (s, C₂), 137.64 (s, C₆), 132.12 (s, C₄), 120.33 (s, C₅), 110.18 (s, C₁), 62.63 (s, C₉), 59.91 (s, C₈), 55.52 (C₇).
- le divanillate de méthyle (DVE ; T= -COOCH₃) (tel qu'obtenu au point A4 de l'exemple 4 ci-dessus), pour obtenir le divanillate de méthyle méthylé (mDVE). Celui-ci a été caractérisé par spectroscopie : 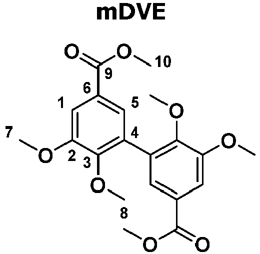
   **RMN ¹H** (400 MHz, DMSO-d6, δ (ppm)): δ 7.59 (d, H₁), 7.40 (d, H₅), 3.92 (s, H₇), 3.83 (s, H₁₀), 3.62 (s, H₈).
   **RMN ¹³C** (400 MHz, DMSO-d6, δ (ppm)): δ 165.67 (s, C₉), 152.27 (s, C₃), 150.36 (s, C₂), 131.28 (s, C₆), 124.70 (s, C₄), 123.96 (s, C₅), 112.76 (s, C₁), 60.29 (s, C₈), 55.92 (s, C₇), 52.18 (C₁₀).

Ledit divanillate de méthyle méthylé a ensuite été hydrolysé dans les conditions précisées ci-après. 10 mmol de divanillate de méthyle ont été solubilisés dans 30 mL de méthanol. 3 g d'hydroxide de sodium (75 mmol) ont été ajoutés au mélange, qui a lors été agité et chauffé à reflux, pendant 4 h. Après refroidissement à température ambiante, une solution aqueuse d'acide chlorhydrique (2 M) a été ajoutée, jusqu'à obtenir un pH = 3. Le précipité obtenu (d'acide divanillique méthylé (mDVAc)) a été ensuite filtré et séché à 80°C, dans une étuve, à pression réduite. Ledit acide divanillique méthylé (mDVAc) a été caractérisé par spectroscopie RMN : 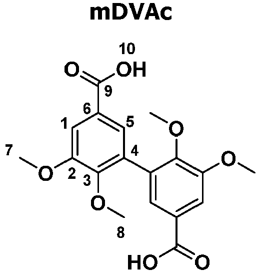
RMN ¹H (400 MHz, DMSO-d6, δ (ppm)): δ 12.94 (s, H₁₀), 7.58 (d, H₁), 7.39 (d, H₅), 3.91 (s, H₇), 3.61 (s, H₈).
RMN ¹³C (400 MHz, DMSO-d6, δ (ppm)): δ 166.83 (s, C₉), 152.20 (s, C₃), 150.07 (s, C₂), 131.34 (s, C₆), 125.91 (s, C₄), 124.13 (s, C₅), 112.93 (s, C₁), 60.28 (s, C₈), 55.87 (s, C₇).

- le dieugénol (DEG ; T= -CH₂-CH=CH₂) (tel qu'obtenu au point A5 de l'exemple 5 ci-dessus), pour obtenir le dieugénol méthylé (mDEG). Ledit mDEG a été caractérisé par spectroscopie RMN : 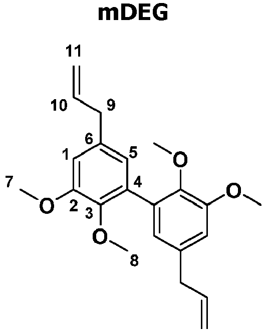
   **RMN ¹H** (400MHz, DMSO-d6, δ (ppm)) : δ 6.85 (d, H₁), 6.54 (d, H₅), 5.96 (m, H₁₀), 5.07 (m, H₁₁), 3.81 (s, H₇), 3.48 (s, H₈), 3.34 (d, H₉)
   **RMN ¹³C** (400MHz, DMSO-d6, δ (ppm)) : δ 152.16 (s, C₂), 144.41 (s, C₃), 137.67 (s, C₁₀), 134.78 (s, C₆), 132.29 (s, C₄), 122.26 (s, C₅), 115.80 (s, C₁₁), 112.17 (s, C₁), 59.91 (s, C₇), 55.58 (s, C₉), 39.22 (s, C₇), 35.76 (s, C₇).

### Synthèse des composés de formule (I) (prépolymères pluriépoxydés)

On a procédé comme décrit précédemment pour époxyder :
- (avec l'épichlorhydrine) les fonctions alcool du mDVA. On a alors obtenu le diglycidyl éther de l'alcool divanillique méthylé **(DiGEmDVA).** Ledit **DiGEmDVA** a été caractérisé par spectroscopie RMN : 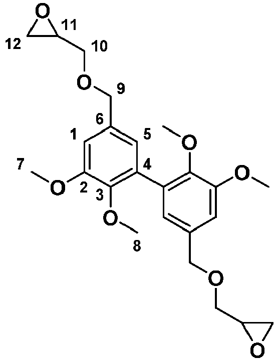
   **¹H NMR** (400MHz, DMSO-d6, δ (ppm)): δ 7.01 (d, H₁), 6.72 (d, H₅), 4.49 (t, H₉), 3.84 (d, H₇), 3.77 (m, H₁₀), 3.52 (s, H₈), 3.31 (m, H_{10b}), 3.15 (m, H₁₁), 2.73 (t, H₁₂), 2.55 (m, H_{12b}).
   **¹³C NMR** (400MHz, DMSO-d6, δ (ppm)): δ 152.20 (s, C₃), 145.48 (s, C₂), 133.25 (s, C₆), 131.98 (s, C₄), 121.66 (s, C₅), 111.35 (s, C₁), 71.90 (s, C₉), 70.71 (s, C₁₀), 59.90 (s, C₈), 55.61 (s, C₇), 50.27 (s, C₁₁), 43.41 (s, C₁₂).

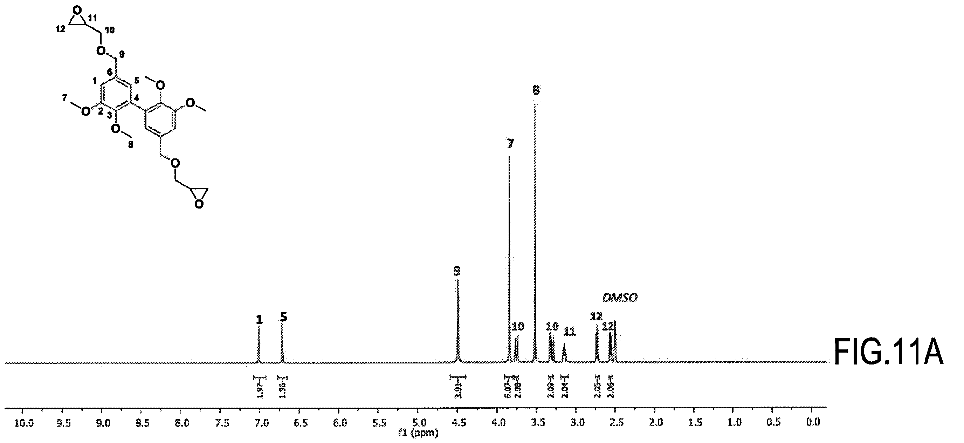

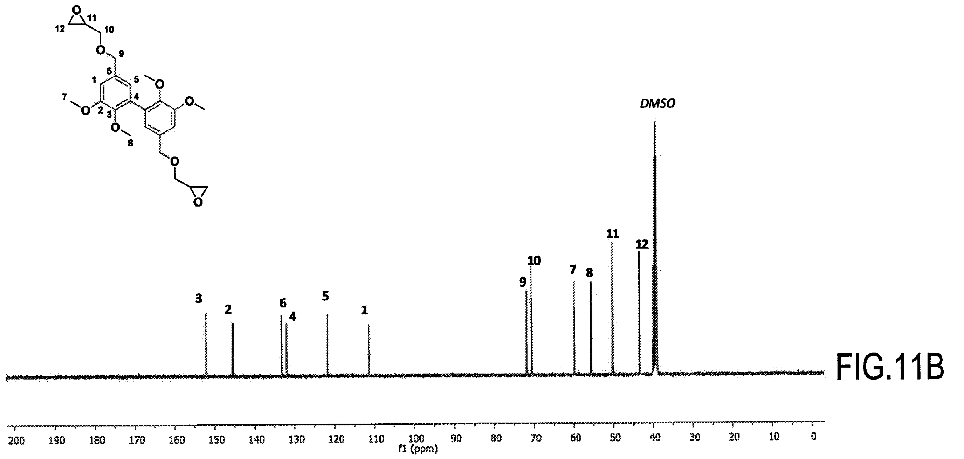

Les spectres sont respectivement montrés sur les figures 11A et 11B.
- (avec l'épichlorhydrine) les fonctions acide du mDVAc : On obtient le diglycidyl éther de l'acide divanillique méthylé **(DiGEmDVAc).** Ledit **DiGEmDVAc** a été caractérisé par spectroscopie RMN : 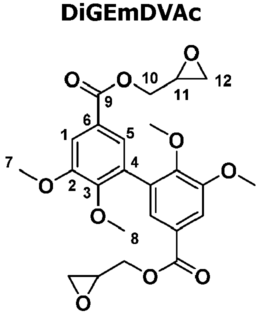
   ¹H NMR (400MHz, DMSO-d6, δ (ppm)): δ 7.61 (d, H₁), 7.45 (d, H₅), 4.65 (d, H₁₀), 4.08 (q, H_{10b}), 3.93 (s, H₇), 3.64 (m, H₈), 3.34 (m, H₁₁), 2.82 (m, H₁₂), 2.72 (m, H_{12b}).
   ¹³C NMR (400MHz, DMSO-d6, δ (ppm)): δ 164.92 (s, C₉), 152.34 (s, C₃), 150.55 (s, C₂), 131.28 (s, C₆), 124.41 (s, C₄), 124.04 (s, C₅), 112.92 (s, C₁), 65.58 (s, C₁₀), 60.34 (s, C₈), 55.97 (s, C₇), 49.01 (s, C₁₁), 43.90 (s, C₁₂).

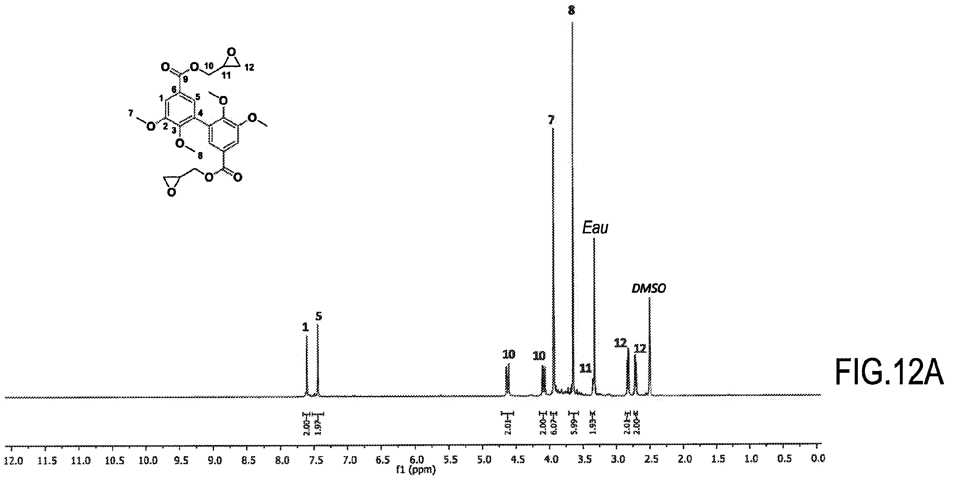

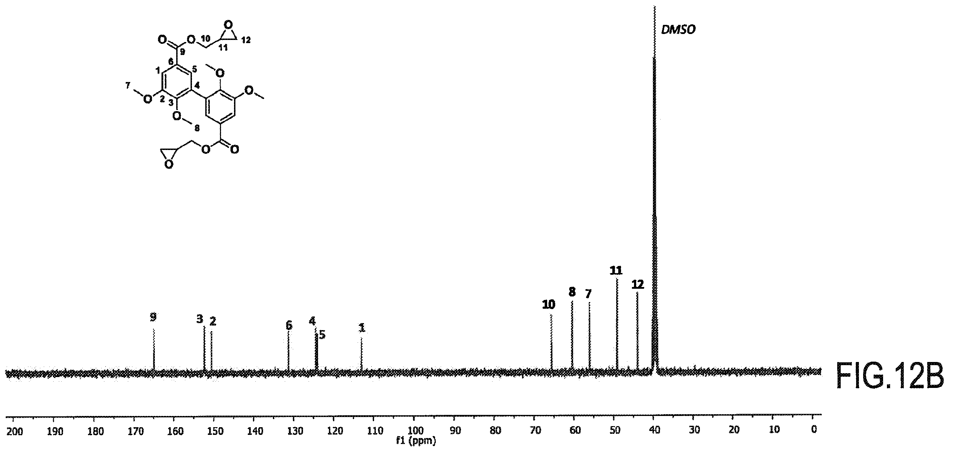

Les spectres sont respectivement montrés sur les figures 12A et 12B.
- (avec l'oxydant CPBA) les doubles liaisons du dieugénol. On obtient le diglycidyl éther du dieugénol méthylé **(DiGEmDEG).** Ledit **DiGEmDEG** a été caractérisé par spectroscopie RMN : 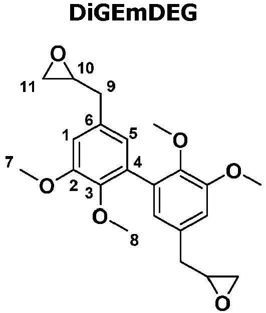
   **RMN ¹H** (400MHz, DMSO-d6, δ (ppm)): δ 6.95 (d, H₁), 6.66 (d, H₅), 3.83 (s, H₇), 3.51 (s, H₈), 3.12 (m, H₁₀), 2.74 (m, H₉), 2.57 (m, H₁₁)
   **RMN ¹³C** (400MHz, DMSO-d6, δ (ppm)): δ 152.15 (s, C₂), 144.77 (s, C₃), 132.67 (s, C₆), 132.24 (s, C₄), 122.84 (s, C₅), 112.80 (s, C₁), 59.95 (s, C₈), 55.65 (s, C₇), 52.05 (s, C₁₀), 46.15 (s, C₁₁), 37.90 (s, C₉).

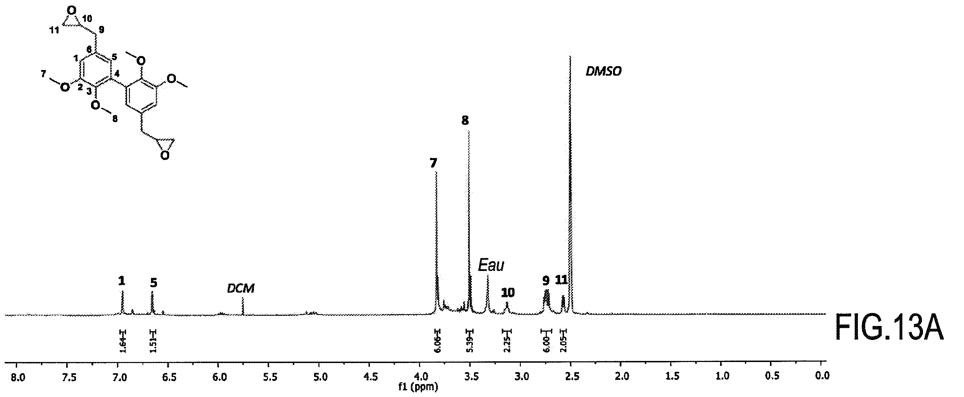

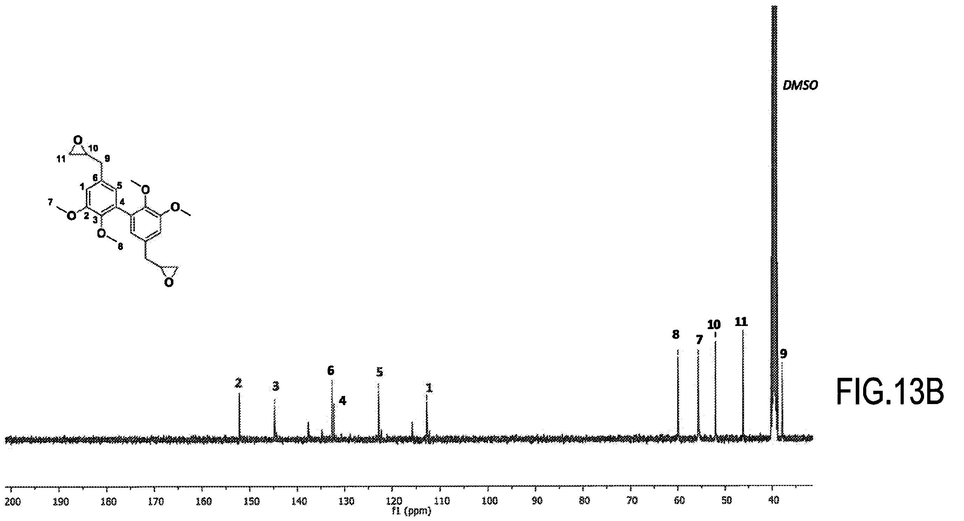

Les spectres sont respectivement montrés sur les figures 13A et 13B.

### B6. Polyépoxydes obtenus à partir desdits composés de formule (I) (prépolymères pluriépoxydés)

Pour la polymérisation des composés de l'invention obtenus dans le cadre de cet exemple, on a également utilisé, à titre de durcisseur, le diaminodiphényl sulfone (DDS), dont la formule chimique a été rappelée ci-dessus.

Ledit durcisseur a été utilisé en le ratio stoechiométrique : époxy/amine = 2/1. Les polyépoxydes n'ont pas été successivement préparés puis analysés par DMA (pour détermination de sa Tg). Ils ont été générés, en petite quantité (quelques mg), lors de la mise en œuvre d'une calorimétrie différentielle à balayage (DSC), pour détermination de leur Tg.

Le taux de coke résiduel, après dégradation à 900°C, déterminé par TGA (ThermoGravimetric Analysis), l'a été sur la petite quantité de DiGEmDVac générée lors de l'analyse DSC. Les résultats figurent dans le tableau 5 ci-après.

**Tableau 5**

| **Prépolyméres précurseurs du polyépoxyde** | **Tg (°C)** | **Char900 (%)** |
|---|---|---|
| **DiGEmDVA** | 211 | 47 |
| **DiGEmDVAc** | 175 | 32 |
| **DiGEmDEG** | 134 | nd* |

| | | |
|---|---|---|
| *nd = non déterminé. | | |

## Revendications

1. Procédé de préparation d'un composé biphényle pluriépoxydé répondant à la formule (I) : 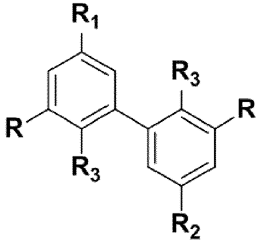 dans laquelle
R = -O-Alk, avec Alk un groupe alkyle, linéaire ou ramifié, comportant de 1 à 6 atomes de carbone,
R₃ = -O-Z, avec Z un groupe alkyle, linéaire ou ramifié, comportant de 2 à 8, avantageusement de 3 à 8, atomes de carbone, et renfermant une fonction époxy, ou -O-Alk', avec Alk' un groupe alkyle, linéaire ou ramifié, comportant de 1 à 6 atomes de carbone ; et :
+ lorsque R₃ = -O-Z, alors,
soit R₁ et R₂, identiques ou différents, sont choisis indépendamment parmi - CH₂-OH et -CH₂-O-Z ;
soit R₁=R₂=-CHO ;
soit R₁ et R₂, identiques ou différents, sont choisis indépendamment parmi -OH et -O-Z ;
soit R₁ et R₂, identiques ou différents, sont choisis indépendamment parmi -COOH et -COO-Z,
soit R₁ et R₂, identiques ou différents, sont choisis indépendamment parmi -CH₂-CH=CH₂ et -CH₂-époxy,
soit R₁ et R₂, identiques ou différents, sont choisis indépendamment parmi -CH=CH-CH₃ et 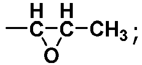 et
+ lorsque R₃ = -O-Alk', alors
R₁ = R₂ est choisi parmi :
-CH₂-O-Z,
-O-Z,
-COO-Z,
avec Z tel que défini ci-dessus en référence à R₃ = -O-Z,
-CH₂-époxy, et 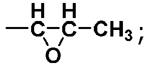
ledit procédé comprenant :
a) la mise à disposition d'un dimère choisi parmi la divanilline, l'alcool divanillique, la diméthoxyhydroquinone, l'acide divanillique, le dieugénol, le dimère de l'isoeugénol, lesdits dimères présentant au moins deux fonctions -OH phénoliques et deux fonctions -O-CH₃, et les analogues desdits dimères présentant lesdites au moins deux fonctions - OH phénoliques et deux fonctions -O-(C₂-C₆)alkyle ;
b) l'éventuelle alkylation des fonctions -OH phénoliques d'un dimère choisi parmi la divanilline, l'alcool divanillique, l'acide divanillique, le dieugénol, le dimère de l'isoeugénol et leurs analogues pour l'obtention de ladite divanilline alkylée, dudit alcool divanillique alkylé, dudit acide divanillique alkylé, dudit dieugénol alkylé, dudit dimère de l'isoeugénol alkylé et de leurs analogues alkylés ; l'alkylation de la divanilline ou d'un de ses analogues étant suivie d'une oxydation pour l'obtention de la di(C₁-C₆)alcoxyhydroquinone alkylée ; et
c1) en l'absence d'une telle alkylation, l'époxydation des deux fonctions -OH phénoliques de la divanilline ou d'un de ses analogues ou l'époxydation des deux, voire trois, voire même quatre, fonctions présentes sur le noyau biphényle susceptibles d'être époxydées, dudit alcool divanillique, de ladite diméthoxyhydroquinone, dudit acide divanillique, dudit dieugénol, dudit dimère de l'isoeugénol ou d'un de leurs analogues ; ou
c2) suite à une telle alkylation, l'époxydation des deux fonctions non alkylées, encore présentes sur le noyau biphényle, de l'alcool divanillique alkylé, de la di(C₁-C₆)alcoxyhydroquinone alkylée, de l'acide divanillique alkylé, du dieugénol alkylé, du dimère de l'isoeugénol alkylé ou d'un de leurs analogues ;
le dimère de l'isoeugénol répondant à la formule suivante :

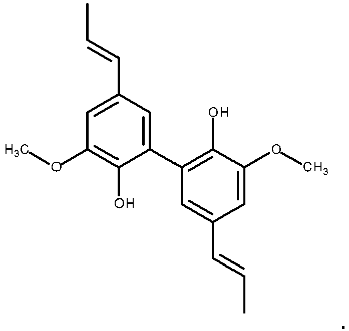

2. Procédé selon la revendication 1, où dans la formule (I) R₃ = -O-Z, ledit groupe alkyle Z étant linéaire et/ou, avantageusement et, renfermant ladite fonction époxy en bout de chaine.

3. Procédé selon la revendication 1 ou 2, où dans la formule (I) R₃ = -O-[CH₂-]ₙ-époxy, avec n un entier de 0, avantageusement de 1, à 6, avantageusement avec 1 ≤ n ≤ 4 et très avantageusement avec n = 1.

4. Procédé selon la revendication 1, où le composé de formule (I) est choisi parmi :
- le diglycidyl éther d'alcool divanillique de formule : 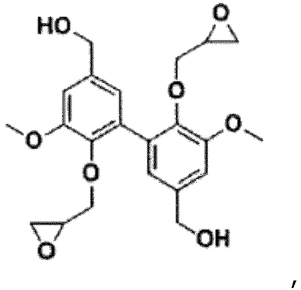
- le triglycidyl éther d'alcool divanillique de formule : 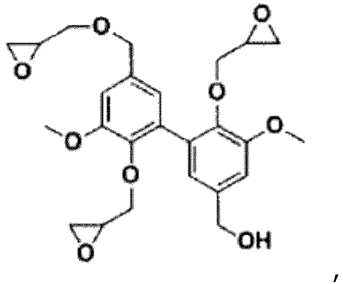 et
- le tétraglycidyl éther d'alcool divanillique de formule : 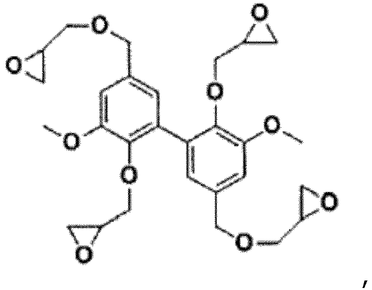 procédé qui comprend :
a) la mise à disposition d'alcool divanillique ;
b) l'époxydation de deux, trois, ou quatre fonctions -OH phénoliques de l'alcool divanillique.

5. Procédé selon la revendication 1, où le composé de formule (I) est le diglycidyl éther de divanilline de formule : 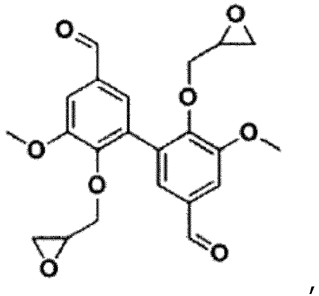 procédé qui comprend :
a) la mise à disposition de divanilline ;
b) l'époxydation des deux fonctions -OH phénoliques de la divanilline.

6. Procédé selon la revendication 1, où le composé de formule (I) est choisi parmi :
- le diglycidyl éther de la diméthoxyhydroquinone de formule : 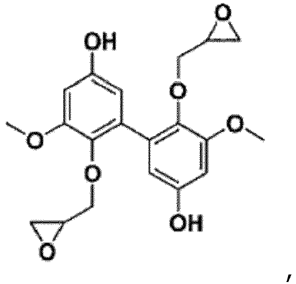
- le triglycidyl éther de la diméthoxyhydroquinone de formule : 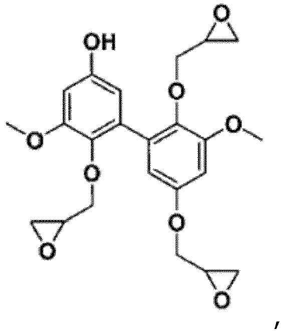 et
- le tétraglycidyl éther de la diméthoxyhydroquinone de formule : 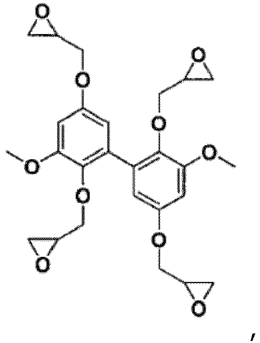 procédé qui comprend :
a) la mise à disposition de diméthoxyhydroquinone ;
b) l'époxydation de deux, trois, ou quatre fonctions -OH phénoliques de la diméthoxyhydroquinone.

7. Procédé selon la revendication 1, où le composé de formule (I) est choisi parmi :
- le diglycidyl éther d'acide divanillique de formule : 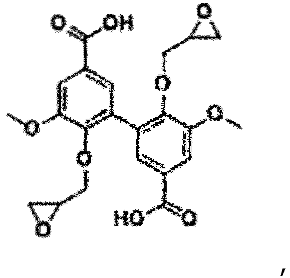
- le triglycidyl éther d'acide divanillique de formule : 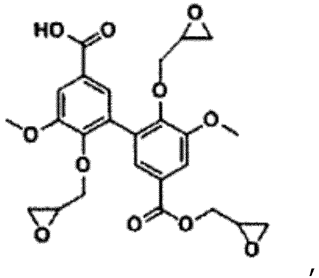 et
- le tétraglycidyl éther d'acide divanillique de formule : 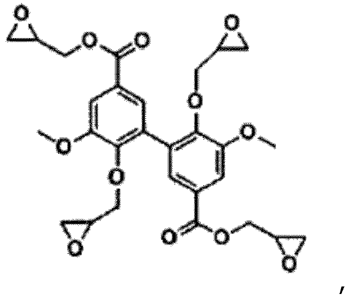 procédé qui comprend :
a) la mise à disposition d'acide divanillique ;
b) l'époxydation de deux, trois, ou quatre fonctions -OH phénoliques de l'acide divanillique.

8. Procédé selon la revendication 1, où le composé de formule (I) est choisi parmi :
- le diglycidyl éther de dieugénol de formule : 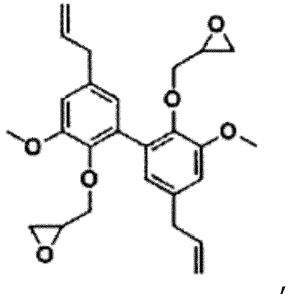
- le triglycidyl éther de dieugénol de formule : 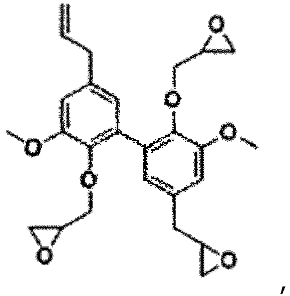 et
- le tétraglycidyl éther de dieugénol de formule : 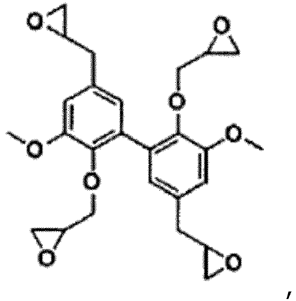 procédé qui comprend :
a) la mise à disposition de dieugénol ;
b) l'époxydation de deux, trois, ou quatre fonctions -OH phénoliques du dieugénol.

9. Procédé selon la revendication 1, où le composé de formule (I) est choisi parmi un composé de formule : 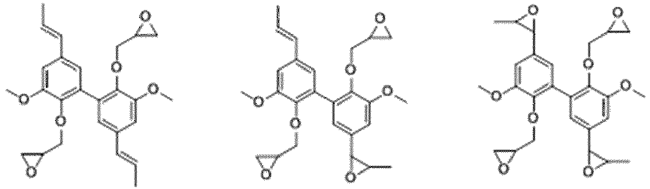 procédé qui comprend :
a) la mise à disposition de dimère de l'isoeugénol ;
b) l'époxydation de deux, trois, ou quatre fonctions -OH phénoliques du dimère de l'isoeugénol de formule :

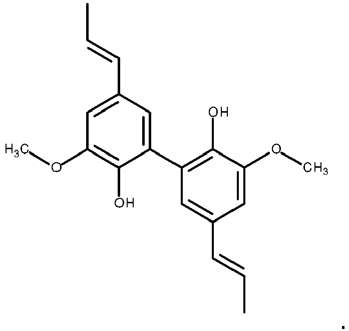

10. Procédé selon la revendication 1, où dans la formule (I) R₃ = -OAlk' et où R₁= R₂ = -CH₂-O-Z, -O-Z ou -COO-Z, ledit groupe alkyle Z étant linéaire et/ou, avantageusement et, renfermant ladite fonction époxy en bout de chaine.

11. Procédé selon la revendication 10, où dans la formule (I) R₁= R₂ = -CH₂-O-[CH₂-]ₙ₋époxy , -O-[CH₂-]ₙ-époxy ou -COO-[CH₂-]ₙ-époxy, avec n un entier de 0, avantageusement de 1, à 6 ; avantageusement de formule (I) dans laquelle R₁= R₂ = - CH₂O-CH₂-époxy , ou -O-CH₂₋époxy ou -COO-CH₂-époxy.

12. Procédé selon l'une quelconque des revendications 1, 10 et 11, où dans la formule (I) R₃ = -O-CH₃.

13. Procédé selon la revendication 1, où le composé de formule (I) est choisi parmi :
- le diglycidyl éther de l'alcool divanillique méthylé de formule : 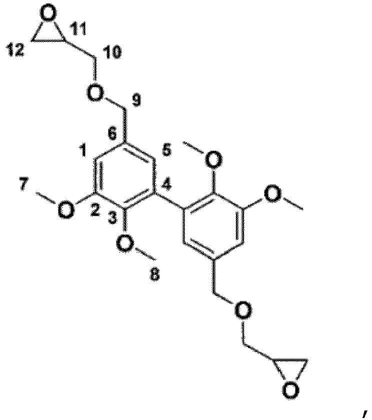
- le diglycidyl éther de l'acide divanillique méthylé de formule : 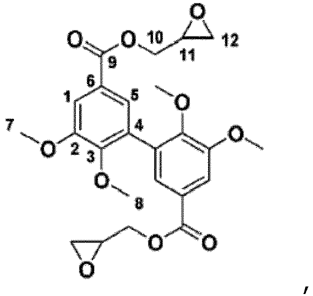
- le diglycidyl éther de la diméthoxyhydroquinone méthylée de formule : 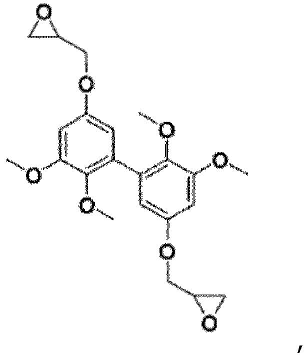
- le diglycidyl éther du dieugénol méthylé de formule : 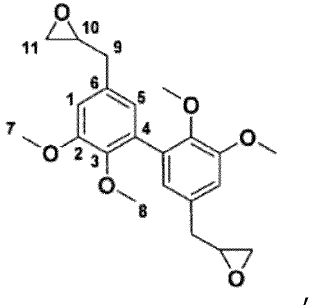 et
- le diglycidyl éther du dimère de l'isoeugénol méthylé de formule : 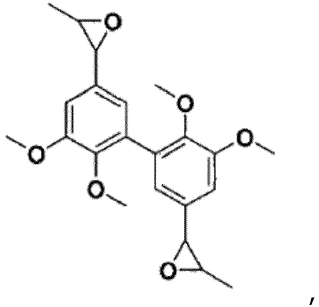
procédé qui comprend :
a) la mise à disposition d'alcool divanillique, d'acide divanillique, de diméthoxyhydroquinone, de dieugénol ou de dimère de l'isoeugénol ;
b) la méthylation des fonctions -OH phénoliques de l'alcool divanillique, de l'acide divanillique, de la diméthoxyhydroquinone, du dieugénol, ou du dimère de l'isoeugénol pour l'obtention d'alcool divanillique méthylé, d'acide divanillique méthylé, de diméthoxyhydroquinone méthylée, de dieugénol méthylé, ou de dimère de l'isoeugénol méthylé ;
b) l'époxydation des deux fonctions non alkylées, encore présentes sur le noyau biphényle, de l'alcool divanillique méthylé, de l'acide divanillique méthylé, de la diméthoxyhydroquinone méthylée, du dieugénol méthylé, ou du dimère de l'isoeugénol méthylé ;
le dimère de l'isoeugénol répondant à la formule suivante :

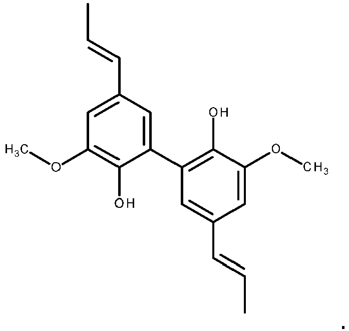

14. Procédé selon l'une quelconque des revendications 1 à 13, **caractérisé en ce que** l'époxydation est mise en oeuvre :
- par réaction avec un composé de formule Cl-Z, dans laquelle Z est un groupe alkyle, linéaire ou ramifié, comportant de 2 à 8, avantageusement de 3 à 8, atomes de carbone, et renfermant une fonction époxy, ledit composé consistant avantageusement en l'épichlorhydrine, ou
- par allylation puis époxydation oxydante des doubles liaisons introduites, ou
- par époxydation oxydante d'au moins une double liaison présente.

## Patentansprüche

1. Verfahren zur Herstellung einer pluriepoxidierten Biphenylverbindung mit der Formel (I) : 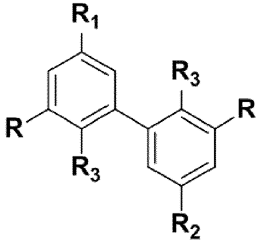 wobei
R = -O-Alk, wobei Alk eine lineare oder verzweigte Alkylgruppe mit 1 bis 6 Kohlenstoffatomen ist,
R₃ = -O-Z, wobei Z eine lineare oder verzweigte Alkylgruppe mit 2 bis 8, vorzugsweise 3 bis 8, Kohlenstoffatomen ist und eine Epoxyfunktion enthält, oder -O-Alk', wobei Alk' eine lineare oder verzweigte Alkylgruppe mit 1 bis 6 Kohlenstoffatomen ist; und :
+ wenn R₃ = -O-Z, dann,
oder R₁ und R₂, die gleich oder verschieden sind, unabhängig voneinander ausgewählt sind aus -CH₂ -OH und -CH₂ -O-Z ;
oder R₁ = R₂ =-CHO ;
oder R₁ und R₂, die gleich oder verschieden sind, unabhängig voneinander aus -OH und -O-Z ausgewählt sind;
oder R₁ und R₂, die gleich oder verschieden sind, unabhängig voneinander ausgewählt sind aus -COOH und -COO-Z,
oder R₁ und R₂, die gleich oder verschieden sind, unabhängig voneinander ausgewählt sind aus -CH₂-CH=CH₂ und -CH₂-Epoxy,
oder R₁ und R₂, die gleich oder verschieden sind, unabhängig voneinander ausgewählt sind aus -CH=CH-CH₃ und 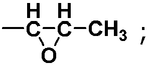 und
+ wenn R₃ = -O-Alk', dann
R₁ = R₂ ist ausgewählt aus:
-CH₂-O-Z,
-O-Z,
-COO-Z,
mit Z wie oben definiert unter Bezugnahme auf R₃ = -O-Z,
-CH₂-Epoxy, und 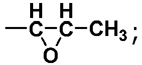
wobei das Verfahren umfasst:
a) Bereitstellung eines Dimers, ausgewählt aus Divanillin, Divanillylalkohol, Dimethoxyhydrochinon, Divanillinsäure, Dieugenol, Isoeugenol-Dimer, wobei die Dimere mindestens zwei phenolische -OH-Funktionen und zwei -O-CH₃-Funktionen aufweisen, und Analoga der Dimere, die die mindestens zwei phenolischen -OH-Funktionen und zwei -O-(C₂-C₆)Alkyl-Funktionen aufweisen;
b) gegebenenfalls Alkylierung der phenolischen -OH-Funktionen eines Dimers, ausgewählt aus Divanillin, Divanillylalkohol, Divanillinsäure, Dieugenol, Isoeugenol-Dimer und deren Analoga, um das alkylierte Divanillin, den alkylierten Divanillylalkohol, die alkylierte Divanillinsäure, das alkylierte Dieugenol, das alkylierte Isoeugenol-Dimer und deren alkylierte Analoga zu erhalten; wobei auf die Alkylierung von Divanillin oder einem Analogon davon eine Oxidation folgt, um das alkylierte Di(C₁-C₆)alkoxyhydrochinon zu erhalten; und
c1) in Abwesenheit einer solchen Alkylierung, die Epoxidierung der beiden phenolischen -OH-Funktionen von Divanillin oder einem Analogon davon, oder die Epoxidierung der zwei, drei oder sogar vier Funktionen, die am Biphenylring vorhanden sind und epoxidiert werden können, des Divanillin-Alkohols, des Dimethoxyhydrochinons, der Divanillinsäure, des Dieugenols, des Isoeugenol-Dimers oder eines Analogons davon; oder
c2) im Anschluss an eine solche Alkylierung die Epoxidierung der beiden nicht alkylierten Funktionen, die noch am Biphenylring vorhanden sind, des alkylierten Divanillylalkohols, des alkylierten Di(C₁-C₆)alkoxyhydrochinons, der alkylierten Divanillinsäure, des alkylierten Dieugenols, des alkylierten Isoeugenol-Dimers oder eines Analogons davon;
wobei das Dimer von Isoeugenol die folgende Formel aufweist:

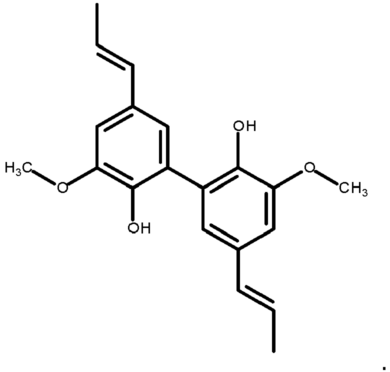

2. Verfahren nach Anspruch 1, wobei in Formel (I) R₃ = -O-Z, wobei die Alkylgruppe Z linear ist und/oder, vorteilhafterweise und, die Epoxyfunktion am Ende der Kette enthält.

3. Verfahren nach Anspruch 1 oder 2, wobei in Formel (I) R₃ = -O-[CH₂-]ₙ-Epoxy, wobei n eine ganze Zahl von 0, vorteilhafterweise 1, bis 6, vorteilhafterweise mit 1 ≤ n ≤ 4 und sehr vorteilhafterweise mit n = 1 ist.

4. Verfahren nach Anspruch 1, wobei die Verbindung der Formel (I) ausgewählt ist aus:
- dem Diglycidylether von Divanillylalkohol der Formel : 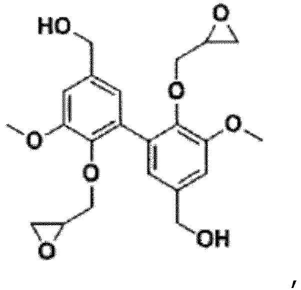
- dem Triglycidylether von Divanillylalkohol der Formel : 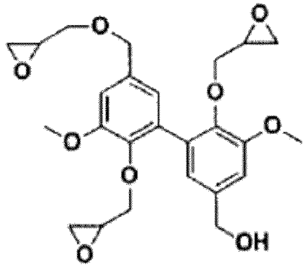 und
- dem Tetraglycidylether von Divanillylalkohol der Formel : 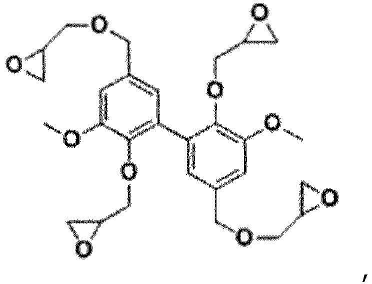 Verfahren, das Folgendes umfasst:
a) Bereitstellung von Divanillinalkohol ;
b) die Epoxidation von zwei, drei oder vier phenolischen -OH-Funktionen von Divanillylalkohol.

5. Verfahren nach Anspruch 1, wobei die Verbindung der Formel (I) ist das Diglycidylether von Divanillin der Formel : 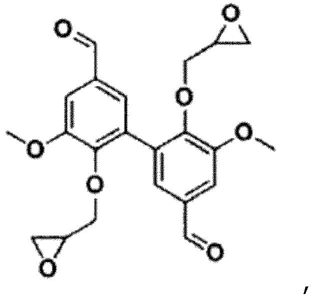 Verfahren, das Folgendes umfasst:
a) die Bereitstellung von Divanillin ;
b) die Epoxidation der beiden phenolischen -OH-Funktionen von Divanillin.

6. Verfahren nach Anspruch 1, wobei die Verbindung der Formel (I) ausgewählt ist aus :
- dem Diglycidylether von Dimethoxyhydrochinon der Formel : 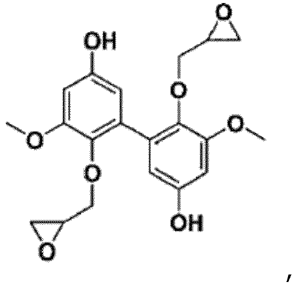
- dem Triglycidylether von Dimethoxyhydrochinon der Formel : 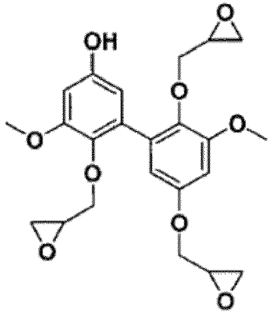 und
- dem Tetraglycidylether von Dimethoxyhydrochinon der Formel : 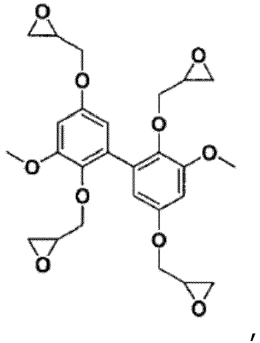 Verfahren, das Folgendes umfasst:
a) die Bereitstellung von Dimethoxyhydrochinon ;
b) die Epoxidation von zwei, drei oder vier phenolischen -OH-Funktionen von Dimethoxyhydrochinon.

7. Verfahren nach Anspruch 1, wobei die Verbindung der Formel (I) ausgewählt ist aus :
- dem Diglycidylether von Divanillinsäure der Formel : 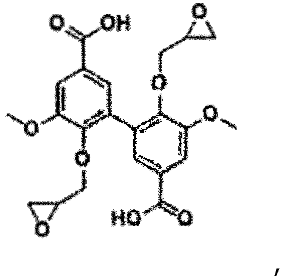
- dem Triglycidylether der Divanillinsäure der Formel : 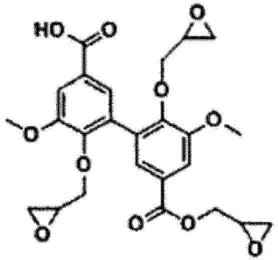 und
- dem Tetraglycidylether von Divanillinsäure der Formel : 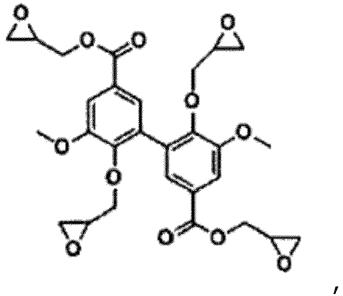 Verfahren, das Folgendes umfasst:
a) die Bereitstellung von Divanillinsäure ;
b) die Epoxidation von zwei, drei oder vier phenolischen -OH-Funktionen der Divanillinsäure.

8. Verfahren nach Anspruch 1, wobei die Verbindung der Formel (I) ausgewählt ist aus :
- dem Diglycidylether von Dieugenol der Formel : 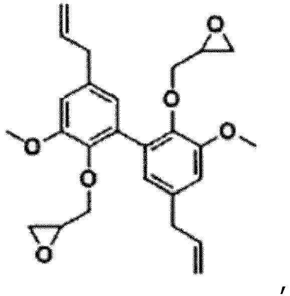
- dem Triglycidylether von Dieugenol der Formel : 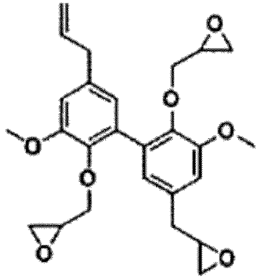 und
- dem Tetraglycidylether von Dieugenol der Formel : 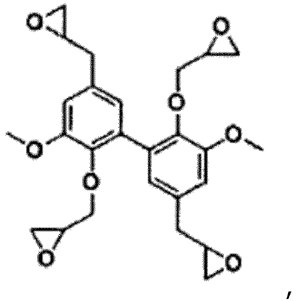 Verfahren, das Folgendes umfasst:
a) die Bereitstellung von Dieugenol ;
b) die Epoxidation von zwei, drei oder vier phenolischen -OH-Funktionen von Dieugenol.

9. Verfahren nach Anspruch 1, wobei die Verbindung der Formel (I) ausgewählt ist aus einer Verbindung der Formel : 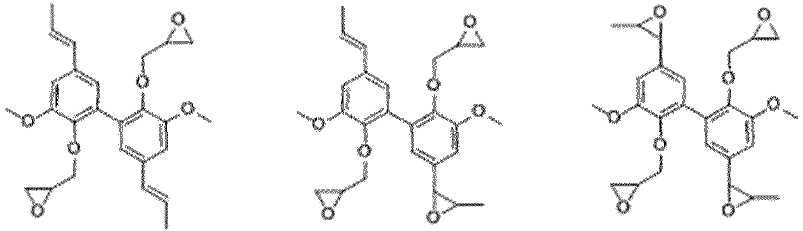 Verfahren, das Folgendes umfasst:
a) die Bereitstellung von Isoeugenol-Dimer ;
b) die Epoxidation von zwei, drei oder vier phenolischen -OH-Funktionen des Isoeugenol-Dimers der Formel :

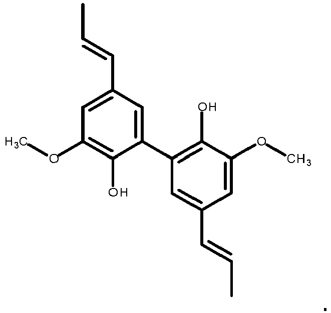

10. Verfahren nach Anspruch 1, wobei in Formel (I) R₃ = -OAlk' und wobei R₁ = R₂ = -CH₂-O-Z, -O-Z oder -COO-Z, wobei die Alkylgruppe Z linear ist und/oder, vorteilhafterweise und, die Epoxyfunktion am Ende der Kette enthält.

11. Verfahren nach Anspruch 10, wobei in Formel (I) R₁ = R₂ = -CH₂-O-[CH₂-]ₙ₋-Epoxy, -O-[CH₂-]ₙ-Epoxy oder -COO-[CH₂-]ₙ-Epoxy, wobei n eine ganze Zahl von 0, vorteilhaft von 1, bis 6 ist ; vorzugsweise in Formel (I) R₁ = R₂ = -CH₂O-CH₂-Epoxy , oder -O-CH₂₋-Epoxy oder -COO-CH₂-Epoxy.

12. Verfahren nach einem der Ansprüche 1, 10 und 11, wobei in Formel (I) R₃ = -OCH₃.

13. Verfahren nach Anspruch 1, wobei die Verbindung der Formel (I) ausgewählt ist aus :
- dem Diglycidylether des methylierten Divanillylalkohols der Formel : 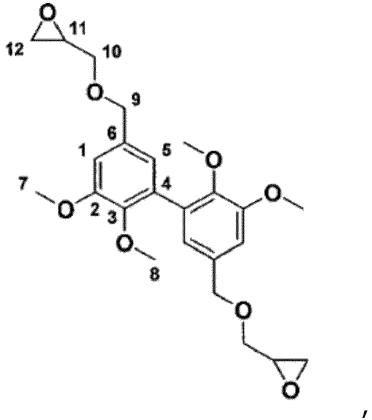
- dem Diglycidylether der methylierten Divanillinsäure der Formel : 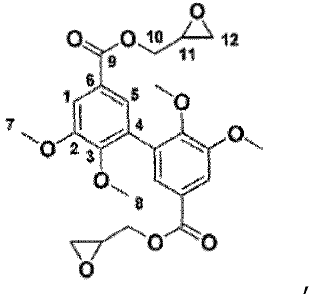
- dem Diglycidylether von methyliertem Dimethoxyhydrochinon der Formel : 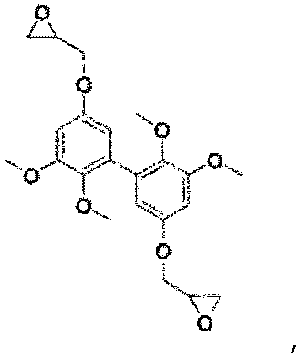
- dem Diglycidylether von methyliertem Dieugenol der Formel : 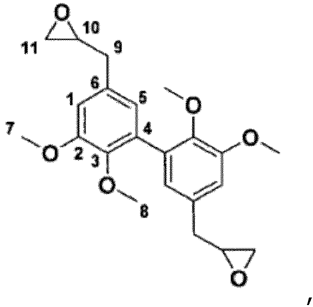 und
- dem Diglycidylether von methyliertem Isoeugenol-Dimers der Formel : 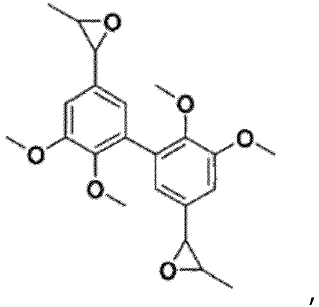
Verfahren, das Folgendes umfasst:
a) die Bereitstellung von Divanillylalkohol, Divanillinsäure, Dimethoxyhydrochinon, Dieugenol oder Isoeugenol-Dimer ;
b) Methylierung der phenolischen -OH-Funktionen von Divanillylalkohol, Divanillinsäure, Dimethoxyhydrochinon, Dieugenol oder Isoeugenol-Dimer, um methylierten Divanillylalkohol, methylierte Divanillinsäure, methyliertes Dimethoxyhydrochinon, methyliertes Dieugenol oder methyliertes Isoeugenol-Dimer zu erhalten;
c) die Epoxidation der beiden nicht alkylierten Funktionen, die noch am Biphenylring vorhanden sind, von methyliertem Divanillylalkohol, methylierter Divanillinsäure, methyliertem Dimethoxyhydrochinon, methyliertem Dieugenol oder methyliertem Isoeugenol-Dimer;
wobei das Dimer von Isoeugenol die folgende Formel aufweist:

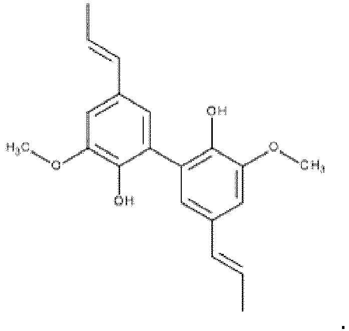

14. Verfahren nach einem der Ansprüche 1 bis 13, **dadurch gekennzeichnet, dass** die Epoxidierung durchgeführt wird :
- durch Reaktion mit einer Verbindung der Formel Cl-Z, in der Z eine lineare oder verzweigte Alkylgruppe mit 2 bis 8, vorzugsweise 3 bis 8, Kohlenstoffatomen ist und eine Epoxyfunktion enthält, wobei die Verbindung vorzugsweise aus Epichlorhydrin besteht, oder
- durch Allylierung und anschließende oxidative Epoxidierung der eingeführten Doppelbindungen, oder
- durch oxidative Epoxidation von mindestens einer vorhandenen Doppelbindung.

## Claims

1. A process for preparing a multi-epoxidized biphenyl compound having the formula (I): 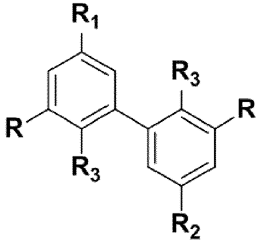 wherein
R = -O-Alk, where Alk is a linear or branched alkyl group having 1 to 6 carbon atoms,
R₃ = -O-Z, where Z is a linear or branched alkyl group containing 2 to 8, advantageously from 3 to 8, carbon atoms and containing an epoxy function, or -O-Alk', where Alk' is a linear or branched alkyl group containing 1 to 6 carbon atoms; and:
+ when R₃ = -O-Z, then,
either R₁ and R₂, which may be the same or different, are independently selected from -CH₂-OH and -CH₂-O-Z;
or R₁=R₂= -CHO;
or R₁ and R₂, which may be the same or different, are independently selected from -OH and -O-Z;
or R₁ and R₂, which may be the same or different, are independently selected from -COOH and -COO-Z,
or R₁ and R₂, which may be the same or different, are independently selected from -CH₂-CH=CH₂ and -CH₂-epoxy,
or R₁ and R₂, which may be the same or different, are independently selected from -CH=CH-CH₃ and 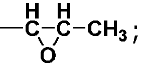 and
+ when R₃ = -O-Alk', then
R₁ = R₂ is selected from:
-CH₂-O-Z,
-O-Z,
-COO-Z,
with Z as defined above with reference to R₃ = -O-Z,
-CH₂-epoxy, and 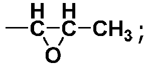
said process comprising:
a) providing a dimer selected from divanillin, divanillyl alcohol, dimethoxyhydroquinone, divanillic acid, dieugenol, the dimer of isoeugenol, said dimers having at least two phenolic -OH functions and two -O-CH₃ functions, and analogs of said dimers having said at least two phenolic -OH functions and two -O-(C₂-C₆)alkyl functions,
b) optionally, the alkylation of the phenolic -OH functions of a dimer chosen from divanillin, divanillyl alcohol, divanillic acid, dieugenol, the dimer of isoeugenol and their analogs in order to obtain said alkylated divanillin, said alkylated divanillyl alcohol, said alkylated divanillic acid, said alkylated dieugenol, said alkylated dimer of isoeugenol and their alkylated analogs; the alkylation of divanillin or an analog thereof being followed by oxidation to obtain alkylated di(C₁-C₆)alkoxyhydroquinone; and
c1) in the absence of such alkylation, epoxidation of the two phenolic -OH functions of divanillin or one of its analogs or epoxidation of the two, or even three, or even four functions present on the biphenyl ring which can be epoxidized, of said divanillyl alcohol, said dimethoxyhydroquinone, said divanillic acid, said dieugenol, said dimer of isoeugenol or of one of their analogs; or
c2) following such alkylation, the epoxidation of the two non-alkylated functions, still present on the biphenyl ring, of the alkylated divanillyl alcohol, alkylated di(C₁-C₆)alkoxyhydroquinone, alkylated divanillic acid, alkylated dieugenol, alkylated dimer of isoeugenol or of one of their analogs;
the dimer of isoeugenol having the following formula:

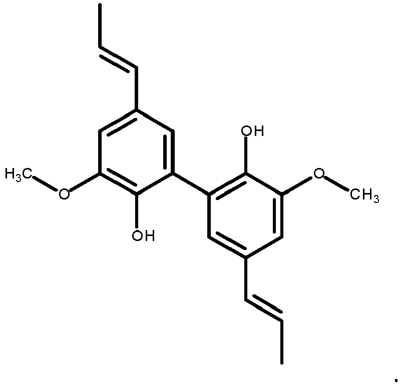

2. The process of claim 1, where in formula (I) R₃ = -O-Z, said alkyl group Z being linear and/or, advantageously and, containing said epoxy function at the chain end.

3. The process of claim 1 or 2, where in formula (I) R₃ = -O-[CH₂-]ₙ-epoxy, where n is an integer from 0, advantageously from 1, to 6, advantageously with 1 ≤ n ≤ 4 and very advantageously with n = 1.

4. The process of claim 1, wherein the compound of formula (I) is selected from:
- the diglycidyl ether of divanillyl alcohol of formula: 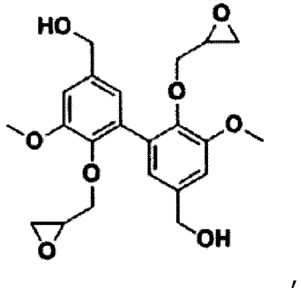
- the triglycidyl ether of divanillyl alcohol of formula: 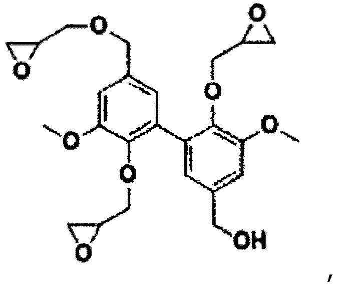 and
- the tetraglycidyl ether of divanillyl alcohol of formula: 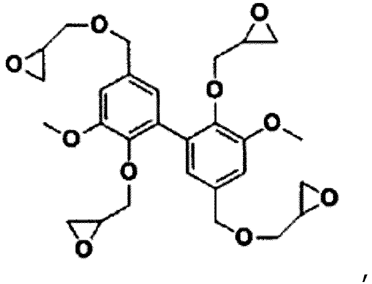 the process comprising:
a) providing divanillyl alcohol;
b) epoxidation of two, three, or four phenolic -OH functions of divanillyl alcohol.

5. The process of claim 1, wherein the compound of formula (I) is the diglycidyl ether of divanillin of formula: 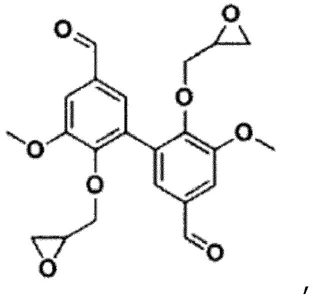 the process comprising:
a) providing divanillin;
b) epoxidation of the two phenolic -OH functions of divanillin.

6. The process of claim 1, wherein the compound of formula (I) is selected from:
- the diglycidyl ether of diméthoxyhydroquinone of formula: 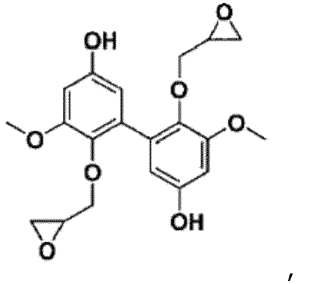
- the triglycidyl ether of dimethoxyhydroquinone of formula: 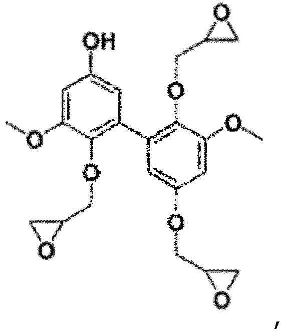 and
- the tetraglycidyl ether of dimethoxyhydroquinone of formula: 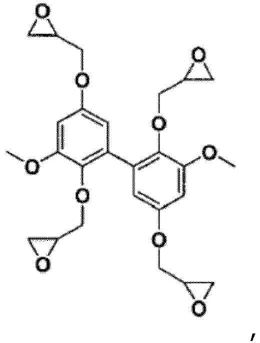 the process comprising:
a) providing dimethoxyhydroquinone;
b) epoxidation of two, three, or four phenolic -OH functions of the dimethoxyhydroquinone.

7. The process of claim 1, wherein the compound of formula (I) is selected from:
- the diglycidyl ether of divanillic acid of formula: 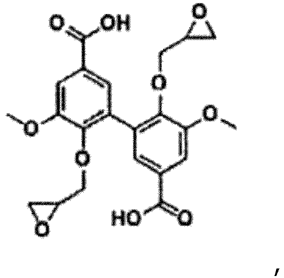
- the triglycidyl ether of divanillic acid of formula: 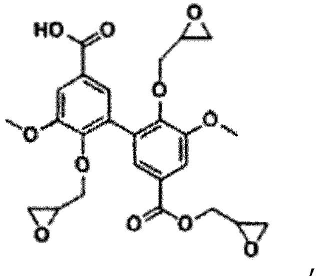 and
- the tetraglycidyl ether of divanillic acid of formula: 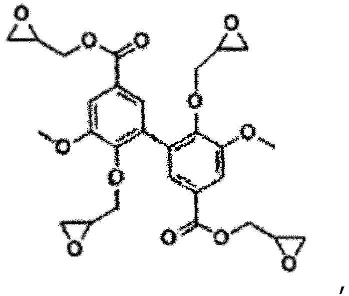 the process comprising:
a) providing divanillic acid;
b) epoxidation of two, three, or four phenolic -OH functions of the divanillic acid.

8. The process of claim 1, wherein the compound of formula (I) is selected from:
- the diglycidyl ether of dieugenol of formula: 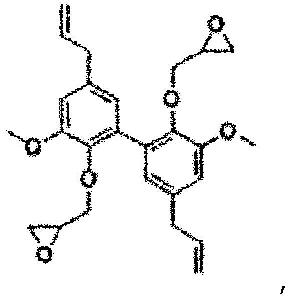
- the triglycidyl ether of dieugenol of formula: 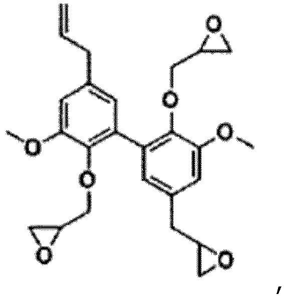 and
- the tetraglycidyl ether of dieugenol of formula: 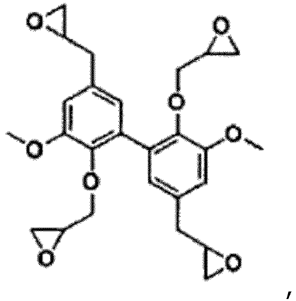 the process comprising:
a) providing dieugenol;
b) epoxidation of two, three, or four phenolic -OH functions of dieugenol.

9. The process of claim 1, wherein the compound of formula (I) is selected from a compound of formula: 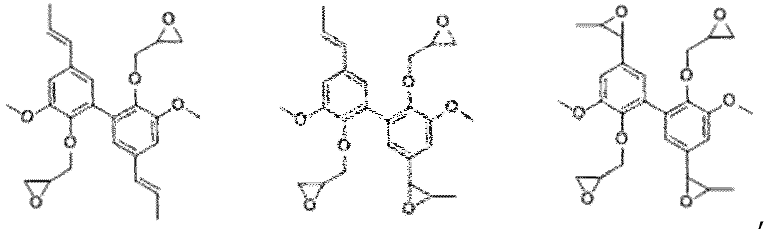 the process comprising:
a) providing the dimer of isoeugenol;
b) epoxidation of two, three, or four phenolic -OH functions of the dimer of isoeugenol.

10. The process of claim 1, where in formula (I) R₃ = -OAlk' and where R₁= R₂ = -CH₂-O-Z, -O-Z or -COO-Z, said alkyl group Z being linear and/or, advantageously and, containing said epoxy function at the chain end.

11. The process of claim 10, where in formula (I) R₁= R₂ = -CH₂-O-[CH₂-]ₙ-epoxy, -O-[CH₂-]ₙ-epoxy or -COO-[CH₂-]ₙ-epoxy, n being an integer from 0, advantageously from 1, to 6; advantageously in formula (I) R₁= R₂ = -CH₂O-CH₂-epoxy, or -O-CH₂-epoxy or -COO-CH₂-epoxy.

12. The process of any one of claims 1, 10 and 11, where in formula (I) R₃ = -O-CH₃.

13. The process of claim 1, where the compound of formula (I) is selected from:
- the diglycidyl ether of methylated divanillyl alcohol of formula: 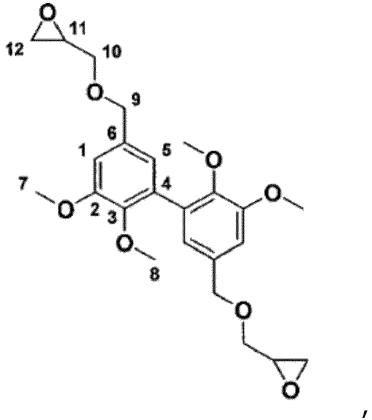
- the diglycidyl ether of methylated divanillic acid of formula: 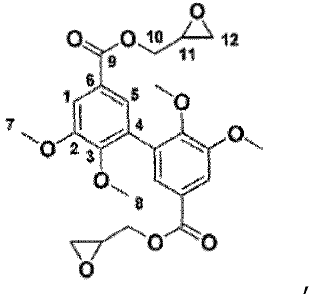
- the diglycidyl ether of methylated dimethoxyhydroquinone of formula: 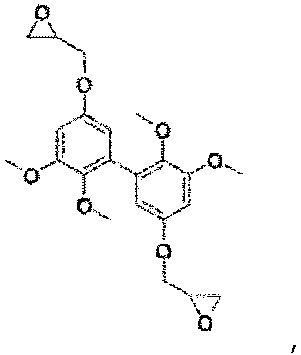
- the diglycidyl ether of methylated dieugenol of formula: 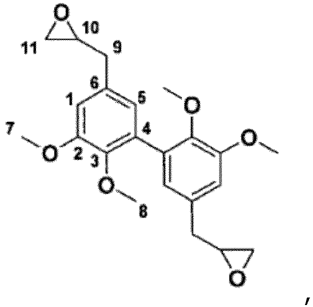 and
- the diglycidyl ether of methylated dimer of isoeugenol of formula: 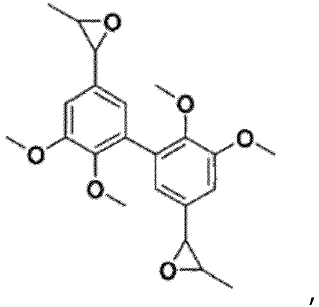
the process comprising:
a) providing divanillyl alcohol, divanillic acid, dimethoxyhydroquinone, dieugenol, or the dimer of isoeugenol;
b) methylation of the phenolic -OH functions of divanillyl alcohol, divanillic acid, diméthoxyhydroquinone, dieugenol, or the dimer of isoeugenol in order to obtain methylated divanillyl alcohol, methylated divanillic acid, methylated diméthoxyhydroquinone, methylated dieugenol, or methylated dimer of isoeugenol; and
c) epoxidation of the two non-alkylated functions, still present on the biphenyl ring, of methylated divanillyl alcohol, methylated divanillic acid, methylated diméthoxyhydroquinone, methylated dieugenol, or methylated dimer of isoeugenol;
the dimer of isoeugenol having the following formula:

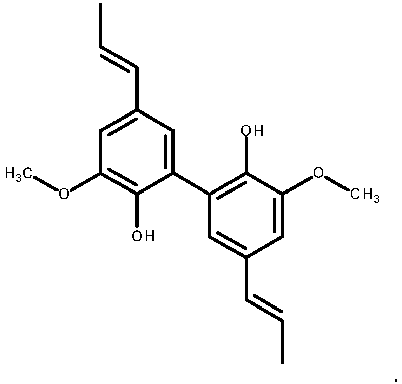

14. The process of any one of claims 1 to 13, wherein the epoxidation is carried out:
- by reaction with a compound of formula Cl-Z, wherein Z is a linear or branched alkyl group containing from 2 to 8, advantageously from 3 to 8, carbon atoms and containing an epoxy function, said compound advantageously consisting of epichlorohydrin, or
- by allylation and subsequent oxidative epoxidation of the double bonds introduced, or
- by oxidative epoxidation of at least one double bond present.
